# EUROPEAN PATENT APPLICATION

(11) **EP 1 536 011 A1**
(43) Date of publication of application: **01.06.2005**
(21) Application number: 03795396.5
(22) Date of filing: 11.09.2003
(51) Int. Cl.: C12N 15/12, A61K 31/7088, A61K 38/00, A61K 39/395, A61K 45/00, A61K 48/00, A61P 25/00

(54) **PREVENTIVES/REMEDIES FOR NEURODEGENERATIVE DISEASES**

(30) Priority: 13.09.2002 JP 2002269091
(71) Applicant: Takeda Pharmaceutical Company Limited, Osaka 541-0045 (JP)
(72) Inventor: MATSUI, Hideki, Tsukuba-shi, Ibaraki 305-0044 (JP); WATANABE, Tomomichi, Tsukuba-shi, Ibaraki 305-0035 (JP)
(74) Representative: Rickard, Timothy Mark Adrian
(86) International application number: PCT/JP2003/011631
(87) International publication number: WO 2004/024920

(57) **Abstract**

A compound or its salts inhibiting the activity of the protein present invention, a compound inhibiting the expression of a gene for the protein, the antisense polynucleotide of the present invention, the antibody of the present invention, and the like can be used as prophylactic/therapeutic agents for neurodegenerative disease, diabetes and the like. A compound or its salts promoting the activity of the protein of the present invention, a compound promoting the expression of a gene for the protein, etc. can be used as prophylactic/therapeutic agents for cancer, rheumatic disease, etc.

## Description

### TECHNICAL FIELD

The present invention relates to a prophylactic/therapeutic agent and diagnostic agent for neurodegenerative disease, and the like. More specifically, the present invention relates to a prophylactic/therapeutic agent and diagnostic agent for diabetes, cancer and rheumatic disease, and screening of the said prophylactic/therapeutic agent.

### BACKGROUND ART

While Alzheimer's disease is typical of the neurodegenerative disease accompanied by a progressive dementia and a loss of cognitive ability, the effective medical treatment has not been found so far. Needless to say, Alzheimer's disease is one of the most important disorders at present on the way to becoming an aging society, and development of its therapeutic agent has an extremely great significance from a medicoeconomical viewpoint as well.

Meanwhile, it is known that an abnormal protein accumulates in endoplasmic reticulum by the factors such as heat shock and glucose starvation that affect biosynthesis of protein and endoplasmic reticulum suffers stress (endoplasmic reticulum stress). When an organism suffers endoplasmic reticulum stress, an endoplasmic reticulum-responsive gene cluster including a chaperon molecule is expressed to repair or degrade abnormal proteins, thus maintaining homeostasis.

Recently, more importance came to be attached to the relation between various neurodegenerative diseases and endoplasmic reticulum stress. Parkin was identified as the pathogenic gene of autosomal recessive juvenile parkinsonism (AR-JP), which is a hereditary Parkinson's disease (Nature, 392, 605-608, 1998), and has been reported to be a ubiquitin ligase involved in the proteolytic system (Nat. Genet., 25, 302-305, 2000). In addition, Pael (Parkin associated endothelin receptor-like) receptor was identified as the substrate of Parkin. This receptor is a protein having difficulty in forming higher-order structure. While this protein that forms higher-order structure only with difficulty is normally degraded rapidly by the action of Parkin, it has been reported that when the proteolytic system is prevented, abnormal Pael receptors accumulate in the endoplasmic reticulum and the cell is led to cell death caused by endoplasmic reticulum stress (Cell, 105, 891-902, 2001.). It has also been reported that a cell harboring presenilin 1 mutation, which is the pathogenic gene of familial Alzheimer's disease, becomes vulnerable to endoplasmic reticulum stress and Irel associated with endoplasmic reticulum stress response is deleted to enhance the production of β amyloid (Biochem. Biophysic. Acta, 1536, 85-96, 2001; J. Biol. Chem., 276, 2108-2114, 2001).

Meanwhile, in order to analyze gene expression exhaustively, the microarray method, in which cDNA or oligonucleotide is immobilized, was developed and the technology to detect changes in disease-specific gene expression has prevailed and its availability has been confirmed. For example, the GeneChip system of Affymetrix Corp. is being frequently used for diagnosis of diseases such as cancer, etc. and discovery of target genes for drug development.

It has been reported that expression of neuronal cell death inducible putative kinase (NIPK) is elevated in a rat-derived cell by NGF starvation and calcium ionophore stimulation, and its coding region bears a kinase domain (BBRC, 258, 260-264, 1999). Recently, it is reported that NIPK binds to ATF4 to regulate a transcription activity factor (Oncogene, 22, 2823-2835, 2003; Experimental Cell Research, 286, 308-320, 2003), NPK binds to Akt1, which is a signal transduction molecule of insulin, to regulate the signal negatively (Science, 300, 1574-1577, 2003). It is reported that activation of signal transduction pathways of Aktl plays an important role in the pathological processes of cancer and rheumatic disease. Though PTEN, a cancer suppressor gene, inactivates Aktl, the function of PTEN is lost by DNA mutation and Aktl is permanently activated in many cancers, which plays an important role in cancerous transformation and malignant alteration of cells (Nature Reviews Cancer, 2, 489-501, 2002). Also, since the Aktl activity is enhanced in synovial cells of a patient with rheumatism and apoptosis is inducible by suppressing the function of Aktl, it is suggested that the activation of Aktl plays an important role in changes in the pathological process of rheumatic disease, which is accompanied by proliferation of synovial cells (Arthritis Rheum., 44, 1555-1567, 2001).

A safe and excellent prophylactic/therapeutic agent for neurodegenerative disease or diabetes has been demanded.

### DISCLOSURE OF INVENTION

As a result of extensive investigations to solve the foregoing problems, the present inventors have found a gene, whose expression is markedly increased when neuronal cell death is induced by endoplasmic reticulum stress, and also have found that the said gene promotes endoplasmic reticulum stress-dependent cell death. Based on these findings, further investigations have been made. Thus, the present invention has come to be accomplished.

That is, the present invention provides the following features, etc.
(1) A prophylactic/therapeutic agent for neurodegenerative disease or diabetes, comprising a compound or its salt inhibiting the activity of a protein comprising the same or substantially the same amino acid sequence as the amino acid sequence represented by SEQ ID NO: 1, its partial peptide, or a salt thereof.
(2) A prophylactic/therapeutic agent for neurodegenerative disease or diabetes, comprising a compound or its salt inhibiting the expression of a gene for a protein comprising the same or substantially the same amino acid sequence as the amino acid sequence represented by SEQ ID NO: 1, its partial peptide, or a salt thereof.
(3) An antisense polynucleotide comprising the entire or part of a base sequence complementary or substantially complementary to a base sequence of a polynucleotide encoding a protein comprising the same or substantially the same amino acid sequence as the amino acid sequence represented by SEQ ID NO: 1, or a partial peptide thereof.
(4) A pharmaceutical comprising the antisense polynucleotide according to (3).
(5) The pharmaceutical according to (4), which is a prophylactic/therapeutic agent for neurodegenerative disease or diabetes.
(6) An antibody to a protein comprising the same or substantially the same amino acid sequence as the amino acid sequence represented by SEQ ID NO: 1, its partial peptide, or a salt thereof.
(7) A pharmaceutical comprising the antibody according to (6).
(8) The pharmaceutical according to (7), which is a prophylactic/therapeutic agent for neurodegenerative disease or diabetes.
(9) A diagnostic agent comprising the antibody according to (6).
(10) The diagnostic agent according to (9), which is a diagnostic agent for neurodegenerative disease or diabetes.
(11) A diagnostic agent for neurodegenerative disease or diabetes, comprising a polynucleotide encoding a protein comprising the same or substantially the same amino acid sequence as the amino acid sequence represented by SEQ ID NO: 1, or its partial peptide.
(12) A method of screening a compound or its salt inhibiting the activity of a protein comprising the same or substantially the same amino acid sequence as the amino acid sequence represented by SEQ ID NO: 1, its partial peptide, or a salt thereof, which comprises using the protein, its partial peptide or a salt thereof.
(13) A kit for screening a compound or its salt inhibiting the activity of a protein comprising the same or substantially the same amino acid sequence as the amino acid sequence represented by SEQ ID NO: 1, its partial peptide, or a salt thereof, comprising the protein, its partial peptide or a salt thereof.
(14) A compound or its salt which is obtainable using the screening method according to (12) or the screening kit according to (13).
(15) A method of screening a compound or its salt inhibiting the expression of a gene for a protein comprising the same or substantially the same amino acid sequence as the amino acid sequence represented by SEQ ID NO: 1, which comprises using a polynucleotide encoding the protein or its partial peptide.
(16) A kit for screening a compound or its salt inhibiting the expression of a gene for a protein comprising the same or substantially the same amino acid sequence as the amino acid sequence represented by SEQ ID NO: 1, comprising a polynucleotide encoding the protein or its partial peptide.
(17) A compound or its salt, which is obtainable using the screening method according to (15) or the screening kit according to (16).
(18) A pharmaceutical comprising the compound or its salt according to (14) or (17).
(19) The pharmaceutical according to (18), which is a prophylactic/therapeutic agent for neurodegenerative disease or diabetes.
(20) A method of preventing/treating neurodegenerative disease or diabetes, which comprises administering an effective dose of the compound or its salt according to (14) or (17) to a mammal.
(21) A method of preventing/treating neurodegenerative disease or diabetes, which comprises inhibiting the activity of a protein comprising the same or substantially the same amino acid sequence as represented by SEQ ID NO: 1, or its partial peptide, or inhibiting the expression of a gene for the protein.
(22) Use of the compound or its salt according to (14) or (17) to manufacture a prophylactic/therapeutic agent for neurodegenerative disease or diabetes.
(23) A prophylactic/therapeutic agent for a cancer or rheumatic disease, comprising a compound or its salt promoting the activity of a protein comprising the same or substantially the same amino acid sequence as the amino acid sequence represented by SEQ ID NO: 1, its partial peptide, or a salt thereof.
(24) A prophylactic/therapeutic agent for a cancer or rheumatic disease, comprising a compound or its salt promoting the expression of a gene for a protein comprising the same or substantially the same amino acid sequence as the amino acid sequence represented by SEQ ID NO: 1, its partial peptide, or a salt thereof.
(25) A pharmaceutical comprising a protein comprising the same or substantially the same amino acid sequence as the amino acid sequence represented by SEQ ID NO: 1, its partial peptide, or a salt thereof.
(26) The pharmaceutical according to (25), which is a prophylactic/therapeutic agent for a cancer or rheumatic disease.
(27) A pharmaceutical comprising a polynucleotide encoding a protein comprising the same or substantially the same amino acid sequence as the amino acid sequence represented by SEQ ID NO: 1, or its partial peptide.
(28) The pharmaceutical according to (27), which is a prophylactic/therapeutic agent for a cancer or rheumatic disease.
(29) A diagnostic agent for cancer or rheumatic disease, comprising a polynucleotide encoding a protein comprising the same or substantially the same amino acid sequence as the amino acid sequence represented by SEQ ID NO: 1, or its partial peptide.
(30) A method of screening a compound or its salt promoting the activity of a protein comprising the same or substantially the same amino acid sequence as the amino acid sequence represented by SEQ ID NO: 1, its partial peptide, or a salt thereof, which comprises using the protein, its partial peptide, or a salt thereof.
(31) A kit for screening a compound or its salt promoting the activity of a protein comprising the same or substantially the same amino acid sequence as the amino acid sequence represented by SEQ ID NO: 1, its partial peptide, or a salt thereof, comprising the protein, its partial peptide, or a salt thereof.
(32) A compound or its salt, which is obtainable using the screening method according to (30) or the screening kit according to (31).
(33) A method of screening a compound or its salt promoting the expression of a gene for a protein comprising the same or substantially the same amino acid sequence as the amino acid sequence represented by SEQ ID NO: 1, which comprises using a polynucleotide encoding the protein or its partial peptide.
(34) A kit for screening a compound or its salt promoting the expression of a gene for a protein comprising the same or substantially the same amino acid sequence as the amino acid sequence represented by SEQ ID NO: 1, comprising a polynucleotide encoding the protein or its partial peptide.
(35) A compound or its salt, which is obtainable using the screening method according to (33) or the screening kit according to (34).
(36) A pharmaceutical comprising the compound or its salt according to (32) or (35).
(37) The pharmaceutical according to (36), which is a prophylactic/therapeutic agent for a cancer or rheumatic disease.
(38) A method of preventing/treating a cancer or rheumatic disease, which comprises administering an effective dose of the compound or its salt according to (32) or (35) to a mammal.
(39) A method of preventing/treating a cancer or rheumatic disease, which comprises promoting the activity of a protein comprising the same or substantially the same amino acid sequence as the amino acid sequence represented by SEQ ID NO: 1 or its partial peptide, or promoting the expression of a gene for the protein.
(40) Use of the compound or its salt according to (32) or (35) to manufacture a prophylactic/therapeutic agent for a cancer or rheumatic disease.

### BRIEF DESCRIPTION OF THE DRAWINGS

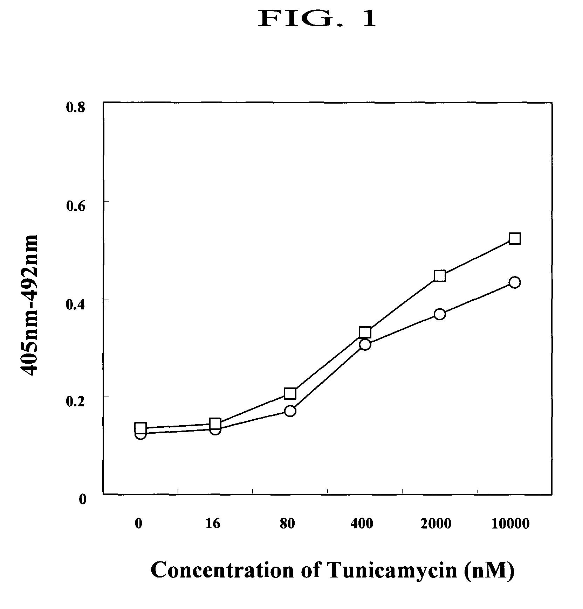

FIG. 1 is a graph showing the DNA break level of cells 24 hours after tunicamycin stimulation at OD405-492. In the figure, the ordinate indicates absorbance and the abscissa indicates the concentration of tunicamycin, wherein -o-designates the DNA break level in control cells and -□- designates the DNA break level in NIPK gene-transfected cells.

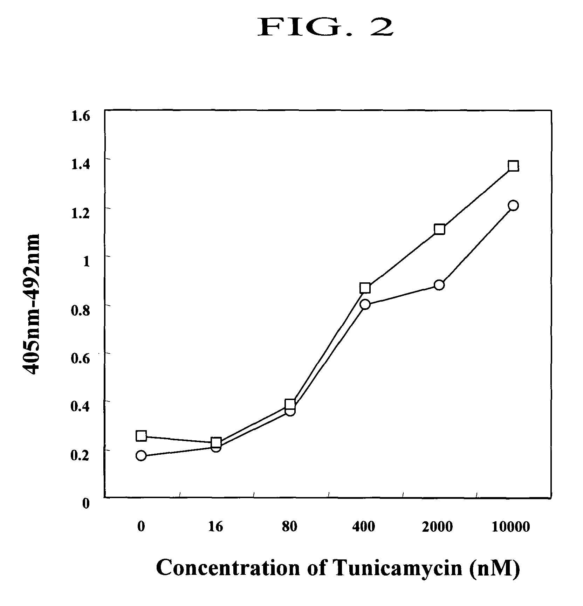

FIG. 2 is a graph showing the DNA break level of cells 48 hours after tunicamycin stimulation at OD405-492. In the figure, the ordinate indicates absorbance and the abscissa indicates the concentration of tunicamycin, wherein -o-designates the DNA break level in control cells and -□- designates the DNA break level in NIPK gene-transfected cells.

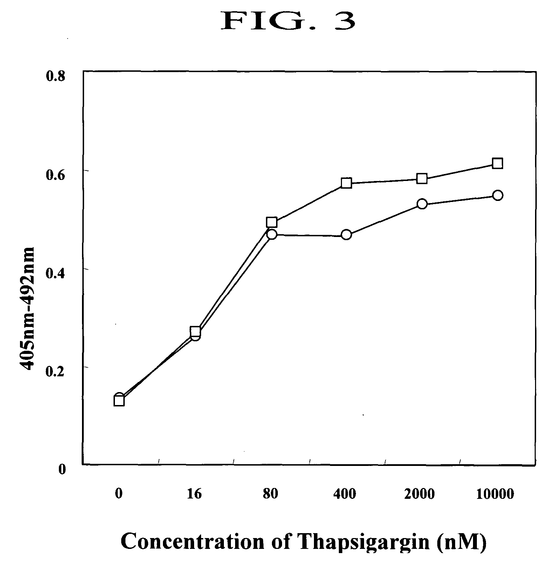

FIG. 3 is a graph showing the DNA break level of cells 24 hours after thapsigargin stimulation at OD405-492. In the figure, the ordinate indicates absorbance and the abscissa indicates the concentration of tunicamycin, wherein -o-designates the DNA break level in control cells and -□- designates the DNA break level in NIPK gene-transfected cells.

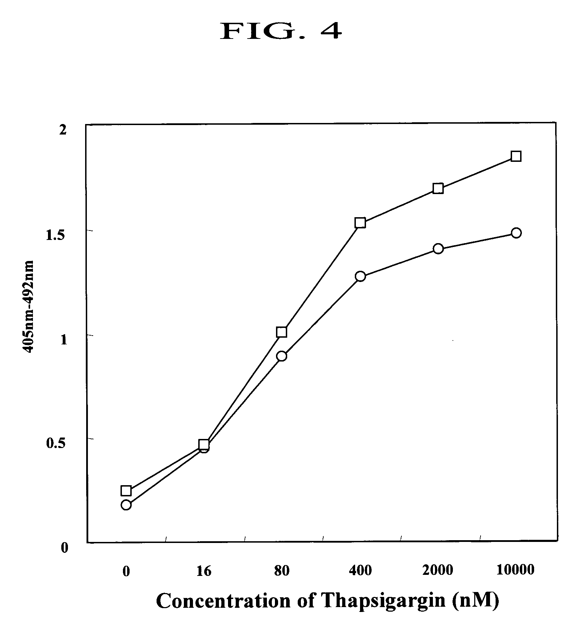

FIG. 4 is a graph showing the respiratory activity of cells 48 hours after thapsigargin stimulation at OD450. In the figure, the ordinate indicates absorbance and the abscissa indicates the concentration of tunicamycin, wherein -o- designates the DNA break level in control cells and -□- designates the DNA break level in NIPK gene-transfected cells.

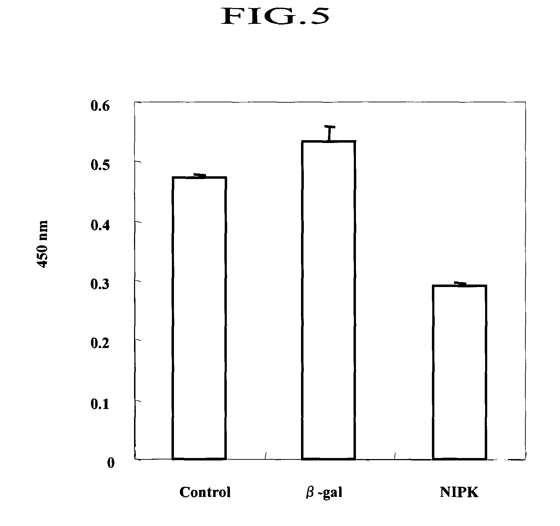

FIG. 5 is a graph showing the respiratory activity of cells 48 hours after thapsigargin stimulation at OD450. In the figure, the ordinate indicates absorbance and the abscissa indicates the transfected gene.

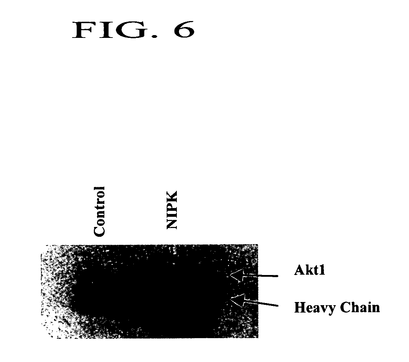

FIG. 6 shows the detection of NIPK bound to Akt1 using anti-Akt1 antibody after immunoprecipitation by anti-V5 antibody.

### BEST MODE FOR CARRYING OUT THE INVENTION

The protein used in the present invention, which has the same or substantially the same amino acid sequence as the amino acid sequence represented by SEQ ID NO: 1 (hereinafter the protein is sometimes referred to as the protein of the present invention or the protein used in the present invention) may be any protein derived from any cells of human and warm-blooded animals (e.g., guinea pig, rat, mouse, fowl, rabbit, swine, sheep, bovine, monkey, etc.) (such as hepatocytes, splenocytes, nerve cells, glial cells, β cells of pancreas, bone marrow cells, mesangial cells, Langerhans' cells, epidermic cells, epithelial cells, goblet cells, endothelial cells, smooth muscle cells, fibroblasts, fibrocytes, myocytes, fat cells, immune cells (e.g., macrophage, T cells, B cells, natural killer cells, mast cells, neutrophils, basophils, eosinophils, monocytes), megakaryocytes, synovial cells, chondrocytes, bone cells, osteoblasts, osteoclasts, mammary gland cells, hepatocytes or interstitial cells; or the corresponding precursor cells, stem cells, cancer cells, etc.); or any tissues where such cells are present, such as brain or any of brain regions (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, prostate, testis, ovary, placenta, uterus, bone, joint, skeletal muscle, etc.; the proteins may also be synthetic proteins.

The amino acid sequence having substantially the same amino acid sequence as that represented by SEQ ID NO: 1 includes amino acid sequences having at least about 50% homology, preferably at least about 60% homology, more preferably at least about 70% homology, much more preferably at least about 80% homology, further much more preferably at least about 90% homology and most preferably at least about 95% homology, to the amino acid sequence shown by SEQ ID NO: 1; and so on.

Homology in the amino acid sequence can be measured using a homology scoring algorithm NCBI BLAST (National Center for Biotechnology Information Basic Local Alignment Search Tool).

Preferred examples of the protein containing substantially the same amino acid sequence as the amino acid sequence represented by SEQ ID NO: 1 include proteins having substantially the same amino acid sequence as the amino acid sequence represented by SEQ ID NO: 1 and having an activity of substantially the same nature as that of the protein having the amino acid sequence represented by SEQ ID NO: 1, etc. Specifically, the protein includes a protein comprising the amino acid sequence represented by SEQ ID NO: 6, SEQ ID NO: 8, SEQ ID NO: 10, SEQ ID NO: 12, SEQ ID NO: 14, SEQ ID NO: 16, SEQ ID NO: 18 or SEQ ID NO: 20; etc.

As the activity of substantially the same nature, there are, for example, a neurofibrillary degeneration promoting activity, a neuronal cell death promoting activity, and the like. The "substantially the same nature" is used to mean that the nature of these activities is equivalent in terms of quality (e.g., physiologically or pharmacologically). Thus, the activities described above are preferably equivalent (e.g., about 0.01 to 100 times, preferably about 0.1 to 10 times, more preferably 0.5 to 2 times), but differences in degree such as a level of these activities, quantitative factors such as a molecular weight of the protein may be present and allowable.

The neurofibrillary degeneration promoting activity can be assayed, e.g., by transfecting an expression vector for the protein of the present invention into cells, staining nerve fibers by immunostaining (using, e.g., an anti-tau antibody, etc.), examining the stained patterns microscopically and determining the degree of changes in nerve fibers.

The neuronal cell death promoting activity can be assayed, e.g., by transfecting an expression vector for the protein of the present invention into cells, adding a cell death inducer (e.g., tunicamycin, thapsigargin, 2-deoxyglucose, β amyloid, okadaic acid, homocysteine, etc.) to the cells and then determining the degree of axonal degeneration, the mitochondrial respiratory activity, the LDH (lactate dehydrogenase) level in the culture supernatant, or the DNA break level accompanied by cell death, etc.

Examples of the protein used in the present invention include so-called muteins such as proteins comprising (1) (i) the amino acid sequence represented by SEQ ID NO: 1, of which at least 1 or 2 (e.g., about 1 to about 100, preferably about 1 to about 30, more preferably about 1 to about 10 and most preferably several (1 to 5)) amino acids are deleted; (ii) the amino acid sequence represented by SEQ ID NO: 1, to which at least 1 or 2 (e.g., about 1 to about 100, preferably about 1 to about 30, more preferably about 1 to about 10 and most preferably several (1 to 5)) amino acids are added; (iii) the amino acid sequence represented by SEQ ID NO: 1, in which at least 1 or 2 (e.g., about 1 to about 100, preferably about 1 to about 30, more preferably about 1 to about 10 and most preferably several (1 to 5)) amino acids are inserted; (iv) the amino acid sequence represented by SEQ ID NO: 1, in which at least 1 or 2 (e.g., about 1 to about 100, preferably about 1 to about 30, more preferably about 1 to about 10 and most preferably several (1 to 5)) amino acids are substituted by other amino acids; or (v) a combination of these amino acid sequences; and the like.

Where the amino acid sequence is inserted, deleted or substituted as described above, the position of its insertion, deletion or substitution is not particularly limited.

Throughout the specification, the proteins are represented in accordance with the conventional way of describing proteins, that is, the N-terminus (amino terminus) at the left hand and the C-terminus (carboxyl terminus) at the right hand. In the protein used in the present invention including the protein having the amino acid sequence represented by SEQ ID NO: 1, the C-terminus may be in any form of a carboxyl group (-COOH), a carboxylate (-COO-), an amide (-CONH₂) and an ester (-COOR).

Herein, examples of the ester group shown by R include a C₁₋₆ alkyl group such as methyl, ethyl, n-propyl, isopropyl, n-butyl, etc.; a C₃₋₈ cycloalkyl group such as cyclopentyl, cyclohexyl, etc.; a C₆₋₁₂ aryl group such as phenyl, α-naphthyl, etc.; a C₇₋₁₄ aralkyl such as a phenyl-C₁₋₂ alkyl group, e.g., benzyl, phenethyl, etc.; an α-naphthyl-C₁₋₂ alkyl group such as α-naphthylmethyl, etc.; pivaloyloxymethyl and the like.

Where the protein used in the present invention contains a carboxyl group (or a carboxylate) at a position other than the C-terminus, the carboxyl group may be amidated or esterified and such an amide or ester is also included within the protein used in the present invention. Examples of the ester group in this case may be the C-terminal esters described above, etc.

Furthermore, examples of the protein used in the present invention include variants wherein the amino group at the N-terminal amino acid residues (e.g., methionine residue) is protected with a protecting group (e.g., a C₁₋₆ acyl group such as a C₁₋₆ alkanoyl group, e.g., formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a C₁₋₆ acyl group such as a C₁₋₆ alkanoyl group, e.g., formyl group, acetyl group, etc.), or conjugated proteins such as glycoproteins having sugar chains; etc.

Specific examples of the protein used in the present invention are a protein comprising the amino acid sequence represented by SEQ ID NO: 1, and the like.

The partial peptide of the protein used in the present invention may be any peptide as long as it is a partial peptide of the protein used in the present invention described above and preferably has the property equivalent to that of the protein used in the present invention described above.

For the purpose of preparing the antibody of the present invention later described, specific examples of the peptide include peptides having the 1 to 30 and 250 to 280 amino acid sequences in the amino acid sequence represented by SEQ ID NO: 1. Preferably used are peptides containing, e.g., at least 20, preferably at least 50, more preferably at least 70, much more preferably at least 100, and most preferably at least 200, amino acids in the constituent amino acid sequence of the protein used in the present invention, and the like.

The partial peptide used in the present invention may contain deletion of at least 1 or 2 (preferably about 1 to about 10 and more preferably several (1 to 5)) amino acids in the amino acid sequence; addition of at least 1 or 2 (preferably about 1 to about 20, more preferably about 1 to about 10 and most preferably several (1 to 5)) amino acids in the amino acid sequence; insertion of at least 1 or 2 (preferably about 1 to about 20, more preferably about 1 to about 10 and most preferably several (1 to 5)) amino acids in the amino acid sequence; or substitution of at least 1 or 2 (preferably about 1 to about 10, more preferably several and most preferably about 1 to about 5) amino acids in the amino acid sequence by other amino acids.

In the partial peptide used in the present invention, the C-terminus may be in any form of a carboxyl group (-COOH), a carboxylate (-COO-), an amide (-CONH₂) or an ester (-COOR).

Furthermore, the partial peptide used in the present invention includes variants having a carboxyl group (or a carboxylate) at a position other than the C-terminus, those having an amino group protected with a protecting group at the N-terminal amino acid residues (e.g., methionine residue); those being cleaved at the N-terminal region in vivo and with the glutamyl group thus formed being pyroglutaminated; those having a substituent on the side chain of an amino acid in the molecule wherein the substituent is protected with a suitable protecting group, or conjugated peptides such as so-called glycopeptides having sugar chains; etc., as in the protein used in the present invention described above.

The partial peptide used in the present invention may also be used as an antigen for producing antibodies.

As salts of the protein or partial peptide used in the present invention, salts with physiologically acceptable acids (e.g., inorganic acids or organic acids) or bases (e.g., alkali metal salts) may be employed, preferably in the form of physiologically acceptable acid addition salts. Examples of such salts include salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The protein or partial peptide used in the present invention or salts thereof may be manufactured by publicly known methods used to purify a protein from human or warm-blooded animal cells or tissues described above. Alternatively, they may also be manufactured by culturing transformants containing DNAs encoding these proteins. Furthermore, they may also be manufactured by a modification of the methods for peptide synthesis, which will be later described.

Where these proteins are manufactured from human or mammalian tissues or cells, human or mammalian tissues or cells are homogenized, extracted with an acid or the like, and the extract is purified/isolated by a combination of chromatography techniques such as reverse phase chromatography, ion exchange chromatography, and the like.

To synthesize the protein or partial peptide used in the present invention or its salts, or amides thereof, commercially available resins that are used for protein synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2',4'-dimethoxyphenyl-hydroxymethyl)phenoxy resin, 4-(2',4'-dimethoxyphenyl-Fmoc-aminoethyl) phenoxy resin, etc. Using these resins, amino acids, in which α-amino groups and functional groups on the side chains are appropriately protected, are condensed on the resin in accordance with the sequence of the objective protein according to various condensation methods publicly known in the art. At the end of the reaction, the protein or partial peptide is excised from the resin and at the same time, the protecting groups are removed. Then, intramolecular disulfide bond-forming reaction is performed in a highly diluted solution to obtain the objective protein or partial peptide, or amides thereof.

For condensation of the protected amino acids described above, a variety of activation reagents for protein synthesis may be used, and carbodiimides are particularly employed. Examples of such carbodiimides include DCC, N,N'-diisopropylcarbodiimide, N-ethyl-N'-(3-dimethylaminopropyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents suitable for use to activate the protected amino acids or condense with the resin may be appropriately chosen from solvents that are known to be usable for protein condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to protein binding reactions and is usually selected in the range of approximately -20°C to 50°C. The activated amino acid derivatives are used generally in an excess of 1.5 to 4 times. The condensation is examined using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole to avoid any possible effect on the subsequent reaction.

Examples of the protecting groups used to protect the starting amino groups include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (linear, branched or cyclic alkyl esterification of, e.g., methyl, ethyl, propyl, butyl, t-butyl, cyclopentyl, cyclohexyl, cycloheptyl, cyclooctyl, 2-adamantyl, etc.), aralkyl esterification (e.g., benzyl ester, 4-nitrobenzyl ester, 4-methoxybenzyl ester, 4-chlorobenzyl ester, benzhydryl ester, etc.), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of groups appropriately used for the esterification include a lower (C₁₋₆) alkanoyl group, such as acetyl group, an aroyl group such as benzoyl group, and a group derived from carbonic acid such as benzyloxycarbonyl group, ethoxycarbonyl group, etc. Examples of a group appropriately used for the etherification include benzyl group, tetrahydropyranyl group, t-butyl group, etc.

Examples of groups for protecting the phenolic hydroxyl group of tyrosine include Bzl, Cl₂-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting material include the corresponding acid anhydrides, azides, activated esters [esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)]. As the amino acids in which the amino groups are activated in the starting material, the corresponding phosphoric amides are employed.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethanesulfonic acid, trifluoroacetic acid, or a mixture solution of these acids; a treatment with a base such as diisopropylethylamine, triethylamine, piperidine or piperazine; reduction with sodium in liquid ammonia, etc. The elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol, 1,2-ethanedithiol, etc. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol, 1,4-butanedithiol, etc. as well as by a treatment with an alkali such as a dilute sodium hydroxide solution, dilute ammonia, etc.

Protection of functional groups that should not be involved in the reaction of the starting materials, protecting groups, elimination of the protecting groups and activation of functional groups involved in the reaction may be appropriately selected from publicly known groups and publicly known means.

In another method for obtaining the amides of the desired protein or partial peptide, for example, the α-carboxyl group of the carboxy terminal amino acid is first protected by amidation; the peptide (protein) chain is then extended from the amino group side to a desired length. Thereafter, a protein or partial peptide, in which only the protecting group of the N-terminal α-amino group of the peptide chain has been eliminated, and a protein or partial peptide, in which only the protecting group of the C-terminal carboxyl group has been eliminated, are manufactured. The two proteins or peptides are condensed in a mixture of the solvents described above. The details of the condensation reaction are the same as described above. After the protected protein or peptide obtained by the condensation is purified, all the protecting groups are eliminated by the method described above to give the desired crude protein or peptide. This crude protein or peptide is purified by various known purification means. Lyophilization of the major fraction gives the amide of the desired protein or peptide.

To prepare the esterified protein or peptide, for example, the α-carboxyl group of the carboxy terminal amino acid is condensed with a desired alcohol to prepare the amino acid ester, which is followed by procedures similar to the preparation of the amidated protein or peptide above to give the desired esterified protein or peptide.

The partial peptide used in the present invention or salts thereof can be manufactured by publicly known methods for peptide synthesis, or by cleaving the protein used in the present invention with an appropriate peptidase. For the methods for peptide synthesis, for example, either solid phase synthesis or liquid phase synthesis may be used. That is, the partial peptide or amino acids that can construct the partial peptide used in the present invention are condensed with the remaining part. Where the product contains protecting groups, these protecting groups are removed to give the desired peptide. Publicly known methods for condensation and elimination of the protecting groups are described in (1) to (5) below.
(i) M. Bodanszky & M.A. Ondetti: Peptide Synthesis, Interscience Publishers, New York (1966)
(ii) Schroeder & Luebke: The Peptide, Academic Press, New York (1965)
(iii) Nobuo Izumiya, et al.: Peptide Gosei-no-Kiso to Jikken (Basics and experiments of peptide synthesis), published by Maruzen Co. (1975)
(iv) Haruaki Yajima & Shunpei Sakakibara: Seikagaku Jikken Koza (Biochemical Experiment) 1, Tanpakushitsu no Kagaku (Chemistry of Proteins) IV, 205 (1977)
(v) Haruaki Yajima ed.: Zoku Iyakuhin no Kaihatsu (A sequel to Development of Pharmaceuticals), Vol. 14, Peptide Synthesis, published by Hirokawa Shoten

After completion of the reaction, the product may be purified and isolated by a combination of conventional purification methods such as solvent extraction, distillation, column chromatography, liquid chromatography and recrystallization to give the partial peptide used in the present invention. When the partial peptide obtained by the above methods is in a free form, the partial peptide can be converted into an appropriate salt by a publicly known method or its modification; when the partial peptide is obtained in a salt form, it can be converted into a free form or other different salt form by a publicly known method or its modification.

The polynucleotide encoding the protein used in the present invention may be any polynucleotide so long as it contains the base sequence encoding the protein used in the present invention described above. Preferably, the polynucleotide is a DNA. The DNA may also be any one of genomic DNA, genomic DNA library, cDNA derived from the cells or tissues described above, cDNA library derived from the cells or tissues described above and synthetic DNA.

The vector used for the library may be any of bacteriophage, plasmid, cosmid, phagemid and the like. In addition, the DNA can be amplified by reverse transcriptase polymerase chain reaction (hereinafter abbreviated as RT-PCR) with total RNA or mRNA fraction prepared from the above-described cells or tissues.

The DNA encoding the protein used in the present invention may be any one of, for example, a DNA comprising the base sequence represented by SEQ ID NO: 2, or a DNA comprising a base sequence hybridizable to the base sequence represented by SEQ ID NO: 2 under high stringent conditions and encoding a protein which has the properties of substantially the same nature as those of the protein having the amino acid sequence represented by SEQ ID NO: 1 described above.

Specific examples of the DNA that is hybridizable to the base sequence represented by SEQ ID NO: 2 under high stringent conditions include DNAs comprising at least about 50% homology, preferably at least about 60% homology, more preferably at least about 70% homology, much more preferably at least about 80% homology, further much more preferably at least about 90% homology and most preferably at least about 95% homology, to the base sequence represented by SEQ ID NO: 2; and the like. Specific examples of the DNA include DNAs comprising the base sequence represented by SEQ ID NO: 5, SEQ ID NO: 7, SEQ ID NO: 9, SEQ ID NO: 11, SEQ ID NO: 13, SEQ ID NO: 15, SEQ ID NO: 17 or SEQ ID NO: 19; and the like.

Homology in the base sequence can be measured under the following conditions (an expectation value = 10; gaps are allowed; filtering = ON; match score = 1; mismatch score = -3) using the homology scoring algorithm NCBI BLAST (National Center for Biotechnology Information Basic Local Alignment Search Tool).

The hybridization can be carried out by publicly known methods or by modifications thereof, for example, by the method described in Molecular Cloning, 2nd (J. Sambrook et al., Cold Spring Harbor Lab. Press, 1989). A commercially available library can also be used according to the instructions of the attached manufacturer's protocol. The hybridization can be carried out preferably under high stringent conditions.

The high stringent conditions used herein are, for example, those in a sodium concentration at about 19 to 40 mM, preferably about 19 to 20 mM at a temperature of about 50 to 70°C, preferably about 60 to 65°C. In particular, hybridization conditions in a sodium concentration at about 19 mM at a temperature of about 65°C are most preferred.

More specifically, as the DNA encoding the protein comprising the amino acid sequence represented by SEQ ID NO: 1, etc.

The polynucleotide (e.g., DNA) encoding the partial peptide used in the present invention may be any polynucleotide so long as it contains the base sequence encoding the partial peptide used in the present invention described above. The polynucleotide may also be any of genomic DNA, genomic DNA library, cDNA derived from the cells and tissues described above, cDNA library derived from the cells and tissues described above and synthetic DNA.

As the DNA encoding the partial peptide used in the present invention, there are employed, for example, a DNA comprising a part of the DNA having the base sequence represented by SEQ ID NO: 2, or a DNA comprising a base sequence hybridizable to the base sequence represented by SEQ ID NO: 2 under high stringent conditions and comprising a part of DNA encoding a protein having the activities of substantially the same nature as those of the protein of the present invention, and the like.

The DNA hybridizable to the base sequence represented by SEQ ID NO: 2 indicates the same meaning as described above.

Methods for the hybridization and the high stringent conditions that can be used are the same as those described above.

For cloning of DNAs that completely encode the protein or partial peptide used in the present invention (hereinafter sometimes merely referred to as the protein of the present invention in the description of cloning of DNAs encoding the protein and partial peptide and their expression), the DNA can be either amplified by PCR using synthetic DNA primers containing a part of the base sequence of the protein of the present invention, or the DNA inserted into an appropriate vector can be selected by hybridization with a labeled DNA fragment or synthetic DNA that encodes a part or entire region of the protein of the present invention. The hybridization can be carried out, for example, according to the method described in Molecular Cloning, 2nd (J. Sambrook et al., Cold Spring Harbor Lab. Press, 1989). Where the hybridization is carried out using commercially available library, the procedures may be conducted in accordance with the protocol described in the attached instructions.

Substitution of the base sequence of DNA can be effected by publicly known methods such as the ODA-LA PCR method, the Gapped duplex method, the Kunkel method, etc., or its modification, using PCR, a publicly known kit available as Mutan™-super Express Km (manufactured by Takara Shuzo Co., Ltd.) or Mutan™-K (manufactured by Takara Shuzo Co., Ltd.), etc.

The cloned DNA encoding the protein can be used as it is, depending upon purpose or, if desired, after digestion with a restriction enzyme or after addition of a linker thereto. The DNA may contain ATG as a translation initiation codon at the 5' end thereof and TAA, TGA or TAG as a translation termination codon at the 3' end thereof. These translation initiation and termination codons may also be added by using an appropriate synthetic DNA adapter.

The expression vector for the protein of the present invention can be manufactured, for example, by (a) excising the desired DNA fragment from the DNA encoding the protein of the present invention, and then (b) ligating the DNA fragment with an appropriate expression vector downstream a promoter in the vector.

Examples of the vector include plasmids derived form E. coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ phage, etc., animal viruses such as retrovirus, vaccinia virus, baculovirus, etc. as well as pA1-11, pXT1, pRc/CMV, pRc/RSV, pcDNA I/Neo, etc.

The promoter used in the present invention may be any promoter if it matches well with a host to be used for gene expression. In the case of using animal cells as the host, examples of the promoter include SRα promoter, SV40 promoter, LTR promoter, CMV promoter, HSV-TK promoter, etc.

Among them, it is preferred to use CMV (cytomegalovirus) promoter, SRα promoter, etc. Where the host is bacteria of the genus Escherichia, preferred examples of the promoter include trp promoter, lac promoter, recA promoter, λP_{L} promoter, lpp promoter, T7 promoter, etc. In the case of using bacteria of the genus Bacillus as the host, preferred example of the promoter are SPO1 promoter, SPO2 promoter, penP promoter, etc. When yeast is used as the host, preferred examples of the promoter are PHO5 promoter, PGK promoter, GAP promoter, ADH promoter, etc. When insect cells are used as the host, preferred examples of the promoter include polyhedrin prompter, P10 promoter, etc.

In addition to the foregoing examples, the expression vector may further optionally contain an enhancer, a splicing signal, a poly A addition signal, a selection marker, SV40 replication origin (hereinafter sometimes abbreviated as SV40ori), etc. Examples of the selection marker include dihydrofolate reductase (hereinafter sometimes abbreviated as dhfr) gene [methotrexate (MTX) resistance], ampicillin resistant gene (hereinafter sometimes abbreviated as Amp^{r}), neomycin resistant gene (hereinafter sometimes abbreviated as Neo^{r}, G418 resistance), etc. In particular, when dhfr gene is used as the selection marker using dhfr gene-deficient Chinese hamster cells, selection can also be made on a thymidine free medium.

If necessary, a signal sequence that matches with a host is added to the N-terminus of the protein of the present invention. Examples of the signal sequence that can be used are PhoA signal sequence, OmpA signal sequence, etc. when bacteria of the genus Escherichia is used as the host; α-amylase signal sequence, subtilisin signal sequence, etc. when bacteria of the genus Bacillus is used as the host; MFα signal sequence, SUC2 signal sequence, etc. when yeast is used as the host; and insulin signal sequence, α-interferon signal sequence, antibody molecule signal sequence, etc. when animal cells are used as the host, respectively.

Using the vector containing the DNA encoding the protein of the present invention thus constructed, transformants can be manufactured.

Examples of the host, which may be employed, are bacteria belonging to the genus Escherichia, bacteria belonging to the genus Bacillus, yeast, insect cells, insects, animal cells, etc.

Specific examples of the bacteria belonging to the genus Escherichia include Escherichia coli K12 DH1 [Proc. Natl. Acad. Sci. U.S.A., 60, 160 (1968)], JM103 [Nucleic Acids Research, 9, 309 (1981)], JA221 [Journal of Molecular Biology, 120, 517 (1978)], HB101 [Journal of Molecular Biology, 41, 459 (1969)], C600 [Genetics, 39, 440 (1954)], etc.

Examples of the bacteria belonging to the genus Bacillus include Bacillus subtilis MI114 [Gene, 24, 255 (1983)], 207-21 [Journal of Biochemistry, 95, 87 (1984)], etc.

Examples of yeast include Saccharomyces cereviseae AH22, AH22R⁻, NA87-11A, DKD-5D, 20B-12, Schizosaccharomyces pombe NCYC1913, NCYC2036, Pichia pastoris KM71, etc.

Examples of insect cells include, for the virus AcNPV, Spodoptera frugiperda cell (Sf cell), MGI cell derived from mid-intestine of Trichoplusia ni, High Five™ cell derived from egg of Trichoplusia ni, cells derived from Mamestra brassicae, cells derived from Estigmena acrea, etc.; and for the virus BmNPV, Bombyx mori N cell (BmN cell), etc. is used. Examples of the Sf cell which can be used are Sf9 cell (ATCC CRL1711), Sf21 cell (both cells are described in Vaughn, J. L. et al., In Vivo, 13, 213-217 (1977)), etc.

As the insect, for example, a larva of Bombyx mori can be used [Maeda et al., Nature, 315, 592 (1985)].

Examples of animal cells include monkey cell COS-7, Vero, Chinese hamster cell CHO (hereinafter referred to as CHO cell), dhfr gene-deficient Chinese hamster cell CHO (hereinafter simply referred to as CHO (dhfr⁻) cell), mouse L cell, mouse AtT-20, mouse myeloma cell, mouse ATDC5 cell, rat GH3, human FL cell, etc.

Bacteria belonging to the genus Escherichia can be transformed, for example, by the method described in Proc. Natl. Acad. Sci. U.S.A., 69, 2110 (1972), Gene, 17, 107 (1982), etc.

Bacteria belonging to the genus Bacillus can be transformed, for example, by the method described in Molecular & General Genetics, 168, 111 (1979), etc.

Yeast can be transformed, for example, by the method described in Methods in Enzymology, 194, 182-187 (1991), Proc. Natl. Acad. Sci. U.S.A., 75, 1929 (1978), etc..

Insect cells or insects can be transformed, for example, according to the method described in Bio/Technology, 6, 47-55 (1988), etc.

Animal cells can be transformed, for example, according to the method described in Saibo Kogaku (Cell Engineering), extra issue 8, Shin Saibo Kogaku Jikken Protocol (New Cell Engineering Experimental Protocol), 263-267 (1995) (published by Shujunsha), or Virology, 52, 456 (1973).

Thus, the transformants transformed with the expression vectors containing the DNAs encoding the protein can be obtained.

Where the host is bacteria belonging to the genus Escherichia or the genus Bacillus, the transformant can be appropriately cultured in a liquid medium, which contains materials required for growth of the transformant such as carbon sources, nitrogen sources, inorganic materials, and the like. Examples of the carbon sources include glucose, dextrin, soluble starch, sucrose, etc.; examples of the nitrogen sources include inorganic or organic materials such as ammonium salts, nitrate salts, corn steep liquor, peptone, casein, meat extract, soybean cake, potato extract, etc.; and, examples of the inorganic materials are calcium chloride, sodium dihydrogenphosphate, magnesium chloride, etc. In addition, yeast extracts, vitamins, growth promoting factors etc. may also be added to the medium. Preferably, pH of the medium is adjusted to about 5 to about 8.

A preferred example of the medium for culturing the bacteria belonging to the genus Escherichia is M9 medium supplemented with glucose and Casamino acids [Miller, Journal of Experiments in Molecular Genetics, 431-433, Cold Spring Harbor Laboratory, New York, 1972]. If necessary, a chemical such as 3β-indolylacrylic acid can be added to the medium thereby to activate the promoter efficiently.

Where the bacteria belonging to the genus Escherichia are used as the host, the transformant is usually cultivated at about 15 to 43°C for about 3 to 24 hours. If necessary, the culture may be aerated or agitated.

Where the bacteria belonging to the genus Bacillus are used as the host, the transformant is cultured generally at about 30 to 40°C for about 6 to 24 hours. If necessary, the culture can be aerated or agitated.

Where yeast is used as the host, the transformant is cultivated, for example, in Burkholder's minimal medium [Bostian, K. L. et al., Proc. Natl. Acad. Sci. U.S.A., 77, 4505 (1980)] or in SD medium supplemented with 0.5% Casamino acids [Bitter, G. A. et al., Proc. Natl. Acad. Sci. U.S.A., 81, 5330 (1984)]. Preferably, pH of the medium is adjusted to about 5 to 8. In general, the transformant is cultivated at about 20 to 35°C for about 24 to 72 hours. If necessary, the culture can be aerated or agitated.

Where insect cells or insects are used as the host, the transformant is cultivated in, for example, Grace's Insect Medium (Grace, T. C. C., Nature), 195, 788 (1962)) to which an appropriate additive such as immobilized 10% bovine serum is added. Preferably, pH of the medium is adjusted to about 6.2 to about 6.4. Normally, the transformant is cultivated at about 27°C for about 3 days to about 5 days and, if necessary, the culture can be aerated or agitated.

Where animal cells are employed as the host, the transformant is cultured in, for example, MEM medium containing about 5 to 20% fetal bovine serum [Science, 122, 501 (1952)], DMEM medium [Virology, 8, 396 (1959)], RPMI 1640 medium [The Journal of the American Medical Association, 199, 519 (1967)], 199 medium [Proceeding of the Society for the Biological Medicine, 73, 1 (1950)], etc. Preferably, pH of the medium is adjusted to about 6 to about 8. The transformant is usually cultivated at about 30°C to about 40°C for about 15 to 60 hours and, if necessary, the culture can be aerated or agitated.

As described above, the protein of the present invention can be produced in the transformant, in the cell membrane of the transformant, or outside of the transformant.

The protein of the present invention can be separated and purified from the culture described above by the following procedures.

When the protein of the present invention is extracted from the bacteria or cells, the bacteria or cell is collected after culturing by a publicly known method and suspended in an appropriate buffer. The bacteria or cell is then disrupted by publicly known methods such as ultrasonication, a treatment with lysozyme and/or freeze-thaw cycling, followed by centrifugation, filtration, etc to produce crude extract of the protein. Thus, the crude extract of the protein can be obtained. The buffer used for the procedures may contain a protein modifier such as urea or guanidine hydrochloride, or a surfactant such as Triton X-100™, etc. When the protein of the present invention is secreted in the culture broth, the supernatant can be separated, after completion of the cultivation, from the bacteria or cell to collect the supernatant by a publicly known method.

The protein contained in the supernatant or the extract thus obtained can be purified by appropriately combining the publicly known methods for separation and purification. Such publicly known methods for separation and purification include a method utilizing difference in solubility such as salting out, solvent precipitation, etc.; a method mainly utilizing difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

When the protein thus obtained is in a free form, the protein can be converted into the salt by publicly known methods or modifications thereof. On the other hand, when the protein is obtained in the form of a salt, it can be converted into the free form or in the form of a different salt by publicly known methods or modifications thereof.

The protein produced by the recombinant can be treated, prior to or after the purification, with an appropriate protein-modifying enzyme so that the protein can be subjected to addition of an appropriate modification or removal of a partial polypeptide. Examples of the protein-modifying enzyme include trypsin, chymotrypsin, arginyl endopeptidase, protein kinase, glycosidase and the like.

The presence of the thus produced protein of the present invention can be determined by an enzyme immunoassay or western blotting using a specific antibody.

The antibodies to the protein or partial peptide used in the present invention, or its salts may be any of polyclonal and monoclonal antibodies, as long as they are capable of recognizing the protein or partial peptide used in the present invention, or its salts.

The antibodies to the protein or partial peptide used in the present invention, or its salts (hereinafter they are sometimes collectively referred to as the protein of the present invention in the description of the antibodies) can be produced by a publicly known method of producing an antibody or antiserum, using the protein of the present invention as an antigen.

### [Preparation of monoclonal antibody]

### (a) Preparation of monoclonal antibody-producing cells

The protein of the present invention is administered to warm-blooded animals either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once every about 2 to about 6 weeks and about 2 to about 10 times in total. Examples of the applicable warm-blooded animals are monkeys, rabbits, dogs, guinea pigs, mice, rats, sheep, goats and fowl, with the use of mice and rats being preferred.

In the preparation of monoclonal antibody-producing cells, a warm-blooded animal, e.g., mouse, immunized with an antigen wherein the antibody titer is noted is selected, then spleen or lymph node is collected after 2 to 5 days from the final immunization and antibody-producing cells contained therein are fused with myeloma cells from homozoic or heterozoic animal to give monoclonal antibody-producing hybridomas. Measurement of the antibody titer in antisera may be carried out, for example, by reacting a labeled protein, which will be described later, with the antiserum followed by assaying the binding activity of the labeling agent bound to the antibody. The fusion may be carried out, for example, by the known method by Koehler and Milstein [Nature, 256, 495, (1975)]. Examples of the fusion accelerator are polyethylene glycol (PEG), Sendai virus, etc., of which PEG is preferably employed.

Examples of the myeloma cells are those collected from warm-blooded animals such as NS-1, P3U1, SP2/0, AP-1, etc. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by incubation at 20 to 40°C, preferably at 30 to 37°C for 1 to 10 minutes, an efficient cell fusion can be carried out.

Various methods can be used for screening of monoclonal antibody-producing hybridomas. Examples of such methods include a method which comprises adding the supernatant of a hybridoma to a solid phase (e.g., a microplate) adsorbed with the protein as an antigen directly or together with a carrier, adding an anti-immunoglobulin antibody (where mouse cells are used for the cell fusion, anti-mouse immunoglobulin antibody is used) labeled with a radioactive substance or an enzyme or Protein A and detecting the monoclonal antibody bound to the solid phase, and a method which comprises adding the supernatant of hybridoma to a solid phase adsorbed with an anti-immunoglobulin antibody or Protein A, adding the protein labeled with a radioactive substance or an enzyme and detecting the monoclonal antibody bound to the solid phase, or the like.

The monoclonal antibody can be screened according to publicly known methods or their modifications. In general, the screening can be performed in a medium for animal cells supplemented with HAT (hypoxanthine, aminopterin and thymidine). Any screening and growth medium can be employed as far as the hybridoma can grow there. For example, RPMI 1640 medium containing 1 to 20%, preferably 10 to 20% fetal bovine serum, GIT medium (Wako Pure Chemical Industries, Ltd.) containing 1 to 10% fetal bovine serum, a serum free medium for cultivation of a hybridoma (SFM-101, Nissui Seiyaku Co., Ltd.) and the like, can be used for the screening and growth medium. The culture is carried out generally at 20 to 40°C, preferably at 37°C, for about 5 days to about 3 weeks, preferably 1 to 2 weeks, normally in 5% CO₂. The antibody titer of the culture supernatant of a hybridoma can be determined as in the assay for the antibody titer in antisera described above.

### (b) Purification of monoclonal antibody

Separation and purification of a monoclonal antibody can be carried out by publicly known methods, such as separation and purification of immunoglobulins [for example, salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A or Protein G and dissociating the binding to obtain the antibody.]

### [Preparation of polyclonal antibody]

The polyclonal antibody of the present invention can be manufactured by publicly known methods or modifications thereof. For example, a warm-blooded animal is immunized with an immunogen (protein antigen) per se, or a complex of immunogen and a carrier protein is formed and the animal is immunized with the complex in a manner similar to the method described above for the manufacture of monoclonal antibodies. The product containing the antibody to the protein of the present invention is collected from the immunized animal followed by separation and purification of the antibody.

In the complex of immunogen and carrier protein used to immunize a warm-blooded animal, the type of carrier protein and the mixing ratio of carrier to hapten may be any type and in any ratio, as long as the antibody is efficiently produced to the hapten immunized by crosslinking to the carrier. For example, bovine serum albumin, bovine thyroglobulin or hemocyanin is coupled to hapten in a carrier-to-hapten weight ratio of approximately 0.1 to 20, preferably about 1 to 5.

A variety of condensation agents can be used for the coupling of carrier to hapten. Glutaraldehyde, carbodiimide, maleimide activated ester and activated ester reagents containing thiol group or dithiopyridyl group are used for the coupling.

The condensation product is administered to warm-blooded animals either solely or together with carriers or diluents to the site that can produce the antibody by the administration. In order to potentiate the antibody productivity upon the administration, complete Freund's adjuvant or incomplete Freund's adjuvant may be administered. The administration is usually made once every about 2 to 6 weeks and about 3 to 10 times in total.

The polyclonal antibody can be collected from the blood, ascites, etc., preferably from the blood of warm-blooded animal immunized by the method described above.

The polyclonal antibody titer in antiserum can be assayed by the same procedure as that for the determination of serum antibody titer described above. The separation and purification of the polyclonal antibody can be carried out, following the method for the separation and purification of immunoglobulins performed as in the separation and purification of monoclonal antibodies described hereinabove.

The antisense polynucleotide having a complementary or substantially complementary base sequence to the base sequence of a polynucleotide encoding the protein or partial peptide used in the present invention (e.g., DNA (hereinafter these DNAs are sometimes collectively referred to as the DNA of the present invention in the description of antisense polynucleotide)) can be any antisense polynucleotide, so long as it possesses a base sequence complementary or substantially complementary to the base sequence of the polynucleotide (e.g., DNA) of the present invention and capable of suppressing the expression of said DNA, but antisense DNA is preferred.

The base sequence substantially complementary to the DNA of the present invention may include, for example, a base sequence having at least about 70% homology, preferably at least about 80% homology, more preferably at least about 90% homology and most preferably at least about 95% homology, to the entire base sequence or to its partial base sequence (i.e., complementary strand to the DNA of the present invention), and the like. Especially in the entire base sequence of the complementary strand to the DNA of the present invention, preferred are (a) an antisense polynucleotide having at least about 70% homology, preferably at least about 80% homology, more preferably at least about 90% homology and most preferably at least about 95% homology, to the complementary strand of the base sequence which encodes the N-terminal region of the protein of the present invention (e.g., the base sequence around the initiation codon) in the case of antisense polynucleotide directed to translation inhibition and (b) an antisense polynucleotide having at least about 70% homology, preferably at least about 80% homology, more preferably at least about 90% homology and most preferably at least about 95% homology, to the complementary strand of the entire base sequence of the DNA of the present invention having intron, in the case of antisense polynucleotide directed to RNA degradation by RNaseH, respectively.

Specific examples include an antisense polynucleotide containing the entire or part of a base sequence complementary or substantially complementary to a base sequence of DNA containing the base sequence represented by SEQ ID NO: 2, preferably an antisense polynucleotide containing the entire or part of a base sequence complementary to a base sequence of DNA containing the base sequence represented by SEQ ID NO: 2 (more preferably, an antisense polynucleotide containing the entire or part of a base sequence complementary to a base sequence of DNA containing the base sequence represented by SEQ ID NO: 2), etc.

The antisense polynucleotide is generally constituted by bases of about 10 to about 40, preferably about 15 to about 30.

To prevent digestion with a hydrolase such as nuclease, etc., the phosphoric acid residue (phosphate) of each nucleotide that constitutes the antisense DNA may be substituted with chemically modified phosphoric acid residues, e.g., phosphorothioate, methyl phosphonate, phosphorodithionate, etc. Also, the sugar (deoxyribose) in each nucleotide may be replaced by a chemically modified structure such as 2'-O-methylation, etc. The base part (pyrimidine, purine) may also be chemically modified and may be any one which hybridizes to a DNA containing the base sequence represented by SEQ ID NO: 2. These antisense polynucleotides may be synthesized using a publicly known DNA synthesizer, etc.

According to the present invention, the antisense polynucleotide capable of inhibiting the replication or expression of a gene for the protein of the present invention can be designed and synthesized based on the base sequence information of cloned or identified protein-encoding DNA. Such a nucleotide (nucleic acid) is hybridizable to RNA of a gene for the protein of the present invention to inhibit the synthesis or function of said RNA or is capable of modulating and/or controlling the expression of a gene for the protein of the present invention via interaction with RNA associated with the protein of the present invention. Polynucleotides complementary to the selected sequences of RNA associated with the protein of the present invention and polynucleotides specifically hybridizable to RNA associated with the protein of the present invention are useful in modulating and/or controlling the in vivo and in vitro expression of the protein gene of the present invention, and are useful for the treatment or diagnosis of diseases, etc. The term "corresponding" is used to mean homologous to or complementary to a particular sequence of the nucleotide including the gene, base sequence or nucleic acid. The term "corresponding" between nucleotides, base sequences or nucleic acids and peptides (proteins) usually refer to amino acids of a peptide (protein) under the order derived from the sequence of nucleotides (nucleic acids) or their complements. In the protein genes, the 5' end hairpin loop, 5' end 6-base-pair repeats, 5' end untranslated region, polypeptide translation initiation codon, protein coding region, ORF translation termination codon, 3' end untranslated region, 3' end palindrome region, and 3' end hairpin loop, may be selected as preferred target regions, though any other region may be selected as a target in the protein genes.

The relationship between the targeted nucleic acids and the polynucleotides complementary to at least a part of the target region, specifically the relationship between the target nucleic acids and the polynucleotides hybridizable to the target region, can be denoted to be "antisense." Examples of the antisense polynucleotides include polynucleotides containing 2-deoxy-D-ribose, polynucleotides containing D-ribose, any other type of polynucleotides which are N-glycosides of a purine or pyrimidine base, or other polymers containing non-nucleotide backbones (e.g., commercially available protein nucleic acids and synthetic sequence-specific nucleic acid polymers) or other polymers containing nonstandard linkages (provided that the polymers contain nucleotides having such a configuration that allows base pairing or base stacking, as is found in DNA or RNA), etc. The antisense polynucleotides may be double-stranded DNA, single-stranded DNA, double-stranded RNA, single-stranded RNA or a DNA:RNA hybrid, and may further include unmodified polynucleotides (or unmodified oligonucleotides), those with publicly known types of modifications, for example, those with labels known in the art, those with caps, methylated polynucleotides, those with substitution of one or more naturally occurring nucleotides by their analogue, those with intramolecular modifications of nucleotides such as those with uncharged linkages (e.g., methyl phosphonates, phosphotriesters, phosphoramidates, carbamates, etc.) and those with charged linkages or sulfur-containing linkages (e.g., phosphorothioates, phosphorodithioates, etc.), those having side chain groups such as proteins (nucleases, nuclease inhibitors, toxins, antibodies, signal peptides, poly-L-lysine, etc.), saccharides (e.g., monosaccharides, etc.), those with intercalators (e.g., acridine, psoralen, etc.), those containing chelators (e.g., metals, radioactive metals, boron, oxidative metals, etc.), those containing alkylating agents, those with modified linkages (e.g., α anomeric nucleic acids, etc.), and the like. Herein the terms "nucleoside", "nucleotide" and "nucleic acid" are used to refer to moieties that contain not only the purine and pyrimidine bases, but also other heterocyclic bases, which have been modified. Such modifications may include methylated purines and pyrimidines, acylated purines and pyrimidines and other heterocyclic rings. Modified nucleotides and modified nucleotides also include modifications on the sugar moiety, wherein, for example, one or more hydroxyl groups may optionally be substituted with a halogen atom(s), an aliphatic group(s), etc., or may be converted into the corresponding functional groups such as ethers, amines, or the like.

The antisense polynucleotide of the present invention is RNA, DNA or a modified nucleic acid (RNA, DNA). Specific examples of the modified nucleic acid are, but not limited to, sulfur and thiophosphate derivatives of nucleic acids and those resistant to degradation of polynucleoside amides or oligonucleoside amides. The antisense nucleic acids of the present invention can be modified preferably based on the following design, that is, by increasing the intracellular stability of the antisense nucleic acid, enhancing the cell permeability of the antisense nucleic acid, increasing the affinity of the nucleic acid to the targeted sense strand to a higher level, or minimizing the toxicity, if any, of the antisense nucleic acid.

Many of such modifications are known in the art, as disclosed in J. Kawakami, et al., Pharm. Tech. Japan, Vol. 8, pp. 247, 1992; Vol. 8, pp. 395, 1992; S. T. Crooke, et al, ed., Antisense Research and Applications, CRC Press, 1993; etc.

The antisense nucleic acid of the present invention may contain altered or modified sugars, bases or linkages. The antisense nucleic acid may also be provided in a specialized form such as liposomes, microspheres, or may be applied to gene therapy, or may be provided in combination with attached moieties. Such attached moieties include polycations such as polylysine that act as charge neutralizers of the phosphate backbone, or hydrophobic moieties such as lipids (e.g., phospholipids, cholesterols, etc.) that enhance the interaction with cell membranes or increase uptake of the nucleic acid. Preferred examples of the lipids to be attached are cholesterols or derivatives thereof (e.g., cholesteryl chloroformate, cholic acid, etc.). These moieties may be attached to the nucleic acid at the 3' or 5' ends thereof and may also be attached thereto through a base, sugar, or intramolecular nucleoside linkage. Other moieties may be capping groups specifically placed at the 3' or 5' ends of the nucleic acid to prevent degradation by nucleases such as exonuclease, RNase, etc. Such capping groups include, but are not limited to, hydroxyl protecting groups known in the art, including glycols such as polyethylene glycol, tetraethylene glycol and the like.

The inhibitory action of the antisense nucleic acid can be examined using the transformant of the present invention, the gene expression system of the present invention in vivo and in vitro, or the translation system for the protein of the present invention in vivo and in vitro. The nucleic acid can be applied to cells by a variety of publicly known methods.

Hereinafter, the protein of the present invention, its partial peptides, or salts thereof (hereinafter sometimes merely referred to as the protein of the present invention), the polynucleotide (e.g., DNA (hereinafter sometimes merely referred to as the DNA of the present invention)) encoding the protein of the present invention or its partial peptides, the antibodies to the protein of the present invention, its partial peptides, or salts thereof (hereinafter sometimes referred to as the antibodies of the present invention) and the antisense polynucleotides to the DNA of the present invention (hereinafter sometimes merely referred to as the antisense polynucleotides of the present invention) are specifically described for their applications.

The protein of the present invention is increasingly expressed when neuronal cell death is induced by endoplasmic reticulum stress to promote endoplasmic reticulum stress-dependent cell death. In addition, the protein of the present invention promotes cell death (neuronal cell death) and neurofibrillary degeneration. Furthermore, the protein of the present invention binds to Akt1, which inhibits the Akt1 activity. Therefore, the protein of the present invention is useful as a marker for early diagnosis in neurodegeneration, diabetes, cancer, rheumatic disorders, etc., for judgment of severity in conditions, or for predicted development of these diseases. Also, the compound or its salts inhibiting the activity of the protein of the present invention, the compound or its salts inhibiting the expression of a gene encoding the protein of the present invention, the antisense polynucleotide of the present invention and pharmaceuticals comprising the antibody to the protein of the present invention can be used as safe prophylactic/therapeutic agents for, e.g., neurodegenerative disease, diabetes and its complications, etc. Moreover, the compound or its salt promoting the activity of the protein of the present invention, the compound or its salt promoting the expression of the gene encoding the protein of the present invention, the protein of the present invention, the DNA of the present invention and the like can be used as safe prophylactic/therapeutic agents for, e.g., cancer, rheumatic disorders, etc.

### (1) Screening of drug candidate compounds for disease

The protein of the present invention is increasingly expressed when neuronal cell death is induced by endoplasmic reticulum stress to promote endoplasmic reticulum stress-dependent cell death. In addition, the protein of the present invention binds to Akt1, which is a main molecule for regulating IGF-1/insulin signal that plays a key role for supporting neuronal survival and tau phosphorylation, to inhibit the Akt1 activity and hence results in tau hyper-phosphorylation. Thus, the protein of the present invention has a neurofibrillary degeneration promoting activity, a neuronal cell death promoting activity, etc.

Therefore, the compound or its salts regulating (inhibiting or promoting, preferably inhibiting) the activity of the protein of the present invention can be safely used as a prophylactic/therapeutic agent for neurodegenerative diseases [e.g., Alzheimer's diseases (familial Alzheimer's disease, juvenile Alzheimer's disease, sporadic Alzheimer's disease, etc.), Parkinson's disease, Down's syndrome, amyotrophic lateral sclerosis, prion disease, Creutzfeldt-Jakob disease, Huntington's chorea, diabetic neuropathy, multiple sclerosis, etc.], diabetes and its complications (e.g., diabetic nephropathy, diabetic neuropathy, diabetic retinopathy, diabetic foot, arteriosclerosis, hypertension, hyperlipemia, etc.), and so on.

On the other hand, the function of PTEN (one of cancer suppressor genes which inactivate Akt1) is lost by DNA mutation and Aktl is permanently activated in many cancers, which plays an important role in cancerous transformation and malignant alteration of cells. Also, the Aktl activity is enhanced in synovial cells of a patient with rheumatism and apoptosis is inducible by suppressing the function of Aktl. For these reasons, the activation of Aktl plays an important role in the pathological process of rheumatic disease, which is accompanied by proliferation of synovial cells.

Therefore, the compound or its salt that regulate (inhibit or promote, preferably promote) the activity of the protein of the present invention can be used, for example, as a safe prophylactic/therapeutic agent for a cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, ovarian cancer, blood tumor, etc.), rheumatic diseases (e.g., chronic articular rheumatism, osteoarthritis, gout, etc.), and so on.

Thus, the protein of the present invention is useful as a reagent for screening the compound or its salts that regulate (inhibit or promote) the activity of the protein of the present invention.

That is, the present invention provides a method of screening the compound or its salts that regulate (inhibit or promote) the activity of the protein of the present invention.

For example, the compound or its salts that regulate (inhibit or promote) the activity of the protein of the present invention is screened by comparing (i) the activity (the neurofibrillary degeneration promoting activity, the neuronal cell death promoting activity, etc.) of the protein of the present invention with (ii) the activity of a mixture of the protein of the present invention and a test compound.

Specifically, the compound or its salts that regulate (inhibit or promote) the activity of the protein of the present invention is screened by the following procedures. For example, (i') when an expression vector for the protein of the present invention is transfected into cells followed by incubation and (ii') when an expression vector for the protein of the present invention is transfected into cells followed by incubation in the presence of a test compound, nerve fibers are stained by immunostaining (using, e.g., an anti-tau antibody, etc.), the stained patterns are examined microscopically, the degree of changes in the nerve fibers is assayed to determine the neurofibrillary degeneration promoting activity, respectively, and comparison is made between (i') and (ii').

More specifically, the compound or its salts that regulate (inhibit or promote) the activity of the protein of the present invention is screened by the following procedures. For example, (i") when an expression vector for the protein of the present invention is transfected into cells followed by incubation and (ii") when an expression vector for the protein of the present invention is transfected into cells followed by incubation in the presence of a test compound, a cell death inducer (e.g., tunicamycin, thapsigargin, 2-deoxyglucose, β amyloid, okadaic acid, homocysteine, etc.) is added to the cells and then the degree of axonal degeneration by calcein staining, the mitochondrial respiratory activity, the LDH (lactate dehydrogenase) level in the culture supernatant, or the DNA break level accompanied by cell death, etc. is assayed to determine the neurofibrillary degeneration promoting activity, respectively, and comparison is made between (i") and (ii").

Akt1 mediates the activation of Cot (which is an oncogene found in the thyroid cancer cell line and one of the mitogen-activated protein kinase kinase kinase family) and IKK (which is a kinase activating transcription of NF-κB by phosphorylation of IκB, an inhibitor of NF-κB) thereby to induce transcription activation of NF-κB (Mol. Cell. Biol., 22, 5962-5974, 2002).

Therefore, the following screening method is also available.

The compound or its salts that regulate (inhibit or promote) the activity of the protein of the present invention is screened, for example, by measuring a reporter expression level (i'") when an expression vector for the protein of the present invention is transfected into cells together with a reporter gene bearing the binding sequence of NFκB contained in the promoter or enhancer, followed by incubation, if necessary, after addition of (a) a ligand-containing fluid such as a microbial lysate, a microbial culture supernatant, an eukaryotic lysate, an eukaryotic lysate culture supernatant, etc., (b) a ligand per se or (c) a substance having an equivalent binding activity to a naturally occurring ligand, and (ii'") when an expression vector for the protein of the present invention is transfected into cells together with a reporter gene bearing the binding sequence of NFκB contained in the promoter or enhancer, followed by incubation in the presence of a test compound, if necessary, after addition of (a) a ligand-containing fluid such as a microbial lysate, a microbial culture supernatant, an eukaryotic lysate, an eukaryotic lysate culture supernatant, etc., (b) a ligand per se or (c) a substance having an equivalent binding activity to a naturally occurring ligand, and comparing (i'") and (ii'").

The reporter expression level can be measured by the reporter protein activity as an indicator using a luciferase activity, an alkaline phosphatase activity, etc.

As the protein of the present invention described above, there are employed proteins produced by culturing cells capable of producing the protein of the present invention, and the like. Furthermore, the culture broth of the cells described above, the supernatant, cell homogenates, etc. may also be employed.

As the cells capable of producing the protein of the present invention, there are used, for example, a host (transformant) transformed with a vector containing the DNA encoding the protein of the present invention. Preferably, animal cells such as COS7 cells, CHO cells, HEK293 cells, etc. are used as the host. For the screening, the transformant, in which the protein of the present invention has been secreted extracellularly or expressed in the cells, e.g., by culturing through the procedures described above, is preferably employed. The procedures for culturing the cells capable of expressing the protein of the present invention are similar to the culturing procedures for the transformant of the present invention described above.

Examples of the test compound include peptides, proteins, non-peptide compounds, synthetic compounds, fermentation products, cell extracts, plant extracts, animal tissue extracts, etc.

For example, when a test compound inhibits the above activity in the cases of (ii), (ii'), (ii") and (ii'") described above by at least about 20%, preferably at least 30% and more preferably at least about 50%, as compared to the cases of (i), (i'), (i") and (i'") described above, the test compound can be selected as the compound capable of inhibiting the activity of the protein of the present invention; when a test compound increases the above activity in the cases of (ii), (ii'), (ii") and (ii'") described above by at least about 20%, preferably at least 30% and more preferably at least about 50%, as compared to the cases of (i), (i'), (i") and (i'") described above, the test compound can be selected as the compound capable of promoting the activity of the protein of the present invention.

The compound having the activity of inhibiting the activity of the protein of the present invention suppresses the physiological activities of the protein of the present invention and is thus useful as a safe and low toxic agent for the prevention/treatment of, for example, neurodegenerative diseases [e.g., Alzheimer's diseases (familial Alzheimer's disease, juvenile Alzheimer's disease, sporadic Alzheimer's disease, etc.), Parkinson's disease, Down's syndrome, amyotrophic lateral sclerosis, prion disease, Creutzfeldt-Jakob disease, Huntington's chorea, diabetic neuropathy, multiple sclerosis, etc.], diabetes and its complications (e.g., diabetic nephropathy, diabetic neuropathy, diabetic retinopathy, diabetic foot, arteriosclerosis, hypertension, hyperlipemia, etc.), and so on.

The compound having the activity of promoting the activity of the protein of the present invention potentiates the physiological activities of the protein of the present invention and is thus useful as a safe and low toxic agent for the prevention/treatment of, for example, a cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, ovarian cancer, blood tumor, etc.), rheumatic diseases (e.g., chronic articular rheumatism, osteoarthritis, gout, etc.), and so on.

The compound or its salt obtained using the screening method or screening kit of the present invention is the compound selected from, for example, peptides, proteins, non-peptide compounds, synthetic compounds, fermentation products, cell extracts, plant extracts, animal tissue extracts, plasma, etc. The salts of these compounds used are those given above as the salts of the peptide of the present invention.

In addition to the effects of the protein of the present invention described above, the gene encoding the protein of the present invention is also increasingly expressed by induced neuronal cell death accompanied by endoplasmic reticulum stress and hence, the compound or its salts that regulate (preferably inhibit) the expression of the gene encoding the protein of the present invention can be used as a prophylactic/therapeutic agent for, e.g., neurodegenerative disease, diabetes and its complications; the compound or its salts that regulate (preferably enhance) the expression of the gene encoding the protein of the present invention can be used as a prophylactic/therapeutic agent for, e.g., cancer, rheumatic disease, etc.

Therefore, the polynucleotide (e.g., DNA) of the present invention is useful as a reagent for screening the compound or its salts that regulate (inhibit or promote) the expression of the gene encoding the protein of the present invention.

For the screening, there is a method of screening which comprises comparing (iii) the case that a cell capable of producing the protein of the present invention is cultured and (iv) the case that a cell capable of producing the protein used in the present invention is cultured in the presence of a test compound.

In the screening method described above, the expression level of the gene described above (specifically, the level of the protein of the present invention or the level of mRNA encoding the said protein) is determined in the cases of (iii) and (iv), followed by comparison.

Examples of the test compound and the cells capable of producing the protein of the present invention are the same as described above.

The level of the protein of the present invention can be determined by publicly known methods, e.g., by measuring the aforesaid protein present in the cell extract, etc., using an antibody capable of recognizing the protein of the present invention, in accordance with methods like western blot analysis, ELISA, etc., or their modifications.

The mRNA level can be determined by publicly known methods, e.g., in accordance with methods such as Northern hybridization using a nucleic acid containing the entire or a part of SEQ ID NO: 2 as a probe, or PCR using a nucleic acid containing the entire or a part of SEQ ID NO: 2 as a primer, or modifications thereof.

For example, when a test compound inhibits the expression of the gene in the case (iv) described above by at least about 20%, preferably at least 30% and more preferably at least about 50%, as compared to the case (iii) above, the test compound can be selected to be the compound capable of inhibiting the expression of the gene encoding the protein of the present invention; when a test compound increases the expression of the gene in the case (iv) described above by at least about 20%, preferably at least 30% and more preferably at least about 50%, as compared to the case (iii) above, the test compound can be selected to be the compound capable of promoting the expression of the gene encoding the protein of the present invention

The screening kit of the present invention comprises the protein used in the present invention, its partial peptide or salts thereof, or the cell capable of producing the protein used in the present invention, or its partial peptide.

The compound or its salts obtained by using the screening method or screening kit of the present invention is the test compound described above, e.g., a compound selected from peptides, proteins, non-peptide compounds, synthetic compounds, fermentation products, cell extracts, plant extracts, animal tissue extracts, plasma, etc., or its salt, which is a compound or its salts regulating the expression of the gene for the protein of the present invention.

The salts of these compounds used are those given above as the salts of the protein of the present invention.

The compound or its salts that regulate (preferably inhibit) the expression of the gene encoding the protein of the present invention can be used, for example, as prophylactic/therapeutic agents for neurodegenerative diseases [e.g., Alzheimer's diseases (familial Alzheimer's disease, juvenile Alzheimer's disease, sporadic Alzheimer's disease, etc.), Parkinson's disease, Down's syndrome, amyotrophic lateral sclerosis, prion disease, Creutzfeldt-Jakob disease, Huntington's chorea, diabetic neuropathy, multiple sclerosis, etc.], diabetes and its complications (e.g., diabetic nephropathy, diabetic neuropathy, diabetic retinopathy, diabetic foot, arteriosclerosis, hypertension, hyperlipemia, etc.) and so on. The compound or its salts that regulate (preferably promote) the expression of the gene encoding the protein of the present invention can be used, for example, as prophylactic/therapeutic agents for a cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, ovarian cancer, blood tumor, etc.), rheumatic diseases (e.g., chronic articular rheumatism, osteoarthritis, gout, etc.) and so on.

Where the compound or its salts obtained by using the screening method or screening kit of the present invention are used as the prophylactic/therapeutic agents described above, these compounds can be converted into pharmaceutical preparations in a conventional manner.

For example, the composition for oral administration includes solid or liquid preparations, specifically, tablets (including dragees and film-coated tablets), pills, granules, powdery preparations, capsules (including soft capsules), syrup, emulsions, suspensions, etc. Such a composition is manufactured by publicly known methods and contains a vehicle, a diluent or excipient conventionally used in the field of pharmaceutical preparations. Examples of the vehicle or excipient for tablets are lactose, starch, sucrose, magnesium stearate, etc.

Examples of the composition for parenteral administration are injectable preparations, suppositories, etc. The injectable preparations may include dosage forms such as intravenous, subcutaneous, intracutaneous and intramuscular injections, drip infusions, intraarticular injection, etc. These injectable preparations may be prepared by methods publicly known. For example, the injectable preparations may be prepared by dissolving, suspending or emulsifying the antibody or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mols) adduct of hydrogenated castor oil)], etc. As the oily medium, there are employed, e.g., sesame oil, soybean oil, etc., which may be used in combination with a solubilizing agent such as benzyl benzoate, benzyl alcohol, etc. The injection thus prepared is usually filled in an appropriate ampoule. The suppository used for rectal administration may be prepared by blending the aforesaid antibody or its salt with conventional bases for suppositories.

Advantageously, the pharmaceutical compositions for oral or parenteral use described above are prepared into pharmaceutical preparations with a unit dose suited to fit a dose of the active ingredients. Such unit dose preparations include, for example, tablets, pills, capsules, injections (ampoules), suppositories, etc. The amount of the aforesaid compound contained is generally 5 to 500 mg per dosage unit form; it is preferred that the aforesaid antibody is contained in about 5 to about 100 mg especially in the form of injection, and in 10 to 250 mg for the other forms.

Each composition described above may further contain other active components unless formulation causes any adverse interaction with the compound described above.

Since the pharmaceutical preparations thus obtained are safe and low toxic, they can be administered to human or warm-blooded animal (e.g., mouse, rat, rabbit, sheep, swine, bovine, horse, fowl, cat, dog, monkey, chimpanzee, etc.) orally or parenterally.

The dose of the compound or its salts may vary depending upon its action, target disease, subject to be administered, route of administration, etc. For example, when the compound or its salt inhibiting the activity of the protein of the present invention or the expression of the gene for said protein is orally administered for the purpose of treating, e.g., Alzheimer's disease, the compound or its salt is generally administered to an adult (as 60 kg body weight) in a daily dose of about 0.1 to about 100 mg, preferably about 1.0 to about 50 mg and more preferably about 1.0 to about 20 mg. In parenteral administration, a single dose of the said compound or its salt may vary depending upon subject to be administered, target disease, etc. When the compound or its salt inhibiting the activity of the protein of the present invention or the expression of the gene for said protein is administered to an adult (as 60 kg body weight) in the form of an injectable preparation for the purpose of treating, e.g., Alzheimer's disease, it is advantageous to administer the compound or its salt by way of intravenous injection in a daily dose of about 0.01 to about 30 mg, preferably about 0.1 to about 20 mg, and more preferably about 0.1 to about 10 mg. For other animal species, the corresponding dose as converted per 60 kg weight can be administered.

For example, when the compound or its salt promoting the activity of the protein of the present invention or the expression of the gene for said protein is orally administered for the purpose of treating, e.g., chronic articular rheumatism, the compound or its salt is generally administered to an adult (as 60 kg body weight) in a daily dose of about 0.1 to about 100 mg, preferably about 1.0 to about 50 mg and more preferably about 1.0 to about 20 mg. In parenteral administration, a single dose of the said compound or its salt may vary depending upon subject to be administered, target disease, etc. When the compound or its salt inhibiting the activity of the protein of the present invention or the expression of the gene for said protein is administered to an adult (as 60 kg body weight) in the form of an injectable preparation for the purpose of treating, e.g., chronic articular rheumatism, it is advantageous to administer the compound or its salt by way of intraarticular injection in a daily dose of about 0.01 to about 30 mg, preferably about 0.1 to about 20 mg, and more preferably about 0.1 to about 10 mg. For other animal species, the corresponding dose as converted per 60 kg weight can be administered.

### (2) Quantification for the protein of the present invention, it partial peptide or salts thereof

The antibody to the protein of the present invention (hereinafter sometimes merely referred to as the antibody of the present invention) is capable of specifically recognizing the protein of the present invention, and thus can be used for quantification of the protein of the present invention in a test sample fluid, in particular, for quantification by sandwich immunoassay; etc.

That is, the present invention provides:
(i) a method of quantifying the protein of the present invention in a test sample fluid, which comprises competitively reacting the antibody of the present invention, a test sample fluid and a labeled form of the protein of the present invention, and measuring the ratio of the labeled form of the protein of the present invention bound to said antibody; and,
(ii) a method of quantifying the protein of the present invention in a test sample fluid, which comprises reacting a test sample fluid simultaneously or continuously with the antibody of the present invention immobilized on a carrier and another labeled antibody of the present invention, and then measuring the activity of the labeling agent on the insoluble carrier.

In the quantification method (ii) described above, it is preferred that one antibody is capable of recognizing the N-terminal region of the protein of the present invention, while another antibody is capable of reacting with the C-terminal region of the protein of the present invention.

The monoclonal antibody to the protein of the present invention (hereinafter sometimes referred to as the monoclonal antibody of the present invention) can be used to quantify the protein of the present invention. In addition, the protein can be detected by means of a tissue staining as well. For these purposes, the antibody molecule per se may be used or F(ab')₂, Fab' or Fab fractions of the antibody molecule may also be used.

The method of quantifying the protein of the present invention using the antibody of the present invention is not particularly limited. Any quantification method can be used, so long as the amount of antibody, antigen or antibody-antigen complex corresponding to the amount of antigen (e.g., the amount of the protein) in a test sample fluid can be detected by chemical or physical means and the amount of the antigen can be calculated from a standard curve prepared from standard solutions containing known amounts of the antigen. For such an assay method, for example, nephrometry, the competitive method, the immunometric method, the sandwich method, etc. are suitably used and in terms of sensitivity and specificity, it is particularly preferred to use the sandwich method described hereinafter.

Examples of the labeling agent used in the assay method using the labeling substance are radioisotopes, enzymes, fluorescent substances, luminescent substances, and the like. As the radioisotopes, there are used, e.g., [¹²⁵I], [¹³¹I], [³H], [¹⁴C], etc. The enzymes described above are preferably enzymes, which are stable and have a high specific activity, and include, e.g., β-galactosidase, β-glucosidase, an alkaline phosphatase, a peroxidase, malate dehydrogenase, etc. As the fluorescent substances, there are used, e.g., fluorescamine, fluorescein isothiocyanate, etc. As the luminescent substances described above there are used, e.g., luminol, a luminol derivative, luciferin, lucigenin, etc. Furthermore, the biotin-avidin system may be used as well for binding of an antibody or antigen to a labeling agent.

For immobilization of the antigen or antibody, physical adsorption may be used. Chemical binding techniques conventionally used for insolubilization or immobilization of proteins, enzymes, etc. may also be used. For carriers, there are used, e.g., insoluble polysaccharides such as agarose, dextran, cellulose, etc.; synthetic resin such as polystyrene, polyacrylamide, silicon, etc., and glass or the like.

In the sandwich method, the immobilized monoclonal antibody of the present invention is reacted with a test fluid (primary reaction), then with a labeled form of another monoclonal antibody of the present invention (secondary reaction), and the activity of the label on the immobilizing carrier is measured, whereby the amount of the protein of the present invention in the test fluid can be quantified. The order of the primary and secondary reactions may be reversed, and the reactions may be performed simultaneously or with an interval. The methods of labeling and immobilization can be performed by the methods described above. In the immunoassay by the sandwich method, the antibody used for immobilized or labeled antibodies is not necessarily one species, but a mixture of two or more species of antibody may be used to increase the measurement sensitivity.

In the methods of assaying the protein of the present invention by the sandwich method of the present invention, antibodies that bind to different sites of the protein of the present invention are preferably used as the monoclonal antibodies of the present invention used for the primary and secondary reactions. That is, in the antibodies used for the primary and secondary reactions are, for example, when the antibody used in the secondary reaction recognizes the C-terminal region of the protein of the present invention, it is preferable to use the antibody recognizing the region other than the C-terminal region for the primary reaction, e.g., the antibody recognizing the N-terminal region.

The monoclonal antibodies of the present invention can be used for the assay systems other than the sandwich method, for example, the competitive method, the immunometric method, nephrometry, etc.

In the competitive method, antigen in a test fluid and the labeled antigen are competitively reacted with antibody, and the unreacted labeled antigen (F) and the labeled antigen bound to the antibody (B) are separated (B/F separation). The amount of the label in B or F is measured, and the amount of the antigen in the test fluid is quantified. This reaction method includes a liquid phase method using a soluble antibody as an antibody, polyethylene glycol for B/F separation and a secondary antibody to the soluble antibody, and an immobilized method either using an immobilized antibody as the primary antibody, or using a soluble antibody as the primary antibody and immobilized antibody as the secondary antibody.

In the immunometric method, antigen in a test fluid and immobilized antigen are competitively reacted with a definite amount of labeled antibody, the immobilized phase is separated from the liquid phase, or antigen in a test fluid and an excess amount of labeled antibody are reacted, immobilized antigen is then added to bind the unreacted labeled antibody to the immobilized phase, and the immobilized phase is separated from the liquid phase. Then, the amount of the label in either phase is measured to quantify the antigen in the test fluid.

In the nephrometry, insoluble precipitate produced after the antigen-antibody reaction in gel or solution is quantified. When the amount of antigen in the test fluid is small and only a small amount of precipitate is obtained, laser nephrometry using scattering of laser is advantageously employed.

For applying each of these immunological methods to the quantification method of the present invention, any particular conditions or procedures are not required. Quantification system for the protein of the present invention or its salts is constructed by adding the usual technical consideration in the art to the conventional conditions and procedures. For the details of these general technical means, reference can be made to the following reviews and texts.

For example, Hiroshi Irie, ed. "Radioimmunoassay" (Kodansha, published in 1974), Hiroshi Irie, ed. "Sequel to the Radioimmunoassay" (Kodansha, published in 1979), Eiji Ishikawa, et al. ed. "Enzyme immunoassay" (Igakushoin, published in 1978), Eiji Ishikawa, et al. ed. "Immunoenzyme assay" (2nd ed.) (Igakushoin, published in 1982), Eiji Ishikawa, et al. ed. "Immunoenzyme assay" (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies)) (all published by Academic Press Publishing).

As described above, the protein of the present invention can be quantified with high sensitivity, using the antibody of the present invention.

Furthermore, when an increased or decreased level of the protein of the present invention is detected by quantifying the level of the protein of the present invention using the antibody of the present invention, it can be diagnosed that one suffers from, for example, neurodegenerative diseases [e.g., Alzheimer's diseases (familial Alzheimer's disease, juvenile Alzheimer's disease, sporadic Alzheimer's disease, etc.), Parkinson's disease, Down's syndrome, amyotrophic lateral sclerosis, prion disease, Creutzfeldt-Jakob disease, Huntington's chorea, diabetic neuropathy, multiple sclerosis, etc.], diabetes and its complications (e.g., diabetic nephropathy, diabetic neuropathy, diabetic retinopathy, diabetic foot, arteriosclerosis, hypertension, hyperlipemia, etc.) or the like; or it is highly likely to suffer from these disease in the future.

In addition, the antibody of the present invention can be used to detect the protein of the present invention, which is present in a test sample such as a body fluid, a tissue, etc. The antibody can also be used to prepare an antibody column for purification of the protein of the present invention, detect the protein of the present invention in each fraction upon purification, analyze the behavior of the protein of the present invention in the cells under investigation; etc.

### (3) Gene diagnostic agent

By using the DNA of the present invention, e.g., as a probe, the DNA can detect an abnormality (gene abnormality) of the DNA or mRNA encoding the protein of the present invention or its partial peptide in human or warm-blooded animal (e.g., rat, mouse, guinea pig, rabbit, fowl, sheep, swine, bovine, horse, cat, dog, monkey, chimpanzee, etc.). Therefore, the DNA of the present invention is useful as a gene diagnostic agent for detecting damages to the DNA or mRNA, its mutation, or decreased expression, increased expression, overexpression, etc. of the DNA or mRNA, and so on.

The gene diagnosis described above using the DNA of the present invention can be performed by, for example, the publicly known Northern hybridization assay or the PCR-SSCP assay (Genomics, 5, 874-879 (1989); Proceedings of the National Academy of Sciences of the United States of America, 86, 2766-2770 (1989)), etc.

When overexpression or decreased expression is detected by, e.g., Northern hybridization or DNA mutation is detected by the PCR-SSCP assay, it can be diagnosed that it is highly likely to suffer from, for example, neurodegenerative diseases [e.g., Alzheimer's diseases (familial Alzheimer's disease, juvenile Alzheimer's disease, sporadic Alzheimer's disease, etc.), Parkinson's disease, Down's syndrome, amyotrophic lateral sclerosis, prion disease, Creutzfeldt-Jakob disease, Huntington's chorea, diabetic neuropathy, multiple sclerosis, etc.], diabetes and its complications (e.g., diabetic nephropathy, diabetic neuropathy, diabetic retinopathy, diabetic foot, arteriosclerosis, hypertension, hyperlipemia, etc.) or the like.

### (4) Pharmaceutical comprising the antisense polynucleotide

The antisense polynucleotide of the present invention that binds to the DNA of the present invention complementarily to suppress the expression of said DNA is low toxic and can suppress the functions of the protein of the present invention or the DNA of the present invention in vivo. Thus, the antisense polynucleotide can be used, for example, as a prophylactic/therapeutic agent for neurodegenerative diseases [e.g., Alzheimer's diseases (familial Alzheimer's disease, juvenile Alzheimer's disease, sporadic Alzheimer's disease, etc.), Parkinson's disease, Down's syndrome, amyotrophic lateral sclerosis, prion disease, Creutzfeldt-Jakob disease, Huntington's chorea, diabetic neuropathy, multiple sclerosis, etc.], diabetes and its complications (e.g., diabetic nephropathy, diabetic neuropathy, diabetic retinopathy, diabetic foot, arteriosclerosis, hypertension, hyperlipemia, etc.), or the like.

Where the antisense polynucleotide described above is used as the aforesaid prophylactic/therapeutic agent, it can be prepared into pharmaceutical preparations by publicly known methods, which are provided for administration.

For example, when the antisense polynucleotide described above is used, the antisense polynucleotide alone is administered directly, or the antisense polynucleotide is inserted into an appropriate vector such as retrovirus vector, adenovirus vector, adenovirus-associated virus vector, etc., followed by treating in a conventional manner. The antisense polynucleotide may then be administered orally or parenterally to human or mammal (e.g., rat, rabbit, sheep, swine, bovine, cat, dog, monkey, etc.) in a conventional manner. The antisense polynucleotide may also be administered as it stands, or may be prepared in pharmaceutical preparations together with a physiologically acceptable carrier to assist its uptake, which are then administered by gene gun or through a catheter such as a catheter with a hydrogel. Alternatively, the antisense polynucleotide may be prepared into an aerosol, which is topically administered into the trachea as an inhaler.

Further for the purposes of improving pharmacokinetics, prolonging a half-life and improving intracellular uptake efficiency, the antisense polynucleotide described above is prepared into pharmaceutical preparations (injectable preparations) alone or together with a carrier such as liposome, etc. and the preparations may be administered intravenously, subcutaneously, or at the affected area, etc.

A dose of the antisense polynucleotide may vary depending on target disease, subject to be administered, route for administration, etc. For example, where the antisense polynucleotide of the present invention is administered for the purpose of treating Alzheimer's disease, the antisense polynucleotide is generally administered to an adult (60 kg body weight) in a daily dose of about 0.1 to 100 mg.

Furthermore, the antisense polynucleotide may also be used as an oligonucleotide probe for diagnosis to examine the presence of the DNA of the present invention in tissues or cells and states of its expression.

As the antisense polynucleotide described above can, the double-stranded RNA containing a part of RNA encoding the protein of the present invention, ribozyme containing a part of RNA encoding the protein of the present invention, etc. can also prevent the expression of the gene encoding the present invention to suppress the in vivo function of the protein used in the present invention or the DNA used in the present invention and hence can be used, for example, as a prophylactic/therapeutic agent for neurodegenerative diseases [e.g., Alzheimer's diseases (familial Alzheimer's disease, juvenile Alzheimer's disease, sporadic Alzheimer's disease, etc.), Parkinson's disease, Down's syndrome, amyotrophic lateral sclerosis, prion disease, Creutzfeldt-Jakob disease, Huntington's chorea, diabetic neuropathy, multiple sclerosis, etc.], diabetes and its complications (e.g., diabetic nephropathy, diabetic neuropathy, diabetic retinopathy, diabetic foot, arteriosclerosis, hypertension, hyperlipemia, etc.), or the like.

The double-stranded RNA can be designed based on a sequence of the polynucleotide of the present invention and manufactured by modifications of publicly known methods (e.g., Nature, 411, 494, 2001).

The ribozyme can be designed based on a sequence of the polynucleotide of the present invention and manufactured by modifications of publicly known methods (e.g., TRENDS in Molecular Medicine, 7, 221, 2001). For example, the ribozyme can be manufactured by ligating a publicly known ribozyme to a part of the RNA encoding the protein of the present invention. A part of the RNA encoding the protein of the present invention includes a portion proximal to a cleavage site on the RNA of the present invention, which may be cleaved by a publicly known ribozyme (RNA fragment).

Where the double-stranded RNA or ribozyme described above is used as the prophylactic/therapeutic agent described above, the double-stranded RNA or ribozyme is prepared into pharmaceutical preparations as in the antisense polynucleotide, and the preparations can be provided for administration.

### (5) Pharmaceutical comprising the antibody of the present invention

The antibody of the present invention, which has the activity of neutralizing the activity of the protein of the present invention can be used, for example, as a prophylactic/therapeutic agent for neurodegenerative diseases [e.g., Alzheimer's diseases (familial Alzheimer's disease, juvenile Alzheimer's disease, sporadic Alzheimer's disease, etc.), Parkinson's disease, Down's syndrome, amyotrophic lateral sclerosis, prion disease, Creutzfeldt-Jakob disease, Huntington's chorea, diabetic neuropathy, multiple sclerosis, etc.], diabetes and its complications (e.g., diabetic nephropathy, diabetic neuropathy, diabetic retinopathy, diabetic foot, arteriosclerosis, hypertension, hyperlipemia, etc.), or the like.

Examples of the composition for oral administration include solid or liquid preparations, specifically, tablets (including dragees and film-coated tablets), pills, granules, powdery preparations, capsules (including soft capsules), syrup, emulsions, suspensions, etc. Such a composition is manufactured by publicly known methods and contains a vehicle, a diluent or an excipient conventionally used in the field of pharmaceutical preparations. Examples of the vehicle or excipient for tablets are lactose, starch, sucrose, magnesium stearate, etc.

Examples of the composition for parenteral administration are injectable preparations, suppositories, vaccine, etc. The injectable preparations may include dosage forms such as intravenous, subcutaneous, intracutaneous and intramuscular injections, drip infusions, etc. These injectable preparations may be prepared by methods publicly known. The injectable preparations may be prepared, e.g., by dissolving, suspending or emulsifying the antibody or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mols) adduct of hydrogenated castor oil)], etc. As the oily medium, there are employed, e.g., sesame oil, soybean oil, etc., which may be used in combination with a solubilizing agent such as benzyl benzoate, benzyl alcohol, etc. The injection thus prepared is preferably filled in an appropriate ampoule. The suppository used for rectal administration may be prepared by blending the aforesaid antibody or its salt with conventional bases for suppositories.

Advantageously, the pharmaceutical compositions for oral or parenteral use described above are prepared into pharmaceutical preparations in a unit dose suited to fit a dose of the active ingredients. Such unit dose preparations include, for example, tablets, pills, capsules, injections (ampoules), suppositories, etc. The amount of the aforesaid antibody contained is generally about 5 to 500 mg per dosage unit form; especially in the form of injection, it is preferred that the aforesaid antibody is contained in about 5 to 100 mg and in about 10 to 250 mg for the other forms.

Each composition described above may further contain other active components unless formulation causes any adverse interaction with the antibody described above.

The prophylactic/therapeutic agent comprising the antibody of the present invention for the diseases described above is low toxic and can be administered to human or mammals (e.g., rats, rabbits, sheep, swine, bovine, cats, dogs, monkeys, etc.) orally or parenterally (e.g., intravenously) in the form of liquid preparation as it is or as a pharmaceutical composition of appropriate dosage form. The dose may vary depending upon subject to be administered, target disease, conditions, route of administration, etc. For example, when the agent is used for the purpose of treating, e.g., Alzheimer's disease in an adult, it is advantageous to administer the antibody of the present invention by way of injection normally in a single dose of about 0.01 to about 20 mg/kg body weight, preferably about 0.1 to about 10 mg/kg body weight, and more preferably about 0.1 to about 5 mg/kg body weight, approximately 1 to 5 times per day. In other parenteral administration and oral administration, the agent can be administered in a dose corresponding to the dose given above. When the condition is especially severe, the dose may be increased according to the condition.

The antibody of the present invention is also useful, for example, as a diagnostic agent for neurodegenerative diseases [e.g., Alzheimer's diseases (familial Alzheimer's disease, juvenile Alzheimer's disease, sporadic Alzheimer's disease, etc.), Parkinson's disease, Down's syndrome, amyotrophic lateral sclerosis, prion disease, Creutzfeldt-Jakob disease, Huntington's chorea, diabetic neuropathy, multiple sclerosis, etc.], diabetes and its complications (e.g., diabetic nephropathy, diabetic neuropathy, diabetic retinopathy, diabetic foot, arteriosclerosis, hypertension, hyperlipemia, etc.), or the like.

### (6) DNA transgenic animal

The present invention provides a non-human mammal bearing DNA encoding the protein of the present invention, which is exogenous (hereinafter abbreviated as the exogenous DNA of the present invention) or its variant DNA (sometimes simply referred to as the exogenous variant DNA of the present invention).

That is, the present invention provides:
(1) A non-human mammal bearing the exogenous DNA of the present invention or its variant DNA;
(2) The mammal according to (1), wherein the non-human mammal is a rodent;
(3) The mammal according to (2), wherein the rodent is mouse or rat; and,
(4) A recombinant vector containing the exogenous DNA of the present invention or its variant DNA and capable of expressing in a mammal; etc.

The non-human mammal bearing the exogenous DNA of the present invention or its variant DNA (hereinafter simply referred to as the DNA transgenic animal of the present invention) can be prepared by transfecting a desired DNA into an unfertilized egg, a fertilized egg, a spermatozoon, a germinal cell containing a primordial germinal cell thereof, or the like, preferably in the embryogenic stage in the development of a non-human mammal (more preferably in the single cell or fertilized cell stage and generally before the 8-cell phase), by standard means, such as the calcium phosphate method, the electric pulse method, the lipofection method, the agglutination method, the microinjection method, the particle gun method, the DEAE-dextran method, etc. Also, it is possible to transfect the exogenous DNA of the present invention into a somatic cell, a living organ, a tissue cell, or the like by the DNA transfection methods, and utilize the transformant for cell culture, tissue culture, etc. In addition, these cells may be fused with the above-described germinal cell by a publicly known cell fusion method to prepare the DNA transgenic animal of the present invention.

Examples of the non-human mammal that can be used include bovine, swine, sheep, goat, rabbits, dogs, cats, guinea pigs, hamsters, mice, rats, etc. Above all, preferred are rodents, especially mice (e.g., C57B1/6 strain, DBA2 strain, etc. for a pure line and for a cross line, B6C3F₁ strain, BDF₁ strain B6D2F₁ strain, BALB/c strain, ICR strain, etc.), rats (Wistar, SD, etc.) or the like, since they are relatively short in ontogeny and life cycle from a standpoint of creating model animals for human disease.

"Mammals" in a recombinant vector that can be expressed in the mammals include the aforesaid non-human mammals, human, etc.

The exogenous DNA of the present invention refers to the DNA of the present invention that is once isolated/extracted from mammals, not the DNA of the present invention inherently possessed by the non-human mammals.

The mutant DNA of the present invention includes mutants resulting from variation (e.g., mutation, etc.) in the base sequence of the original DNA of the present invention, specifically DNAs resulting from base addition, deletion, substitution with other bases, etc. and further including abnormal DNA.

The abnormal DNA is intended to mean DNA that expresses the protein of the present invention which is abnormal and exemplified by the DNA, etc. that expresses a protein for suppressing the function of the protein of the present invention which is normal.

The exogenous DNA of the present invention may be any one of those derived from a mammal of the same species as, or a different species from, the mammal as the target animal. In transfecting the DNA of the present invention into the target animal, it is generally advantageous to use the DNA as a DNA construct in which the DNA is ligated downstream a promoter capable of expressing the DNA in the target animal. For example, in the case of transfecting the human DNA of the present invention, a DNA transgenic mammal that expresses the DNA of the present invention to a high level, can be prepared by microinjecting a DNA construct (e.g., vector, etc.) ligated with the human DNA of the present invention into a fertilized egg of the target non-human mammal downstream various promoters which are capable of expressing the DNA derived from various mammals (e.g., rabbits, dogs, cats, guinea pigs, hamsters, rats, mice, etc.) bearing the DNA of the present invention highly homologous to the human DNA.

As expression vectors for the protein of the present invention, there are Escherichia coli-derived plasmids, Bacillus subtilis-derived plasmids, yeast-derived plasmids, bacteriophages such as ? phage, retroviruses such as Moloney leukemia virus, etc., and animal viruses such as vaccinia virus, baculovirus, etc. Of these vectors, Escherichia coli-derived plasmids, Bacillus subtilis-derived plasmids, or yeast-derived plasmids, etc. are preferably used.

Examples of these promoters for regulating the DNA expression described above include (i) promoters for DNA derived from viruses (e.g., simian virus, cytomegalovirus, Moloney leukemia virus, JC virus, breast cancer virus, poliovirus, etc.), and (ii) promoters derived from various mammals (human, rabbits, dogs, cats, guinea pigs, hamsters, rats, mice, etc.), for example, promoters of albumin, insulin II, uroplakin II, elastase, erythropoietin, endothelin, muscular creatine kinase, glial fibrillary acidic protein, glutathione S-transferase, platelet-derived growth factor β, keratins K1, K10 and K14, collagen types I and II, cyclic AMP-dependent protein kinase βI subunit, dystrophin, tartarate-resistant alkaline phosphatase, atrial natriuretic factor, endothelial receptor tyrosine kinase (generally abbreviated as Tie2), sodium-potassium adenosine triphosphorylase (Na,K-ATPase), neurofilament light chain, metallothioneins I and IIA, metalloproteinase I tissue inhibitor, MHC class I antigen (H-2L), H-ras, renin, dopamine β-hydroxylase, thyroid peroxidase (TPO), protein chain elongation factor 1α (EF-1α), β actin, α and β myosin heavy chains, myosin light chains 1 and 2, myelin base protein, thyroglobulins, Thy-1, immunoglobulins, H-chain variable region (VNP), serum amyloid component P, myoglobin, troponin C, smooth muscle α actin, preproencephalin A, vasopressin, etc. Among them, cytomegalovirus promoters, human protein elongation factor 1α (EF-1α) promoters, human and fowl β actin promoters, etc., which are capable of high expression in the whole body are preferred.

Preferably, the vectors described above have a sequence that terminates the transcription of the desired messenger RNA in the DNA transgenic animal (generally termed a terminator); for example, a sequence of each DNA derived from viruses and various mammals, and SV40 terminator of the simian virus and the like are preferably used.

In addition, for the purpose of enhancing the expression of the desired exogenous DNA to a higher level, the splicing signal and enhancer region of each DNA, a portion of the intron of an eukaryotic DNA may also be ligated at the 5' upstream of the promoter region, or between the promoter region and the translational region, or at the 3' downstream of the translational region, depending upon purposes.

The translational region for the normal protein of the present invention can be obtained using as a starting material the entire genomic DNA or its portion of liver, kidney, thyroid cell or fibroblast origin from human or various mammals (e.g., rabbits, dogs, cats, guinea pigs, hamsters, rats, mice, etc.) or of various commercially available genomic DNA libraries, or using cDNA prepared by a publicly known method from RNA of liver, kidney, thyroid cell or fibroblast origin as a starting material. Also, an exogenous abnormal DNA can produce the translational region through variation of the translational region of normal protein obtained from the cells or tissues described above by point mutagenesis.

The translational region can be prepared by a conventional DNA engineering technique, in which the DNA is ligated downstream the aforesaid promoter and if desired, upstream the translation termination site, as a DNA construct capable of being expressed in the transgenic animal.

The exogenous DNA of the present invention is transfected at the fertilized egg cell stage in a manner such that the DNA is certainly present in all the germinal cells and somatic cells of the target mammal. The fact that the exogenous DNA of the present invention is present in the germinal cells of the animal prepared by DNA transfection means that all offspring of the prepared animal will maintain the exogenous DNA of the present invention in all of the germinal cells and somatic cells thereof. The offspring of the animal that inherits the exogenous DNA of the present invention also have the exogenous DNA of the present invention in all of the germinal cells and somatic cells thereof.

The non-human mammal in which the normal exogenous DNA of the present invention has been transfected can be passaged as the DNA-bearing animal under ordinary rearing environment, by confirming that the exogenous DNA is stably retained by crossing.

By the transfection of the exogenous DNA of the present invention at the fertilized egg cell stage, the DNA is retained to be excess in all of the germinal and somatic cells. The fact that the exogenous DNA of the present invention is excessively present in the germinal cells of the prepared animal after transfection means that the DNA of the present invention is excessively present in all of the germinal cells and somatic cells thereof. The offspring of the animal that inherits the exogenous DNA of the present invention have excessively the DNA of the present invention in all of the germinal cells and somatic cells thereof.

It is possible to obtain homozygotic animals having the transfected DNA in both homologous chromosomes and breed male and female of the animal so that all the progeny have this DNA in excess.

In a non-human mammal bearing the normal DNA of the present invention, the normal DNA of the present invention has expressed at a high level, and may eventually develop hyperfunction in the function of the protein of the present invention by accelerating the function of endogenous normal DNA. Therefore, the animal can be utilized as a pathologic model animal for such a disease. For example, using the normal DNA transgenic animal of the present invention, it is possible to elucidate the mechanism of hyperfunction in the function of the protein of the present invention and the pathological mechanism of the disease associated with the protein of the present invention and to investigate how to treat these diseases.

Furthermore, a mammal transfected with the exogenous normal DNA of the present invention exhibits a symptom of increasing the protein of the present invention liberated. Thus, the animal is usable for screening test of prophylactic/therapeutic agents for diseases associated with the protein of the present invention, for example, the prophylactic/therapeutic agent for neurodegenerative diseases [e.g., Alzheimer's diseases (familial Alzheimer's disease, juvenile Alzheimer's disease, sporadic Alzheimer's disease, etc.), Parkinson's disease, Down's syndrome, amyotrophic lateral sclerosis, prion disease, Creutzfeldt-Jakob disease, Huntington's chorea, diabetic neuropathy, multiple sclerosis, etc.], diabetes and its complications (e.g., diabetic nephropathy, diabetic neuropathy, diabetic retinopathy, diabetic foot, arteriosclerosis, hypertension, hyperlipemia, etc.), or the like.

On the other hand, a non-human mammal having the exogenous abnormal DNA of the present invention can be passaged under normal breeding conditions as the DNA-bearing animal by confirming stable retention of the exogenous DNA via crossing. Furthermore, the exogenous DNA of interest can be utilized as a starting material by inserting the DNA into the plasmid described above. The DNA construct with a promoter can be prepared by conventional DNA engineering techniques. The transfection of the abnormal DNA of the present invention at the fertilized egg cell stage is preserved to be present in all of the germinal and somatic cells of the target mammal. The fact that the abnormal DNA of the present invention is present in the germinal cells of the animal after DNA transfection means that all of the offspring of the prepared animal have the abnormal DNA of the present invention in all of the germinal and somatic cells. Such an offspring that passaged the exogenous DNA of the present invention will have the abnormal DNA of the present invention in all of the germinal and somatic cells. A homozygous animal having the introduced DNA on both of homologous chromosomes can be acquired, and by crossing these male and female animals, all the offspring can be bred to retain the DNA.

In a non-human mammal bearing the abnormal DNA of the present invention, the abnormal DNA of the present invention has expressed to a high level, and may eventually develop the function inactive type inadaptability to the protein of the present invention by inhibiting the functions of endogenous normal DNA. Therefore, the animal can be utilized as a pathologic model animal for such a disease. For example, using the abnormal DNA transgenic animal of the present invention, it is possible to elucidate the mechanism of the function inactive type inadaptability to the protein of the present invention and the pathological mechanism of the disease associated with the protein of the present invention and to investigate how to treat the disease.

More specifically, the transgenic animal of the present invention expressing the abnormal DNA of the present invention at a high level is expected to serve as an experimental model to elucidate the mechanism of the functional inhibition (dominant negative effect) of a normal protein by the abnormal protein of the present invention in the function inactive type inadaptability of the protein of the present invention.

Since a mammal bearing the abnormal exogenous DNA of the present invention shows a symptom of increasing the protein of the present invention liberated, the animal is also expected to serve for screening test of prophylactic/therapeutic agents for the function inactive type inadaptability of the protein of the present invention, e.g., prophylactic/therapeutic agents for neurodegenerative diseases [e.g., Alzheimer's diseases (familial Alzheimer's disease, juvenile Alzheimer's disease, sporadic Alzheimer's disease, etc.), Parkinson's disease, Down's syndrome, amyotrophic lateral sclerosis, prion disease, Creutzfeldt-Jakob disease, Huntington's chorea, diabetic neuropathy, multiple sclerosis, etc.], diabetes and its complications (e.g., diabetic nephropathy, diabetic neuropathy, diabetic retinopathy, diabetic foot, arteriosclerosis, hypertension, hyperlipemia, etc.), or the like.

Other potential applications of two kinds of the DNA transgenic animals of the present invention described above further include:
(i) Use as a cell source for tissue culture;
(ii) Elucidation of the relation to a peptide that is specifically expressed or activated by the protein of the present invention, by direct analysis of DNA or RNA in tissues of the DNA transgenic animal of the present invention or by analysis of the peptide tissues expressed by the DNA;
(iii) Research on the function of cells derived from tissues that are usually cultured only with difficulty, using cells in tissues bearing the DNA cultured by a standard tissue culture technique;
(iv) Screening a drug that enhances the functions of cells using the cells described in (iii) above; and, (v) Isolation and purification of the variant protein of the present invention and preparation of an antibody thereto; etc.

Furthermore, clinical conditions of a disease associated wit the protein of the present invention, including the function inactive type inadaptability to the protein of the present invention can be determined by using the DNA transgenic animal of the present invention. Also, pathological findings on each organ in a disease model associated with the protein of the present invention can be obtained in more detail, leading to the development of a new method for treatment as well as the research and therapy of any secondary diseases associated with the disease.

It is also possible to obtain a free DNA-transfected cell by withdrawing each organ from the DNA transgenic animal of the present invention, mincing the organ and degrading with a proteinase such as trypsin, etc., followed by establishing the line of culturing or cultured cells. Furthermore, the DNA transgenic animal of the present invention can serve to identify cells capable of producing the protein of the present invention, and to study in association with apoptosis, differentiation or propagation or on the mechanism of signal transduction in these properties to inspect any abnormality therein. Accordingly, the DNA transgenic animal can provide an effective research material for the protein of the present invention and for investigation of the function and effect thereof.

To develop a drug for the treatment of diseases associated with the protein of the present invention, including the function inactive type inadaptability to the protein of the present invention, using the DNA transgenic animal of the present invention, an effective and rapid method for screening can be provided by using the method for inspection and the method for quantification, etc. described above. It is also possible to investigate and develop a method for DNA therapy for the treatment of diseases associated with the protein of the present invention, using the DNA transgenic animal of the present invention or a vector capable of expressing the exogenous DNA of the present invention.

### (7) Knockout animal

The present invention provides a non-human mammal embryonic stem cell bearing the DNA of the present invention inactivated and a non-human mammal deficient in expressing the DNA of the present invention.

Thus, the present invention provides:
(1) A non-human mammal embryonic stem cell in which the DNA of the present invention is inactivated;
(2) The embryonic stem cell according to (1), wherein the DNA is inactivated by introducing a reporter gene (e.g., β-galactosidase gene derived from Escherichia coli);
(3) The embryonic stem cell according to (1), which is resistant to neomycin;
(4) The embryonic stem cell according to (1), wherein the non-human mammal is a rodent;
(5) The embryonic stem cell according to (4), wherein the rodent is mouse;
(6) A non-human mammal deficient in expressing the DNA of the present invention, wherein the DNA is inactivated;
(7) The non-human mammal according to (6), wherein the DNA is inactivated by inserting a reporter gene (e.g., β-galactosidase derived from Escherichia coli) therein and the reporter gene is capable of being expressed under control of a promoter for the DNA of the present invention;
(8) The non-human mammal according to (6), which is a rodent;
(9) The non-human mammal according to (8), wherein the rodent is mouse; and,
(10) A method of screening a compound that promotes or inhibits (preferably inhibits) the promoter activity to the DNA of the present invention, which comprises administering a test compound to the mammal of (7) and detecting expression of the reporter gene.

The non-human mammal embryonic stem cell in which the DNA of the present invention is inactivated refers to a non-human mammal embryonic stem cell that suppresses the ability of the non-human mammal to express the DNA by artificially mutating the DNA of the present invention, or the DNA has no substantial ability to express the protein of the present invention (hereinafter sometimes referred to as the knockout DNA of the present invention) by substantially inactivating the activities of the protein of the present invention encoded by the DNA (hereinafter merely referred to as ES cell).

As the non-human mammal, the same examples as described above apply.

Techniques for artificially mutating the DNA of the present invention include deletion of a part or all of the DNA sequence and insertion of or substitution with other DNA, by genetic engineering. By these variations, the knockout DNA of the present invention may be prepared, for example, by shifting the reading frame of a codon or by disrupting the function of a promoter or exon.

Specifically, the non-human mammal embryonic stem cell in which the DNA of the present invention is inactivated (hereinafter merely referred to as the ES cell with the DNA of the present invention inactivated or the knockout ES cell of the present invention) can be obtained by, for example, isolating the DNA of the present invention that the desired non-human mammal possesses, inserting a DNA fragment having a DNA sequence constructed by inserting a drug resistant gene such as a neomycin resistant gene or a hygromycin resistant gene, or a reporter gene such as lacZ (β-galactosidase gene) or cat (chloramphenicol acetyltransferase gene), etc. into its exon site thereby to disable the functions of exon, or integrating to a chromosome of the target animal by, e.g., homologous recombination, a DNA sequence that terminates gene transcription (e.g., polyA additional signal, etc.) in the intron between exons, thus inhibiting the synthesis of complete messenger RNA and eventually destroying the gene (hereinafter simply referred to as a targeting vector). The thus-obtained ES cells to the southern hybridization analysis with a DNA sequence on or near the DNA of the present invention as a probe, or to PCR analysis with a DNA sequence on the targeting vector and another DNA sequence near the DNA of the present invention which is not included in the targeting vector as primers, to select the knockout ES cell of the present invention.

The parent ES cells to inactivate the DNA of the present invention by homologous recombination, etc. may be of a strain already established as described above, or may originally be established in accordance with a modification of the known method by Evans and Kaufman described above. For example, in the case of mouse ES cells, currently it is common practice to use ES cells of the 129 strain. However, since their immunological background is obscure, the C57BL/6 mouse or the BDF₁ mouse (F₁ between C57BL/6 and DBA/2), wherein the low ovum availability per C57BL/6 in the C57BL/6 mouse has been improved by crossing with DBA/2, may be preferably used, instead of obtaining a pure line of ES cells with the clear immunological genetic background and for other purposes. The BDF₁ mouse is advantageous in that, when a pathologic model mouse is generated using ES cells obtained therefrom, the genetic background can be changed to that of the C57BL/6 mouse by back-crossing with the C57BL/6 mouse, since its background is of the C57BL/6 mouse, as well as being advantageous in that ovum availability per animal is high and ova are robust.

In establishing ES cells, blastocytes at 3.5 days after fertilization are commonly used. In the present invention, embryos are preferably collected at the 8-cell stage, after culturing until the blastocyte stage; the embryos are used to efficiently obtain a large number of early stage embryos.

Although the ES cells used may be of either sex, male ES cells are generally more convenient for generation of a germ cell line chimera. It is also desirable that sexes are identified as soon as possible to save painstaking culture time.

Methods for sex identification of the ES cell include the method in which a gene in the sex-determining region on the Y-chromosome is amplified by the PCR process and detected. When this method is used, one colony of ES cells (about 50 cells) is sufficient for sex-determination analysis, which karyotype analysis, for example G-banding method, requires about 10⁶ cells; therefore, the first selection of ES cells at the early stage of culture can be based on sex identification, and male cells can be selected early, which saves a significant amount of time at the early stage of culture.

Also, second selection can be achieved by, for example, confirmation of the number of chromosomes by the G-banding method. It is usually desirable that the chromosome number of the obtained ES cells be 100% of the normal number. However, when it is difficult to obtain the cells having the normal number of chromosomes due to physical operations, etc. in the cell establishment, it is desirable that the ES cell is again cloned to a normal cell (e.g., in a mouse cell having the number of chromosomes being 2n = 40) after knockout of the gene of the ES cells.

Although the embryonic stem cell line thus obtained shows a very high growth potential, it must be subcultured with great care, since it tends to lose its ontogenic capability. For example, the embryonic stem cell line is cultured at about 37°C in a carbon dioxide incubator (preferably 5% carbon dioxide and 95% air, or 5% oxygen, 5% carbon dioxide and 90% air) in the presence of LIF (1 to 10000 U/ml) on appropriate feeder cells such as STO fibroblasts, treated with a trypsin/EDTA solution (normally 0.001 to 0.5% trypsin/0.1 to about 5 mM EDTA, preferably about 0.1 % trypsin/1 mM EDTA) at the time of passage to obtain separate single cells, which are then plated on freshly prepared feeder cells. This passage is normally conducted every 1 to 3 days; it is desirable that cells be observed at the passage and cells found to be morphologically abnormal in culture, if any, be abandoned.

Where ES cells are allowed to reach a high density in mono-layers or to form cell aggregates in suspension under appropriate conditions, it is possible to differentiate the ES cells to various cell types, for example, pariental and visceral muscles, cardiac muscle or the like [M. J. Evans and M. H. Kaufman, Nature, 292, 154, 1981; G. R. Martin, Proc. Natl. Acad. Sci. U.S.A., 78, 7634, 1981; T. C. Doetschman et al., Journal of Embryology Experimental Morphology, 87, 27, 1985]. The cells deficient in expression of the DNA of the present invention, which are obtained from the differentiated ES cells of the present invention, are useful for cytological study of the protein of the present invention in vitro.

The non-human mammal deficient in expression of the DNA of the present invention can be identified from a normal animal by measuring the mRNA level in the subject animal by a publicly known method, and indirectly comparing the degrees of expression.

As the non-human mammal, the same examples given above apply.

With respect to the non-human mammal deficient in expression of the DNA of the present invention, the DNA of the present invention can be knockout by transfecting a targeting vector, prepared as described above, to mouse embryonic stem cells or mouse oocytes, and conducting homologous recombination in which a targeting vector DNA sequence, wherein the DNA of the present invention is inactivated by the transfection, is replaced with the DNA of the present invention on a chromosome of a mouse embryonic stem cell or mouse embryo.

The knockout cells with the disrupted DNA of the present invention can be identified by the southern hybridization analysis using as a probe a DNA fragment on or near the DNA of the present invention, or by the PCR analysis using as primers a DNA sequence on the targeting vector and another DNA sequence at the proximal region of other than the DNA of the present invention derived from mouse used in the targeting vector. When non-human mammal stem cells are used, a cell line wherein the DNA of the present invention is inactivated by homologous recombination is cloned; the resulting clones are injected to, e.g., a non-human mammalian embryo or blastocyst, at an appropriate stage such as the 8-cell stage. The resulting chimeric embryos are transplanted to the uterus of the pseudopregnant non-human mammal. The resulting animal is a chimeric animal constructed with both cells having the normal locus of the DNA of the present invention and those having an artificially mutated locus of the DNA of the present invention.

When some germ cells of the chimeric animal have a mutated locus of the DNA of the present invention, an individual, which entire tissue is composed of cells having a mutated locus of the DNA of the present invention can be selected from a series of offspring obtained by crossing between such a chimeric animal and a normal animal, e.g., by coat color identification, etc. The individuals thus obtained are normally deficient in heterozygous expression of the protein of the present invention. The individuals deficient in homozygous expression of the protein of the present invention can be obtained from offspring of the intercross between those deficient in heterozygous expression of the protein of the present invention.

When an oocyte is used, a DNA solution may be injected, e.g., into the prenucleus by microinjection thereby to obtain a transgenic non-human mammal having a targeting vector introduced in its chromosome. From such transgenic non-human mammals, those having a mutation at the locus of the DNA of the present invention can be obtained by selection based on homologous recombination.

As described above, the individuals in which the DNA of the present invention is knockout permit passage rearing under ordinary rearing conditions, after the individuals obtained by their crossing have proven to have been knockout.

Furthermore, the genital system may be obtained and retained by conventional methods. That is, by crossing male and female animals each having the inactivated DNA, homozygote animals having the inactivated DNA in both loci can be obtained. The homozygotes thus obtained may be reared so that one normal animal and two or more homozygotes are produced from a mother animal to efficiently obtain such homozygotes. By crossing male and female heterozygotes, homozygotes and heterozygotes having the inactivated DNA are proliferated and passaged.

The non-human mammal embryonic stem cell, in which the DNA of the present invention is inactivated, is very useful for preparing a non-human mammal deficient in expression of the DNA of the present invention.

Since the non-human mammal, in which the DNA of the present invention is inactivated, lacks various biological activities derived from the protein of the present invention, such an animal can be a disease model suspected of inactivated biological activities of the protein of the present invention and thus, offers an effective study to investigate the causes for and therapy for these diseases.

### (7a) Method of screening the compound having therapeutic/prophylactic effects on diseases caused by deficiency, damages, etc. of the DNA of the present invention

The non-human mammal deficient in expression of the DNA of the present invention can be employed for screening the compound having therapeutic/prophylactic effects on diseases caused by deficiency, damages, etc. of the DNA of the present invention.

That is, the present invention provides a method of screening the compound or its salts having therapeutic/prophylactic effects on diseases caused by deficiency, damages, etc. of the DNA of the present invention, for example, neurodegenerative diseases [e.g., Alzheimer's diseases (familial Alzheimer's disease, juvenile Alzheimer's disease, sporadic Alzheimer's disease, etc.), Parkinson's disease, Down's syndrome, amyotrophic lateral sclerosis, prion disease, Creutzfeldt-Jakob disease, Huntington's chorea, diabetic neuropathy, multiple sclerosis, etc.], diabetes and its complications (e.g., diabetic nephropathy, diabetic neuropathy, diabetic retinopathy, diabetic foot, arteriosclerosis, hypertension, hyperlipemia, etc.), or the like, which comprises administering a test compound to a non-human mammal deficient in expression of the DNA of the present invention and observing/measuring a change occurred in the animal.

As the non-human mammal deficient in expression of the DNA of the present invention, which can be employed for the screening method, the same examples as described above apply.

Examples of the test compound include peptides, proteins, non-peptide compounds, synthetic compounds, fermentation products, cell extracts, plant extracts, animal tissue extracts, blood plasma, etc. These compounds may be novel compounds or publicly known compounds.

Specifically, the non-human mammal deficient in expression of the DNA of the present invention is treated with a test compound, comparison is made with an intact animal for control and a change in each organ, tissue, disease conditions, etc. of the animal is used as an indicator to assess the therapeutic/prophylactic effects of the test compound.

For treating an animal to be tested with a test compound, for example, oral administration, intravenous injection, etc. are applied, and the treatment can be appropriately selected depending on conditions of the test animal, properties of the test compound, etc. Furthermore, a dose of the test compound to be administered can be appropriately chosen depending on the administration route, nature of the test compound, etc.

For screening of the compound having therapeutic/prophylactic effects on, e.g., neurodegenerative diseases [e.g., Alzheimer's diseases (familial Alzheimer's disease, juvenile Alzheimer's disease, sporadic Alzheimer's disease, etc.), Parkinson's disease, Down's syndrome, amyotrophic lateral sclerosis, prion disease, Creutzfeldt-Jakob disease, Huntington's chorea, diabetic neuropathy, multiple sclerosis, etc.], diabetes and its complications (e.g., diabetic nephropathy, diabetic neuropathy, diabetic retinopathy, diabetic foot, arteriosclerosis, hypertension, hyperlipemia, etc.), or the like, a test compound is administered to the non-human mammal deficient in expression of the DNA of the present invention. Differences in the number of neuronal cell death, the level of various proteins, etc. from the group administered with no test compound are observed in the tissues described above with passage of time.

In the screening method, when a test compound is administered to a test animal and the disease conditions of the test animal are improved by at least about 10%, preferably at least about 30% and more preferably at least about 50%, the test compound can be selected as the compound having therapeutic/prophylactic effects on the diseases described above.

The compound obtained using the above screening method is a compound selected from the test compounds described above and exhibits therapeutic/prophylactic effects on diseases caused by deficiencies, damages, etc. of the protein of the present invention. Therefore, the compound can be employed as a safe and low toxic prophylactic/ therapeutic drugs for these diseases. Furthermore, compounds derived from the compound obtained by the screening described above may also be used as well.

The compound obtained by the screening method above may form salts, and may be used in the form of salts with physiologically acceptable acids (e.g., inorganic acids, organic acids, etc.) or bases (e.g., alkali metal salts), preferably in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

A pharmaceutical comprising the compound obtained by the above screening method or salts thereof can be manufactured in a manner similar to the method for preparing the pharmaceutical comprising the protein of the present invention described hereinabove.

Since the pharmaceutical preparation thus obtained is safe and low toxic, it can be administered to human or mammal (e.g., rat, mouse, guinea pig, rabbit, sheep, swine, bovine, horse, cat, dog, monkey, etc.).

The dose of the compound or its salt may vary depending upon target disease, subject to be administered, route of administration, etc. For example, when the compound is orally administered, the compound is administered to the adult patient with Alzheimer's disease (as 60 kg body weight) generally in a dose of about 0.1 to 100 mg, preferably about 1.0 to 50 mg and more preferably about 1.0 to 20 mg. In parenteral administration, a single dose of the compound may vary depending upon subject to be administered, target disease, etc. When the compound is administered to the adult patient with Alzheimer's disease (as 60 kg body weight) in the form of an injectable preparation, it is advantageous to administer the compound in a single dose of about 0.01 to about 30 mg, preferably about 0.1 to about 20 mg and more preferably about 0.1 to about 10 mg a day. For other animal species, the corresponding dose as converted per 60 kg weight can be administered.

### (7b) Method of screening a compound that promotes or inhibits the activity of a promoter to the DNA of the present invention

The present invention provides a method of screening a compound or its salts that promote or inhibit the activity of a promoter to the DNA of the present invention, which comprises administering a test compound to a non-human mammal deficient in expression of the DNA of the present invention and detecting the expression of a reporter gene.

In the screening method described above, an animal in which the DNA of the present invention is inactivated by introducing a reporter gene and the reporter gene is expressed under control of a promoter to the DNA of the present invention is used as the non-human mammal deficient in expression of the DNA of the present invention, which is selected from the aforesaid non-human mammals deficient in expression of the DNA of the present invention.

The same examples of the test compound apply to specific compounds described above.

As the reporter gene, the same specific examples apply to this screening method. Preferably, there are used β-galactosidase (lacZ), soluble alkaline phosphatase gene, luciferase gene and the like.

Since the reporter gene is present under control of a promoter to the DNA of the present invention in the non-human mammal deficient in expression of the DNA of the present invention wherein the DNA of the present invention is substituted with the reporter gene, the activity of the promoter can be detected by tracing the expression of a substance encoded by the reporter gene.

When a part of the DNA region encoding the protein of the present invention is substituted with, e.g., β-galactosidase gene (lacZ) derived from *Escherichia coli*, β-galactosidase is expressed in a tissue where the protein of the present invention should originally be expressed, instead of the protein of the present invention. Thus, the state of expression of the protein of the present invention can be readily observed in vivo of an animal by staining with a reagent, e.g., 5-bromo-4-chloro-3-indolyl-β-galactopyranoside (X-gal) which is substrate for β-galactosidase. Specifically, a mouse deficient in the protein of the present invention, or its tissue section is fixed with glutaraldehyde, etc. After washing with phosphate buffered saline (PBS), the system is reacted with a staining solution containing X-gal at room temperature or about 37°C for approximately 30 minutes to an hour. After the β-galactosidase reaction is terminated by washing the tissue preparation with 1 mM EDTA/PBS solution, the color formed is observed. Alternatively, mRNA encoding lacZ may be detected in a conventional manner.

The compound or salts thereof obtained using the screening method described above are compounds that are selected from the test compounds described above and that promote or inhibit the promoter activity to the DNA of the present invention.

The compound obtained by the screening method above may form salts, and may be used in the form of salts with physiologically acceptable acids (e.g., inorganic acids, etc.) or bases (e.g., alkali metals, etc.) or the like, especially in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

The compound or its salts promoting or inhibiting the promoter activity to the DNA of the present invention can regulate the expression of the protein of the present invention and can regulate the functions of the protein. Thus, the compound or its salt is useful as prophylactic/therapeutic agents for, e.g., neurodegenerative diseases [e.g., Alzheimer's diseases (familial Alzheimer's disease, juvenile Alzheimer's disease, sporadic Alzheimer's disease, etc.), Parkinson's disease, Down's syndrome, amyotrophic lateral sclerosis, prion disease, Creutzfeldt-Jakob disease, Huntington's chorea, diabetic neuropathy, multiple sclerosis, etc.], diabetes and its complications (e.g., diabetic nephropathy, diabetic neuropathy, diabetic retinopathy, diabetic foot, arteriosclerosis, hypertension, hyperlipemia, etc.), or the like.

In addition, compounds derived from the compound obtained by the screening described above may also be used as well.

A pharmaceutical comprising the compound obtained by the above screening method or salts thereof can be manufactured in a manner similar to the method for preparing the pharmaceutical comprising the protein of the present invention described above.

Since the pharmaceutical preparation thus obtained is safe and low toxic, it can be administered to human or mammal (e.g., rat, mouse, guinea pig, rabbit, sheep, swine, bovine, horse, cat, dog, monkey, etc.).

A dose of the compound or salts thereof may vary depending on target disease, subject to be administered, route for administration, etc.; when the compound that inhibits the promoter activity to the DNA of the present invention is orally administered, the compound is administered to the adult patient with Alzheimer's disease (as 60 kg body weight) normally in a daily dose of about 0.1 to 100 mg, preferably about 1.0 to 50 mg and more preferably about 1.0 to 20 mg. In parenteral administration, a single dose of the compound varies depending on subject to be administered, target disease, etc. but when the compound of inhibiting the promoter activity to the DNA of the present invention is administered to the adult patient with Alzheimer's disease (as 60 kg body weight) in the form of injectable preparation, it is advantageous to administer the compound intravenously to the patient in a daily dose of about 0.01 to about 30 mg, preferably about 0.1 to about 20 mg and more preferably about 0.1 to about 10 mg. For other animal species, the corresponding dose as converted per 60 kg weight can be administered.

As stated above, the non-human mammal deficient in expression of the DNA of the present invention is extremely useful for screening the compound or its salt that promotes or inhibits the promoter activity to the DNA of the present invention and, can greatly contribute to elucidation of causes for various diseases suspected of deficiency in expression of the DNA of the present invention and for the development of prophylactic/therapeutic agents for these diseases.

In addition, a so-called transgenic animal (gene transferred animal) can be prepared by using a DNA containing the promoter region of the protein of the present invention, ligating genes encoding various proteins at the downstream and injecting the same into oocyte of an animal. It is thus possible to synthesize the protein therein specifically and study its activity in vivo. When an appropriate reporter gene is ligated to the promoter site described above and a cell line that expresses the gene is established, the resulting system can be utilized as the search system for a low molecular compound having the action of specifically promoting or inhibiting the in vivo productivity of the protein itself of the present invention.

### (8) Prophylactic/therapeutic agents for various diseases associated with the protein of the present invention

The protein of the present invention binds to Akt1 to inhibit the Akt1 activity.

Therefore, where the DNA encoding the protein of the present invention involves any abnormality or is deleted, or where the expression level of the protein of the present invention is reduced, various diseases such as a cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, ovarian cancer, blood tumor, etc.), rheumatic diseases (e.g., chronic articular rheumatism, osteoarthritis, gout, etc.) and the like are developed.

Thus, the protein of the present invention and the DNA of the present invention can be used as prophylactic/therapeutic agents for, e.g., a cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, ovarian cancer, blood tumor, etc.), rheumatic diseases (e.g., chronic articular rheumatism, osteoarthritis, gout, etc.), and the like.

For example, when the activity of the protein of the present invention cannot be expected in a patient sufficiently or normally due to a decrease or deficiency of the protein of the present invention in the body, the role of the protein of the present invention can be exhibited sufficiently or normally, (a) by administering the DNA of the present invention directly to the patient thereby to express the protein of the present invention; (b) by inserting the DNA of the present invention into cells to express the protein of the present invention and transplant the cells to the patient; or (c) by administering the protein of the present invention to the patient; etc.

Where the DNA of the present invention is used as the prophylactic/therapeutic agents described above, the DNA of the present invention is administered alone; alternatively, the DNA is inserted into an appropriate vector such as retrovirus vector, adenovirus vector, adenovirus-associated virus vector, etc. and then administered to human or other warm-blooded animal in a conventional manner. The DNA of the present invention may also be administered as intact DNA, or with pharmacologically acceptable carrier such as adjuvants to assist its uptake by gene gun or through a catheter such as a catheter with a hydrogel.

When the protein of the present invention is used as the prophylactic/therapeutic agents described above, it is preferred to use the protein with a purity of at least 90%, preferably at least 95%, more preferably at least 98% and most preferably at least 99%.

The protein of the present invention can be used orally, for example, in the form of tablets which may be sugar coated if necessary, capsules, elixirs, microcapsules etc., or parenterally in the form of injectable preparations such as a sterile solution or suspension in water or other pharmaceutically acceptable liquid. These preparations can be manufactured by mixing the protein of the present invention with a physiologically acceptable carrier, a flavoring agent, an excipient, a vehicle, an antiseptic agent, a stabilizer, a binder, etc. in a unit dosage form required in a generally accepted manner that is applied to making pharmaceutical preparations. The active ingredient in these preparations is controlled in such a dose that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin and alginic acid, a lubricant such as magnesium stearate, a sweetening agent such as sucrose, lactose and saccharin, a flavoring agent such as peppermint, akamono oil and cherry, and the like. When the unit dosage is in the form of capsules, liquid carriers such as oils and fats may further be used together with the additives described above. A sterile composition for injection may be formulated by conventional procedures used to make pharmaceutical preparations, e.g., by dissolving or suspending the active ingredients in a vehicle such as water for injection with a naturally occurring vegetable oil such as sesame oil and coconut oil, etc. to prepare the pharmaceutical preparations.

Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol or the like), a polyalcohol (e.g., propylene glycol, polyethylene glycol, etc.), a nonionic surfactant (e.g., polysorbate 80TM, HCO-50, etc.), and the like. Examples of the oily medium include sesame oil and soybean oil, which may also be used in combination with a dissolution aid such as benzyl benzoate, benzyl alcohol, etc. The agent may further be formulated with a buffer (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus prepared liquid for injection is normally filled in an appropriate ampoule.

The vector inserted with the DNA of the present invention is prepared into pharmaceutical preparations as described above and provided normally for use parenterally.

Since the thus obtained pharmaceutical preparation is safe and low toxic, the preparation can be administered to warm-blooded animals (e.g., human, rats, mice, guinea pigs, rabbits, chicken, sheep, swine, bovine, horses, cats, dogs, monkeys, chimpanzees, etc.).

The dose of the protein of the present invention varies depending on target disease, subject to be administered, routes for administration, etc. When the protein of the present invention is orally administered for the purpose of treating, e.g., chronic articular rheumatism, the protein, etc. is administered to adult (as 60 kg body weight) in a dose of normally about 0.1 mg to about 100 mg, preferably about 1.0 to about 50 mg, and more preferably about 1.0 to about 20 mg per day. In parenteral administration, the single dose of the protein, etc. varies depending on subject to be administered, target disease, etc. When the protein of the present invention is administered to an adult (as 60 kg body weight) in the form of injection for the purpose of treating, e.g., chronic articular rheumatism, it is advantageous to administer the protein by injecting the protein into the affected site in a daily dose of about 0.01 to about 30 mg, preferably about 0.1 to about 20 mg, and more preferably about 0.1 to about 10 mg. For other animal species, the corresponding dose as converted per 60 kg body weight can be administered.

In the specification and drawings, the codes of bases, amino acids, etc. are denoted in accordance with the IUPAC-IUB Commission on Biochemical Nomenclature or by the common codes in the art, examples of which are shown below. For amino acids that may have the optical isomer, L form is presented unless otherwise indicated.
- DNA :: deoxyribonucleic acid
- cDNA :: complementary deoxyribonucleic acid
- A :: adenine
- T :: thymine
- G :: guanine
- C :: cytosine
- RNA :: ribonucleic acid
- mRNA :: messenger ribonucleic acid
- dATP :: deoxyadenosine triphosphate
- dTTP :: deoxythymidine triphosphate
- dGTP :: deoxyguanosine triphosphate
- dCTP :: deoxycytidine triphosphate
- ATP :: adenosine triphosphate
- EDTA :: ethylenediaminetetraacetic acid
- SDS :: sodium dodecyl sulfate
- Gly :: glycine
- Ala :: alanine
- Val :: valine
- Leu :: leucine
- Ile :: isoleucine
- Ser :: serine
- Thr :: threonine
- Cys :: cysteine
- Met :: methionine
- Glu :: glutamic acid
- Asp :: aspartic acid
- Lys :: lysine
- Arg :: arginine
- His :: histidine
- Phe :: phenylalanine
- Tyr :: tyrosine
- Trp :: tryptophan
- Pro :: proline
- Asn :: asparagine
- Gln :: glutamine
- pGlu :: pyroglutamic acid
- Sec :: selenocysteine

Substituents, protecting groups and reagents generally used in this specification are presented as the codes below.
- Me :: methyl group
- Et :: ethyl group
- Bu :: butyl group
- Ph :: phenyl group
- TC :: thiazolidine-4(R)-carboxamido group
- Tos :: p-toluenesulfonyl
- CHO :: formyl
- Bzl :: benzyl
- Cl₂-Bzl :: 2,6-dichlorobenzyl
- Bom :: benzyloxymethyl
- Z :: benzyloxycarbonyl
- Cl-Z :: 2-chlorobenzyloxycarbonyl
- Br-Z :: 2-bromobenzyl oxycarbonyl
- Boc :: t-butoxycarbonyl
- DNP :: dinitrophenol
- Trt :: trityl
- Bum :: t-butoxymethyl
- Fmoc :: N-9-fluorenyl methoxycarbonyl
- HOBt :: 1-hydroxybenztriazole
- HOOBt :: 3,4-dihydro-3-hydroxy-4-oxo-1,2,3-benzotriazine
- HONB :: 1-hydroxy-5-norbornene-2,3-dicarboxyimide
- DCC:: N,N'-dicyclohexylcarbodiimide

The sequence identification numbers in the sequence listing of the specification indicate the following sequences.

### [SEQ ID NO: 1]

This shows the amino acid sequence of human neuronal cell death inducible putative kinase (NIPK).

### [SEQ ID NO: 2]

This shows the base sequence of DNA encoding human NIPK.

### [SEQ ID NO: 3]

This shows the base sequence of primer used in EXAMPLES 3 and 5.

### [SEQ ID NO: 4]

This shows the base sequence of primer used in EXAMPLE 3.

### [SEQ ID NO: 5]

This shows the base sequence of GenBank Accession No. NM021158.

### [SEQ ID NO: 6]

This shows the amino acid sequence encoded by the base sequence represented by SEQ ID NO: 5.

### [SEQ ID NO: 7]

This shows the base sequence of GenBank Accession No. NM021158, in which A is replaced by G at 251 position.

### [SEQ ID NO: 8]

This shows the amino acid sequence encoded by the base sequence represented by SEQ ID NO: 7.

### [SEQ ID NO: 9]

This shows the base sequence of GenBank Accession No. AAF30480.

### [SEQ ID NO: 10]

This shows the amino acid sequence encoded by the base sequence represented by SEQ ID NO: 9.

### [SEQ ID NO: 11]

This shows the base sequence of GenBank Accession No. AAH76218.

### [SEQ ID NO: 12]

This shows the amino acid sequence encoded by the base sequence represented by SEQ ID NO: 11.

### [SEQ II7 NO: 13]

This shows the base sequence of GenBank Accession No. AAI58064.

### [SEQ ID NO: 14]

This shows the amino acid sequence encoded by the base sequence represented by SEQ ID NO: 13.

### [SEQ ID NO: 15]

This shows the base sequence of GenBank Accession No. AAI59850.

### [SEQ ID NO: 16]

This shows the amino acid sequence encoded by the base sequence represented by SEQ ID NO: 15.

### [SEQ ID NO: 17]

This shows the base sequence of GenBank Accession No. AAS06709. [SEQ ID NO: 18]

This shows the amino acid sequence encoded by the base sequence represented by SEQ ID NO: 17.

### [SEQ ID NO: 19]

This shows the base sequence of GenBank Accession No. AF250311.

### [SEQ ID NO: 20]

This shows the amino acid sequence encoded by the base sequence represented by SEQ ID NO: 19.

### [SEQ ID NO: 21]

This shows the base sequence of primer used in EXAMPLE 4.

### [SEQ ID NO: 22]

This shows the base sequence of primer used in EXAMPLE 4.

### [SEQ ID NO: 23]

This shows the base sequence of primer used in EXAMPLE 5.

### [SEQ ID NO: 24]

This shows the base sequence of primer used in EXAMPLE 5.

### [SEQ ID NO: 25]

This shows the base sequence of primer used in EXAMPLE 5.

Hereinafter, the present invention will be described more specifically with reference to EXAMPLES but is not deemed to be limited thereto.

### EXAMPLE 1

In order to clarify a cluster of expression-shifted genes responsive to endoplasmic reticulum stress, the following experiment was carried out.

Rat primary nerve cells prepared from CD rat (Charles River Japan, Inc.) of TP-17 were suspended in B27-supplemented Neurobasal medium (NB medium:
GIBCO) charged in a type I collagen-coated 24-well plate and then plated at 250,000 cells/well, followed by culturing for 3 days.
   (i) Tunicamycin (Tnc) was added to the culture cells above in a final concentration of 100 nM, (ii) thapsigargin (Thap) was added to the culture cells above in a final concentration of 2 nM, or (iii) the culture cells described above were cultured in glucose-free B27-suplemented DMEM medium with 2 medium changes and then 2-deoxyglucose (2DG) was added in 3 mM. The total RNA was extracted from the cells 4, 8 and 24 hours, respectively, in accordance with the operating manual using RNeasy Mini kit (Qiagen). Cells added to the respective media (drug-free cells) in place of the drugs above (Tnc, Thap and 2DG) were used for control. Using the oligonucleotide microarray (Rat Genome U34A; Affymetrix Corp.), gene expression analysis was conducted on these materials.

The experimental method was carried out in accordance with the expression analysis technical manual of Affymetrix Corp. Gene expression profiles were compared between the cells stimulated with each drug and the cells with no drug. The results indicate that marked expression of the neuronal cell death inducible putative kinase (NIPK) gene was induced by these respective endoplasmic reticulum stresses [(i), (ii) and (iii) described above] (TABLE 1).

**[TABLE 1]**

| Cell | Gene Expression Level^{a} |
|---|---|
| 4 hours after Tnc stimulation | 0.41 |
| 4 hours after non-stimulation | ND |
| 8 hours after Tnc stimulation | 1.64 |
| 8 hours after non-stimulation | ND |
| 24 hours after Tnc | 1.32 |
| 24 hours after non-stimulation | ND |
| 4 hours after Thap stimulation | 0.93 |
| 4 hours after non-stimulation | ND |
| 8 hours after Thap | 2.70 |
| 4 hours after non-stimulation | ND |
| 24 hours after Thap stimulation | 1.64 |
| 24 hours after non-stimulation | ND |
| 4 hours after 2DG addition | 0.72 |
| 4 hours after non-stimulation | ND |
| 8 hours after 2DG addition | 1.79 |
| 8 hours after non-stimulation | ND |
| 24 hours after 2DG addition | 2.45 |
| 24 hours after non-stimulation | ND |
| a: The medial value for the expression level of genes showing the presence in which expression was detected by oligonucleotide microarray, was taken as 1 to standardize the gene expression level. | |
| ND: not detected | |

### EXAMPLE 2

In order to examine if β amyloid stimulation would affect the expression of NIPK gene with enhanced expression by endoplasmic reticulum stress, gene expression analysis was performed as in EXAMPLE 1, using cells stimulated with β amyloid.

Rat primary nerve cells prepared as in EXAMPLE 1 were suspended in N2-supplemented DMEM medium (GIBCO) and then plated at 250,000 cells/well, followed by incubation for 4 days. After the incubation, β amyloid suspended in the medium above in a final concentration of 25 µM was added to the medium. As in EXAMPLE 1, the total RNA was extracted from the cells 4, 8 and 24 hours after the addition and provided for gene analysis. For control, the cells to which the medium described above was added instead of β amyloid were used. Expression of NIPK was more enhanced in the cells stimulated with β, when compared to control (TABLE 2).

**[TABLE 2]**

| Cell | Gene Expression Level^{a} |
|---|---|
| 4 hours after β amyloid stimulation | ND |
| 4 hours after non-stimulation | ND |
| 8 hours after β amyloid stimulation | ND |
| 8 hours after non-stimulation | ND |
| 24 hours after β amyloid stimulation | 0.34 |
| 24 hours after non-stimulation | ND |
| a: The medial value for the expression level of genes showing the presence in which expression was detected by oligonucleotide microarray, was taken as 1 to standardize the gene expression level. | |

### EXAMPLE 3

In order to examine if NIPK would affect cell death, the NIPK gene expression cells were prepared using transient transfection and provided for the cell death induction system.

Thapsigargin was added to human neuroblastoma SK-N-AS cells (purchased from ATCC) in a final concentration of 500 nM, followed by incubation for 8 hours. After the incubation, the total RNA was extracted using ISOGEN (Nippon Gene). Using as a template the total RNA obtained, reverse transcription was carried out on RNA PCR kit (TAKARA). For amplification of human NIPK gene, PCR was carried out with synthetic primers (SEQ ID NO: 3 and SEQ ID NO: 4) and Pfu turbo (Stratagene) as an enzyme under the conditions (1) - (5) below to obtain the specific PCR product.
(1) 95°C for 1 minute
(2) 95°C for 20 seconds - 68°C for 15 seconds - 72°C for 1 minute, 3 times
(3) 95°C for 20 seconds - 66°C for 15 seconds - 72°C for 1 minute, 3 times
(4) 95°C for 20 seconds - 64°C for 15 seconds - 72°C for 1 minute, 35 times
(5) 72°C for 5 minutes

The PCR product obtain was cloned into pCDNA3.1-/V5-His TOPO (Invitrogen) to transfect Escherichia coli DH5α. The resulting colony was cultured in LB medium and the vector was recovered using QIAGEN plasmid midi kit (Qiagen). This NIPK gene expression vector was transfected to SK-N-AS cells using Nucleofector (AMAXA). For control, pCDNA3.1 (Invitrogen) was transfected to SK-N-AS cells.

The transfectant cells were plated, respectively, on a type I collagen coated 96-well plate (IWAKI) in 7500 cells/well. After incubation overnight, tunicamycin or thapsigargin were added to the cells in various concentrations and cultured for 1 to 2 days to induce cell death. After the incubation, DNA cleavage accompanied by cell death was detected using CELL DEATH DETECTION ELISA PLUS kit (Roche). The experimental method was carried out in accordance with the operating manual attached to the kit.

The results are shown in FIGS. 1 to 4.

The DNA cleavage increased in human NIPK gene transfectant cells when compared to pCDNA3.1-transfected SK-N-AS cells (control cells). From the results, it is evident that NIPK has the cell death promoting action.

### EXAMPLE 4

In order to examine if NIPK would affect cell death, rat NIPK gene expression cells were prepared using a lentivirus and provided for the cell death induction system.

Tunicamycin was added to rat hippocampal neurons in a final concentration of 100 nM, followed by incubation for 8 hours. After the incubation, the total RNA was extracted from the cells using RNeasy Mini kit (Qiagen). Using as a template the total RNA obtained, reverse transcription was carried out on RNA PCR kit (TAKARA). For amplification of rat NIPK gene, PCR was carried out with synthetic primers (SEQ ID NO: 21 and SEQ ID NO: 22) and Pfu turbo (Stratagene) as an enzyme under the conditions (1) - (3) below to obtain the specific PCR product.
(1) 94°C for 1 minute
(2) 94°C for 30 seconds -65°C for 30 seconds -72°C for 30 seconds, 25 times
(3) 72°C for 5 minutes

The PCR product obtain was cloned into pLenti6/V5-TOPO vector using viraPower Lentiviral Directional TOPO Expression Kit (INVITROGEN) to transfect Escherichia coli DH5α. Using Nucleofector (AMAXA), the NIPK lentivirus vector prepared or LacZ lentivirus vector and packaging Mix were co-transfected into HEK293FT cells (Invitrogen). The medium was exchanged 24 hours after and the culture supernatant was recovered 48 and 72 hours after. The culture supernatant recovered was centrifuged at 40000 x g for 4 hours. The precipitates were dissolved in a 1/100 volume of neurobasal medium. After allowing to stand at 4°C overnight, the solution was warmed to 37°C and provided for use.

Hippocampal neurons were isolated from TP17 embryos of SD-IGS rat (purchased from Charles River Japan, Inc.) in a conventional manner and plated on a poly-L-lysine coated 24-well plate (SUMILON) in 250,000 cells/well. After culturing for 4 days, 500 µl of medium containing a lentivirus (corresponding to 50 ml of the culture supernatant) was added to the medium and 24 hours after, the whole volume of the medium was exchanged with 3 nM thapsigargin-containing medium. Two days after culturing, a 1/10 volume of WST-8 was added to the medium followed by culturing at 37°C for 90 minutes. Thereafter, 200 µl of the medium was recovered and absorbance was determined at 450 nm.

The results are shown in FIG. 5.

When compared to LacZ transfected rat neurons (control cells), reduced mitochondrial respiratory activity was observed in rat NIPK gene transfectant cells. The results reveal that NIPK has the cell death promoting activity.

### EXAMPLE 5

In order to confirm that NIPK binds to Akt1, the following experiment was carried out.

Using human NIPK cloned into pCDNA3.1-/V5-His TOPO (Invitrogen) vector (EXAMPLE 3) as a template, PCR was carried out with synthetic primers (SEQ ID NO: 3 and SEQ ID NO: 23) and Pfu turbo (Stratagene) as an enzyme under the conditions (1) - (3) to obtain the specific PCR product.
(1) 94°C for 5 minutes
(2) 94°C for 30 seconds - 60°C for 30 seconds - 72°C for 1 minute, 25 times
(3) 72°C for 5 minutes

The PCR product obtained was cloned into pCDNA3.1-/V5-His TOPO (Invitrogen) to transfect Escherichia coli DH5α. The resulting colony was cultured in LB medium to recover V5-tagged human NIPK gene expression vector.

Aktlgene expression vector was prepared by the following procedures. Using Quick-clone human whole brain cDNA (Clontech) as a template, PCR was carried out with synthetic primers (SEQ ID NO: 24 and SEQ ID NO: 25) and Pfu turbo (Stratagene) as an enzyme under the conditions (1) - (3) below to obtain the specific PCR product.
(1) 94°C for 5 minutes
(2) 94°C for 30 seconds - 58°C for 30 seconds - 72°C for 2 minutes, 35 times
(3) 72°C for 5 minutes

Using as a template 2 µl of the PCR product obtained, PCR was carried out with the same synthetic primers and enzyme under the conditions (1) - (3) below to obtain the specific PCR product.
(1) 94°C for 5 minutes
(2) 94°C for 30 seconds - 58°C for 30 seconds - 72°C for 2 minutes, 20 times
(3) 72°C for 5 minutes

The PCR product obtained was cloned into pCDNA3.1-/V5-His TOPO (Invitrogen) to transfect Escherichia coli DH5α. The resulting colony was cultured in LB medium to recover the vector using QIAGEN endofree maxi kit (Qiagen).

The V5-tagged human NIPK gene expression vector obtained above and Akt1gene expression vector were co-transfected into COS7 cells using Nucleofector (AMAXA). The cells were plated on a type I collagen coated Petri dish of 10 cm at 4,000,000 cells/dish, followed by culturing at 37°C for 18 hours. The medium was removed and the cells were recovered. The cells were then suspended in lysis buffer [50 mM Tris-HCl (pH7.5), 150 mM NaCl, 0.5 % NonidetP-40, 1 mM β-mercaptoethanol, protease inhibitor cocktail (ROCHE)] and homogenated with a homogenizer. To the supernatant was recovered by centrifugation rec-protein G Sepharose 4B (ZYMED) was added. After reacting them at 4°C for 2 hours, the supernatant was recovered and rec-protein G Sepharose 4B and anti-V5 antibody (INVITROGEN) were added thereto, followed by reacting at 4°C for 18 hours while rotating. Washing 4 times with 1 ml of lysis buffer was followed by electrophoresis and subsequent western blotting using anti-Akt1 antibody (SANTA CRUZ).

The results are shown in FIG. 6.

When V5-tagged NIPI and Akt1 were co-expressed, Akt1 was detected, whereas Akt1 was not detected when GFP (Wako Pure Chemical) and Akt1 were co-expressed (control group). Based on this it was confirmed that NIPK bound to Akt1 within the cells, suggesting that the binding of NIPK to Akt1 would cause reduced activity of Akt1, leading to promotion of the neuronal death mediated by Akt1 suppressing the neuron protecting activity. Accordingly, it can be expected to treat neurodegenerative diseases by inhibiting NIPK.

### INDUSTRIAL APPLICABILITY

The compound or its salts that inhibit the activity of a protein comprising the same or substantially the same amino acid sequence as the amino acid sequence represented by SEQ ID NO: 1, the compound or its salts that inhibit the expression of a gene for the protein, the antibody to the protein, the antisense polynucleotide to the protein, etc. can be used, for example, as prophylactic/therapeutic agents for neurodegenerative diseases [e.g., Alzheimer's diseases (familial Alzheimer's disease, juvenile Alzheimer's disease, sporadic Alzheimer's disease, etc.), Parkinson's disease, Down's syndrome, amyotrophic lateral sclerosis, prion disease, Creutzfeldt-Jakob disease, Huntington's chorea, diabetic neuropathy, multiple sclerosis, etc.], diabetes and its complications (e.g., diabetic nephropathy, diabetic neuropathy, diabetic retinopathy, diabetic foot, arteriosclerosis, hypertension, hyperlipemia, etc.), and the like; the compound or its salts that promote the activity of the protein, the compound or its salts that promote the expression of a gene for the protein, the protein, the polynucleotide encoding the protein, etc. can be used, for example, as prophylactic/therapeutic agents for cancers (e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, ovarian cancer, blood tumor, etc.), rheumatic diseases (e.g., chronic articular rheumatism, osteoarthritis, gout, etc.) and the like. Also, the various polynucleotides of the present invention are useful for diagnosis of, e.g., the diseases described above.

ccgatgccct tagccccaac ccgatcccat ctctgggagg ctgcccaggt ggtccctgat 1020
ggactggggc tggacgaagc cagggaagag gagggagaca gagaagtggt tctgtatggc 1080

<210> 20
<211> 360
<212> PRT
<213> Homo sapience

<400> 20

<210> 21
<211> 30
<212> DNA
<213> Artificial Sequence

<220>
<223> Primer

<400> 21
caccatgcga gccacatctc tggctgcttc                                    30

<210> 22
<211> 30
<212> DNA
<213> Artificial Sequence

<220>
<223> Primer

SEQUENCE LISTING

<110> Takeda Pharmaceutical Company Limited

<120> Preventives/remedies for neurodegenerative disease

<130> P04-252PCT

<150> JP2002-269091
<151> 2002-09-13

<160> 25

<210> 1
<211> 358
<212> PRT
<213> Homo sapience

<400> 1
Met Arg Ala Thr Pro Leu Ala Ala Pro Ala Gly Ser Leu Ser Arg Lys
                5                   10                  15
Lys Arg Leu Glu Leu Asp Asp Asn Leu Asp Thr Glu Arg Pro Val Gln
            20                  25                  30
Lys Arg Ala Arg Ser Gly Pro Gln Pro Arg Leu Pro Pro Cys Leu Leu
        35                  40                  45
Pro Leu Ser Pro Pro Thr Ala Pro Asp Arg Ala Thr Ala Val Ala Thr
    50                  55                  60
Ala Ser Arg Leu Gly Pro Tyr Val Leu Leu Glu Pro Glu Glu Gly Gly
65                  70                  75                  80
Arg Ala Tyr Gln Ala Leu His Cys Pro Thr Gly Thr Glu Tyr Thr Cys
                85                  90                  95
Lys Val Tyr Pro Val Gln Glu Ala Leu Ala Val Leu Glu Pro Tyr Ala
            100                 105                 110
Arg Leu Pro Pro His Lys His Val Ala Arg Pro Thr Glu Val Leu Ala
        115                 120                 125
Gly Thr Gln Leu Leu Tyr Ala Phe Phe Thr Arg Thr His Gly Asp Met
    130                 135                 140
His Ser Leu Val Arg Ser Arg His Arg Ile Pro Glu Pro Glu Ala Ala
145                 150                 155                 160
Val Leu Phe Arg Gln Met Ala Thr Ala Leu Ala His Cys His Gln His
                165                 170                 175
Gly Leu Val Leu Arg Asp Leu Lys Leu Cys Arg Phe Val Phe Ala Asp
            180                 185                 190
Arg Glu Arg Lys Lys Leu Val Leu Glu Asn Leu Glu Asp Ser Cys Val
        195                 200                 205
Leu Thr Gly Pro Asp Asp Ser Leu Trp Asp Lys His Ala Cys Pro Ala
    210                 215                 220
Tyr Val Gly Pro Glu Ile Leu Ser Ser Arg Ala Ser Tyr Ser Gly Lys
225                 230                 235                 240
Ala Ala Asp Val Trp Ser Leu Gly Val Ala Leu Phe Thr Met Leu Ala
                245                 250                 255
Gly His Tyr Pro Phe Gln Asp Ser Glu Pro Val Leu Leu Phe Gly Lys
            260                 265                 270
Ile Arg Arg Gly Ala Tyr Ala Leu Pro Ala Gly Leu Ser Ala Pro Ala
        275                 280                 285
Arg Cys Leu Val Arg Cys Leu Leu Arg Arg Glu Pro Ala Glu Arg Leu
    290                 295                 300
Thr Ala Thr Gly Ile Leu Leu His Pro Trp Leu Arg Gln Asp Pro Met
305                 310                 315                 320
Pro Leu Ala Pro Thr Arg Ser His Leu Trp Glu Ala Ala Gln Val Val
                325                 330                 335
Pro Asp Gly Leu Gly Leu Asp Glu Ala Arg Glu Glu Glu Gly Asp Arg
            340                 345                 350
Glu Val Val Leu Tyr Gly
        355

<210> 2
<211> 1074
<212> DNA
<213> Homo sapience

<400> 2
   atgcgagcca cccctctggc tgctcctgcg ggttccctgt ccaggaagaa gcggttggag      60
   ttggatgaca acttagatac cgagcgtccc gtccagaaac gagctcgaag tgggccccag     120
   cccagactgc ccccctgcct gttgcccctg agcccaccta ctgctccaga tcgtgcaact     180
   gctgtggcca ctgcctcccg tcttgggccc tatgtcctcc tggagcccga ggagggcggg 240
   cgggcctacc aggccctgca ctgccctaca ggcactgagt atacctgcaa ggtgtacccc 300
   gtccaggaag ccctggccgt gctggagccc tatgcgcggc tgcccccgca caagcatgtg 360
   gctcggccca ctgaggtcct ggctggtacc cagctcctct acgccttttt cactcggacc 420
   catggggaca tgcacagcct ggtgcgaagc cgccaccgta tccctgagcc tgaggctgcc 480
   gtgctcttcc gccagatggc caccgccctg gcgcactgtc accagcacgg tctggtcctg 540
   cgtgatctca agctgtgtcg ctttgtcttc gctgaccgtg agaggaagaa gctggtgctg 600
   gagaacctgg aggactcctg cgtgctgact gggccagatg attccctgtg ggacaagcac 660
   gcgtgcccag cctacgtggg acctgagata ctcagctcac gggcctcata ctcgggcaag 720
   gcagccgatg tctggagcct gggcgtggcg ctcttcacca tgctggccgg ccactacccc 780
   ttccaggact cggagcctgt cctgctcttc ggcaagatcc gccgcggggc ctacgccttg 840
   cctgcaggcc tctcggcccc tgcccgctgt ctggttcgct gcctccttcg tcgggagcca 900
   gctgaacggc tcacagccac aggcatcctc ctgcacccct ggctgcgaca ggacccgatg 960
   cccttagccc caacccgatc ccatctctgg gaggctgccc aggtggtccc tgatggactg 1020
   gggctggacg aagccaggga agaggaggga gacagagaag tggttctgta tggc 1074

<210> 3
<211> 32
<212> DNA
<213> Artificial Sequence

<220>
<223> Primer

<400> 3
gccatgcgag ccacccctct ggctgctcct gc 32

<210> 4
<211> 28
<212> DNA
<213> Artificial Sequence

<220>
<223> Primer

<400> 4
cctagccata cagaaccact tctctgtc 28

<210> 5
<211> 1074
<212> DNA
<213> Homo sapience

<400> 5
   atgcgagcca cccctctagc tgctcctgcg ggttccctgt ccaggaagaa gcggttggag 60
   ttggatgaca acttagatac cgagcgtccc gtccagaaac gagctcgaag tgggccccag 120
   cccagactgc ccccctgcct gttgcccctg agcccaccta ctgctccaga tcgtgcaact 180
   gctgtggcca ctgcctcccg tcttgggccc tatgtcctcc tggagcccga ggagggcggg 240
   cgggcctacc aggccctgca ctgccctaca ggcactgagt atacctgcaa ggtgtacccc 300
   gtccaggaag ccccggccgt gctggagccc tatgcgcggc tgcccccgca caagcatgtg 360
   gctcggccca ctgaggtcct ggctggtacc cagctcctct acgccttttt cactcggacc 420
   catggggaca tgcacagcct ggtgcgaagc cgccaccgta tccctgagcc tgaggctgcc 480
   gtgctcttcc gccagatggc caccgccctg gcgcactgtc accagcacgg tctggtcctg 540
   cgtgatctca agctgtgtcg ctttgtcttc gctgaccgtg agaggaagaa gctggtgctg 600
   gagaacctgg aggactcctg cgtgctgact gggccagatg attccctgtg ggacaagcac 660
   gcgtgcccag cctacgtggg acctgagata ctcagctcac gggcctcata ctcgggcaag 720
   gcagccgatg tctggagcct gggcgtggcg ctcttcacca tgctggccgg ccactacccc 780
   ttccaggact cggagcctgt cctgctcttc ggcaagatcc gccgcggggc ctacgccttg 840
   cctgcaggcc tctcggcccc tgcccgctgt ctggttcgct gcctccttcg tcgggagcca 900
   gctgaacggc tcacagccac aggcatcctc ctgcacccct ggctgcgaca ggacccgatg 960
   cccttagccc caacccgatc ccatctctgg gaggctgccc aggtggtccc tgatggtctg 1020
   gggctggacg aagccaggga agaggaggga gacagagaag tggttctgta tggc 1074

<210> 6
<211> 358
<212> PRT
<213> Homo sapience

<400> 6
Met Arg Ala Thr Pro Leu Ala Ala Pro Ala Gly Ser Leu Ser Arg Lys
                5                   10                  15
Lys Arg Leu Glu Leu Asp Asp Asn Leu Asp Thr Glu Arg Pro Val Gln
            20                  25                  30
Lys Arg Ala Arg Ser Gly Pro Gln Pro Arg Leu Pro Pro Cys Leu Leu
        35                  40                  45

Pro Leu Ser Pro Pro Thr Ala Pro Asp Arg Ala Thr Ala Val Ala Thr
    50                  55                  60
Ala Ser Arg Leu Gly Pro Tyr Val Leu Leu Glu Pro Glu Glu Gly Gly
65                  70                  75                  80
Arg Ala Tyr Gln Ala Leu His Cys Pro Thr Gly Thr Glu Tyr Thr Cys
                85                  90                  95
Lys Val Tyr Pro Val Gln Glu Ala Pro Ala Val Leu Glu Pro Tyr Ala
            100                 105                 110
Arg Leu Pro Pro His Lys His Val Ala Arg Pro Thr Glu Val Leu Ala
        115                 120                 125
Gly Thr Gln Leu Leu Tyr Ala Phe Phe Thr Arg Thr His Gly Asp Met
    130                 135                 140
His Ser Leu Val Arg Ser Arg His Arg Ile Pro Glu Pro Glu Ala Ala
145                 150                 155                 160
Val Leu Phe Arg Gln Met Ala Thr Ala Leu Ala His Cys His Gln His
                165                 170                 175
Gly Leu Val Leu Arg Asp Leu Lys Leu Cys Arg Phe Val Phe Ala Asp
            180                 185                 190
Arg Glu Arg Lys Lys Leu Val Leu Glu Asn Leu Glu Asp Ser Cys Val
        195                 200                 205
Leu Thr Gly Pro Asp Asp Ser Leu Trp Asp Lys His Ala Cys Pro Ala
    210                 215                 220
Tyr Val Gly Pro Glu Ile Leu Ser Ser Arg Ala Ser Tyr Ser Gly Lys
225                 230                 235                 240
Ala Ala Asp Val Trp Ser Leu Gly Val Ala Leu Phe Thr Met Leu Ala
                245                 250                 255
Gly His Tyr Pro Phe Gln Asp Ser Glu Pro Val Leu Leu Phe Gly Lys
            260                 265                 270
Ile Arg Arg Gly Ala Tyr Ala Leu Pro Ala Gly Leu Ser Ala Pro Ala
        275                 280                 285
Arg Cys Leu Val Arg Cys Leu Leu Arg Arg Glu Pro Ala Glu Arg Leu
    290                 295                 300
Thr Ala Thr Gly Ile Leu Leu His Pro Trp Leu Arg Gln Asp Pro Met
305                 310                 315                 320
Pro Leu Ala Pro Thr Arg Ser His Leu Trp Glu Ala Ala Gln Val Val
                325                 330                 335
Pro Asp Gly Leu Gly Leu Asp Glu Ala Arg Glu Glu Glu Gly Asp Arg
            340                 345                 350
Glu Val Val Leu Tyr Gly
        355

<210> 7
<211> 1074
<212> DNA
<213> Homo sapience

<400> 7
atgcgagcca cccctctagc tgctcctgcg ggttccctgt ccaggaagaa gcggttggag      60
ttggatgaca acttagatac cgagcgtccc gtccagaaac gagctcgaag tgggccccag     120
cccagactgc ccccctgcct gttgcccctg agcccaccta ctgctccaga tcgtgcaact     180
gctgtggcca ctgcctcccg tcttgggccc tatgtcctcc tggagcccga ggagggcggg     240
cgggcctacc gggccctgca ctgccctaca ggcactgagt atacctgcaa ggtgtacccc     300
gtccaggaag ccccggccgt gctggagccc tatgcgcggc tgcccccgca caagcatgtg     360
gctcggccca ctgaggtcct ggctggtacc cagctcctct acgccttttt cactcggacc     420
catggggaca tgcacagcct ggtgcgaagc cgccaccgta tccctgagcc tgaggctgcc     480
gtgctcttcc gccagatggc caccgccctg gcgcactgtc accagcacgg tctggtcctg     540
cgtgatctca agctgtgtcg ctttgtcttc gctgaccgtg agaggaagaa gctggtgctg     600
gagaacctgg aggactcctg cgtgctgact gggccagatg attccctgtg ggacaagcac     660
gcgtgcccag cctacgtggg acctgagata ctcagctcac gggcctcata ctcgggcaag     720
gcagccgatg tctggagcct gggcgtggcg ctcttcacca tgctggccgg ccactacccc     780
ttccaggact cggagcctgt cctgctcttc ggcaagatcc gccgcggggc ctacgccttg     840
cctgcaggcc tctcggcccc tgcccgctgt ctggttcgct gcctccttcg tcgggagcca     900
gctgaacggc tcacagccac aggcatcctc ctgcacccct ggctgcgaca ggacccgatg     960
cccttagccc caacccgatc ccatctctgg gaggctgccc aggtggtccc tgatggtctg    1020
gggctggacg aagccaggga agaggaggga gacagagaag tggttctgta tggc          1074

<210> 8
<211> 358
<212> PRT
<213> Homo sapience

<400> 8
Met Arg Ala Thr Pro Leu Ala Ala Pro Ala Gly Ser Leu Ser Arg Lys
                5                   10                  15
Lys Arg Leu Glu Leu Asp Asp Asn Leu Asp Thr Glu Arg Pro Val Gln

20                  25                  30
Lys Arg Ala Arg Ser Gly Pro Gln Pro Arg Leu Pro Pro Cys Leu Leu
            35                  40                  45
Pro Leu Ser Pro Pro Thr Ala Pro Asp Arg Ala Thr Ala Val Ala Thr
        50                  55                  60
Ala Ser Arg Leu Gly Pro Tyr Val Leu Leu Glu Pro Glu Glu Gly Gly
 65                  70                  75                  80
Arg Ala Tyr Arg Ala Leu His Cys Pro Thr Gly Thr Glu Tyr Thr Cys
                 85                  90                  95
Lys Val Tyr Pro Val Gln Glu Ala Pro Ala Val Leu Glu Pro Tyr Ala
            100                 105                 110
Arg Leu Pro Pro His Lys His Val Ala Arg Pro Thr Glu Val Leu Ala
        115                 120                 125
Gly Thr Gln Leu Leu Tyr Ala Phe Phe Thr Arg Thr His Gly Asp Met
    130                 135                 140
His Ser Leu Val Arg Ser Arg His Arg Ile Pro Glu Pro Glu Ala Ala
145                 150                 155                 160
Val Leu Phe Arg Gln Met Ala Thr Ala Leu Ala His Cys His Gln His
                165                 170                 175
Gly Leu Val Leu Arg Asp Leu Lys Leu Cys Arg Phe Val Phe Ala Asp
            180                 185                 190
Arg Glu Arg Lys Lys Leu Val Leu Glu Asn Leu Glu Asp Ser Cys Val
        195                 200                 205
Leu Thr Gly Pro Asp Asp Ser Leu Trp Asp Lys His Ala Cys Pro Ala
    210                 215                 220
Tyr Val Gly Pro Glu Ile Leu Ser Ser Arg Ala Ser Tyr Ser Gly Lys
225                 230                 235                 240
Ala Ala Asp Val Trp Ser Leu Gly Val Ala Leu Phe Thr Met Leu Ala
                245                 250                 255
Gly His Tyr Pro Phe Gln Asp Ser Glu Pro Val Leu Leu Phe Gly Lys
            260                 265                 270
Ile Arg Arg Gly Ala Tyr Ala Leu Pro Ala Gly Leu Ser Ala Pro Ala
        275                 280                 285
Arg Cys Leu Val Arg Cys Leu Leu Arg Arg Glu Pro Ala Glu Arg Leu
    290                 295                 300
Thr Ala Thr Gly Ile Leu Leu His Pro Trp Leu Arg Gln Asp Pro Met
305                 310                 315                 320
Pro Leu Ala Pro Thr Arg Ser His Leu Trp Glu Ala Ala Gln Val Val
                325                 330                 335
Pro Asp Gly Leu Gly Leu Asp Glu Ala Arg Glu Glu Glu Gly Asp Arg
            340                 345                 350
Glu Val Val Leu Tyr Gly
        355

<210> 9
<211> 1074
<212> DNA
<213> Homo sapience

<400> 9
atgcgagcca cccctctggc tgctcctgcg ggttccctgt ccaggaagaa gcggttggag      60
ttggatgaca acttagatac cgagcgtccc gtccagaaac gagctcgaag tgggccccag     120
cccagactgc ccccctgcct gttgcccctg agcccaccta ctgctccaga tcgtgcaact     180
gctgtggcca ctgcctcccg tcttgggccc tatgtcctcc tggagcccga ggagggcggg     240
cgggcctacc aggccctgca ctgccctaca ggcactgagt atacctgcaa ggtgtacccc     300
gtccaggaag ccctggccgt gctggagccc tacgcgcggc tgcccccgca caagcatgtg     360
gctcggccca ctgaggtcct ggctggtacc cagctcctct acgccttttt cactcggacc     420
catggggaca tgcacagcct ggtgcgaacg cgccaccgta tccctgagcc tgaggctgcc     480
gtgctcttcc gccagatggc caccgccctg gcgcactgtc accagcacgg tctggtcctg     540
cgtgatctca agctgtgtcg ctttgtcttc gctgaccgtg agaggaagaa gctggtgctg     600
gagaacctgg aggactcctg cgtgctgact gggccagatg attccctgtg ggacaagcac     660
gcgtgcccag cctacgtggg acctgagata ctcagctcac gggcctcata ctcgggcaag     720
gcagccgatg tctggagcct gggcgtggcg ctcttcacca tgctggccgg ccactacccc     780
ttccaggact cggagcctgt cctgctcttc ggcaagatcc gccgcggggc ctacgccttg     840
cctgcaggcc tctcggcccc tgcccgctgt ctggttcgct gcctccttcg tcgggagcca     900
gctgaacggc tcacagccac aggcatcctc ctgcacccct ggctgcgaca ggacccgatg     960
cccttagccc caacccgatc ccatctctgg gaggctgccc aggtggtccc tgatggactg    1020
gggctggacg aagccaggga agaggaggga gacagagaag tggttctgta tggc          1074

<210> 10
<211> 358
<212> PRT
<213> Homo sapience

<400> 10

Met Arg Ala Thr Pro Leu Ala Ala Pro Ala Gly Ser Leu Ser Arg Lys
                5                   10                  15
Lys Arg Leu Glu Leu Asp Asp Asn Leu Asp Thr Glu Arg Pro Val Gln
            20                  25                  30
Lys Arg Ala Arg Ser Gly Pro Gln Pro Arg Leu Pro Pro Cys Leu Leu
        35                  40                  45
Pro Leu Ser Pro Pro Thr Ala Pro Asp Arg Ala Thr Ala Val Ala Thr
    50                  55                  60
Ala Ser Arg Leu Gly Pro Tyr Val Leu Leu Glu Pro Glu Glu Gly Gly
65                  70                  75                  80
Arg Ala Tyr Gln Ala Leu His Cys Pro Thr Gly Thr Glu Tyr Thr Cys
                85                  90                  95
Lys Val Tyr Pro Val Gln Glu Ala Leu Ala Val Leu Glu Pro Tyr Ala
            100                 105                 110
Arg Leu Pro Pro His Lys His Val Ala Arg Pro Thr Glu Val Leu Ala
        115                 120                 125
Gly Thr Gln Leu Leu Tyr Ala Phe Phe Thr Arg Thr His Gly Asp Met
    130                 135                 140
His Ser Leu Val Arg Thr Arg His Arg Ile Pro Glu Pro Glu Ala Ala
145                 150                 155                 160
Val Leu Phe Arg Gln Met Ala Thr Ala Leu Ala His Cys His Gln His
                165                 170                 175
Gly Leu Val Leu Arg Asp Leu Lys Leu Cys Arg Phe Val Phe Ala Asp
            180                 185                 190
Arg Glu Arg Lys Lys Leu Val Leu Glu Asn Leu Glu Asp Ser Cys Val
        195                 200                 205
Leu Thr Gly Pro Asp Asp Ser Leu Trp Asp Lys His Ala Cys Pro Ala
    210                 215                 220
Tyr Val Gly Pro Glu Ile Leu Ser Ser Arg Ala Ser Tyr Ser Gly Lys
225                 230                 235                 240
Ala Ala Asp Val Trp Ser Leu Gly Val Ala Leu Phe Thr Met Leu Ala
                245                 250                 255
Gly His Tyr Pro Phe Gln Asp Ser Glu Pro Val Leu Leu Phe Gly Lys
            260                 265                 270
Ile Arg Arg Gly Ala Tyr Ala Leu Pro Ala Gly Leu Ser Ala Pro Ala
        275                 280                 285
Arg Cys Leu Val Arg Cys Leu Leu Arg Arg Glu Pro Ala Glu Arg Leu
    290                 295                 300
Thr Ala Thr Gly Ile Leu Leu His Pro Trp Leu Arg Gln Asp Pro Met
305                 310                 315                 320
Pro Leu Ala Pro Thr Arg Ser His Leu Trp Glu Ala Ala Gln Val Val
                325                 330                 335
Pro Asp Gly Leu Gly Leu Asp Glu Ala Arg Glu Glu Glu Gly Asp Arg
            340                 345                 350
Glu Val Val Leu Tyr Gly
        355

<210> 11
<211> 969
<212> DNA
<213> Homo sapience

<400> 11
atgcgagcca cccctttggc tgcttctgcg ggttccctgt ccaggaagaa gcggttggag      60
ttggatgaca acttagatac cgagcgtccc gtccagaaac gagctcgaag tgggccccag     120
cccagactgc ccccctgcct gttgcccctg agcccaccta ctgctccaga tcgtgcaact     180
gctgtggcca ctgcctcccg tcttgggccc tatgtcctcc tggagcccga ggagggcggg     240
cgggcctacc aggccctgca ctgccctaca ggcactgagt atacctgcaa ggtgtacccc     300
gtccaggaag ccctggccgt gctggaaccc tatgcgcggc tgcccccgga caagcatgtg     360
gcttggccca ctgagggcct ggctggtacc caacttctct acgccttttt cacttggacc     420
catggggaca tgcacaggct gatagggcac acgccctgtg cgcactgtga ccagacacgg     480
aagaagctgg tgctggagaa cctggaggac tcctgcgtgc tgactgggcc agatgattcc     540
ctgtgggaca agcacgcgtg cccagcctac gtgggacctg agatactcag ctcacgggcc     600
tcatactcgg gcaaggcagc caatgtctgg agcctgggcg tggcgctctt caccatgctg     660
gccggccact accccttcca ggactcggag cctgtcctgc tcttcggcaa gatccgccgc     720
ggggcctacg ccttgcctgc aggcctctcg gcccctgccc gctgtctggt tcgctgcctc     780
cttcgtcggg agccagctga acggctcaca gccacaggca tcctcctgca cccctggctg     840
cgacaggacc cgatgccctt agccccaacc cgatcccatc tctgggaggc tgcccaggtg     900
gtccctgatg gactggggct ggacgaagcc agggaagagg agggagacag agaagtggtt     960
ctgtatggc                                                             969

<210> 12
<211> 323
<212> PRT
<213> Homo sapience

<400> 12
Met Arg Ala Thr Pro Leu Ala Ala Ser Ala Gly Ser Leu Ser Arg Lys
                5                   10                  15
Lys Arg Leu Glu Leu Asp Asp Asn Leu Asp Thr Glu Arg Pro Val Gln
            20                  25                  30
Lys Arg Ala Arg Ser Gly Pro Gln Pro Arg Leu Pro Pro Cys Leu Leu
        35                  40                  45
Pro Leu Ser Pro Pro Thr Ala Pro Asp Arg Ala Thr Ala Val Ala Thr
    50                  55                  60
Ala Ser Arg Leu Gly Pro Tyr Val Leu Leu Glu Pro Glu Glu Gly Gly
65                  70                  75                  80
Arg Ala Tyr Gln Ala Leu His Cys Pro Thr Gly Thr Glu Tyr Thr Cys
                85                  90                  95
Lys Val Tyr Pro Val Gln Glu Ala Leu Ala Val Leu Glu Pro Tyr Ala
            100                 105                 110
Arg Leu Pro Pro Asp Lys His Val Ala Trp Pro Thr Glu Gly Leu Ala
        115                 120                 125
Gly Thr Gln Leu Leu Tyr Ala Phe Phe Thr Trp Thr His Gly Asp Met
    130                 135                 140
His Arg Leu Ile Gly His Thr Pro Cys Ala His Cys Asp Gln Thr Arg
145                 150                 155                 160
Lys Lys Leu Val Leu Glu Asn Leu Glu Asp Ser Cys Val Leu Thr Gly
                165                 170                 175
Pro Asp Asp Ser Leu Trp Asp Lys His Ala Cys Pro Ala Tyr Val Gly
            180                 185                 190
Pro Glu Ile Leu Ser Ser Arg Ala Ser Tyr Ser Gly Lys Ala Ala Asn
        195                 200                 205
Val Trp Ser Leu Gly Val Ala Leu Phe Thr Met Leu Ala Gly His Tyr
    210                 215                 220
Pro Phe Gln Asp Ser Glu Pro Val Leu Leu Phe Gly Lys Ile Arg Arg
225                 230                 235                 240
Gly Ala Tyr Ala Leu Pro Ala Gly Leu Ser Ala Pro Ala Arg Cys Leu
                245                 250                 255
Val Arg Cys Leu Leu Arg Arg Glu Pro Ala Glu Arg Leu Thr Ala Thr
            260                 265                 270
Gly Ile Leu Leu His Pro Trp Leu Arg Gln Asp Pro Met Pro Leu Ala
        275                 280                 285
Pro Thr Arg Ser His Leu Trp Glu Ala Ala Gln Val Val Pro Asp Gly
    290                 295                 300
Leu Gly Leu Asp Glu Ala Arg Glu Glu Glu Gly Asp Arg Glu Val Val
305                 310                 315                 320
Leu Tyr Gly

<210> 13
<211> 1074
<212> DNA
<213> Homo sapience

<400> 13
atgcgagcca cccctctggc tgctcctgcg ggttccctgt ccaggaagaa gcggttggag     60
ttggatgaca acttagatac cgagcgtccc gtccagaaac gagctcgaag tgggccccag    120
cccagactgc ccccctgcct gttgcccctg agcccaccta ctgctccaga tcgtgcaact    180
gctgtggcca ctgcctcccg tcttgggccc tatgtcctcc tggagcccga ggagggcggg    240
cgggcctacc aggccctgca ctgccctaca ggcactgagt atacctgcag ggtgtacccc    300
gtccaggaag ccctggccgt gctggagccc tatgcgcggc tgcccccgca caagcatgtg    360
gctcggccca ctgaggtcct ggctggtacc cagctcctct acgccttttt cactcggacc    420
catggggaca tgcacagcct ggtgcgaagc cgccaccgta tccctgagcc tgaggctgcc    480
gtgctcttcc gccagatggc caccgccctg gcgcactgtc accagcacgg tctggtcctg    540
cgtgatctca agctgtgtcg ctttgtcttc gctgaccgtg agaggaagaa gctggtgctg    600
gagaacctgg aggactcctg cgtgctgact gggccagatg attccctgtg ggacaagcac    660
gcgtgcccag cctacgtggg acctgagata ctcagctcac gggcctcata ctcgggcaag    720
gcagccgatg tctggagcct gggcgtggcg ctcttcacca tgctggccgg ccactacccc    780
ttccaggact cggagcctgt cctgctcttc ggcaagatcc gccgcggggc ctacgccttg    840
cctgcaggcc tctcggcccc tgcccgctgt ctggttcgct gcctccttcg tcgggagcca    900
gctgaacggc tcacagccac aggcatcctc ctgcacccct ggctgcgaca ggacccgatg    960
cccttagccc caacccgatc ccatctctgg gaggctgccc aggtggtccc tgatggactg   1020
gggctggacg aagccaggga agaggaggga gacagagaag tggttctgta tggc          1074

<210> 14
<211> 358
<212> PRT
<213> Homo sapience

<400> 14

Met Arg Ala Thr Pro Leu Ala Ala Pro Ala Gly Ser Leu Ser Arg Lys
                5                   10                  15
Lys Arg Leu Glu Leu Asp Asp Asn Leu Asp Thr Glu Arg Pro Val Gln
            20                  25                  30
Lys Arg Ala Arg Ser Gly Pro Gln Pro Arg Leu Pro Pro Cys Leu Leu
        35                  40                  45
Pro Leu Ser Pro Pro Thr Ala Pro Asp Arg Ala Thr Ala Val Ala Thr
    50                  55                  60
Ala Ser Arg Leu Gly Pro Tyr Val Leu Leu Glu Pro Glu Glu Gly Gly
65                  70                  75                  80
Arg Ala Tyr Gln Ala Leu His Cys Pro Thr Gly Thr Glu Tyr Thr Cys
                85                  90                  95
Arg Val Tyr Pro Val Gln Glu Ala Leu Ala Val Leu Glu Pro Tyr Ala
            100                 105                 110
Arg Leu Pro Pro His Lys His Val Ala Arg Pro Thr Glu Val Leu Ala
        115                 120                 125
Gly Thr Gln Leu Leu Tyr Ala Phe Phe Thr Arg Thr His Gly Asp Met
    130                 135                 140
His Ser Leu Val Arg Ser Arg His Arg Ile Pro Glu Pro Glu Ala Ala
145                 150                 155                 160
Val Leu Phe Arg Gln Met Ala Thr Ala Leu Ala His Cys His Gln His
                165                 170                 175
Gly Leu Val Leu Arg Asp Leu Lys Leu Cys Arg Phe Val Phe Ala Asp
            180                 185                 190
Arg Glu Arg Lys Lys Leu Val Leu Glu Asn Leu Glu Asp Ser Cys Val
        195                 200                 205
Leu Thr Gly Pro Asp Asp Ser Leu Trp Asp Lys His Ala Cys Pro Ala
    210                 215                 220
Tyr Val Gly Pro Glu Ile Leu Ser Ser Arg Ala Ser Tyr Ser Gly Lys
225                 230                 235                 240
Ala Ala Asp Val Trp Ser Leu Gly Val Ala Leu Phe Thr Met Leu Ala
                245                 250                 255
Gly His Tyr Pro Phe Gln Asp Ser Glu Pro Val Leu Leu Phe Gly Lys
            260                 265                 270
Ile Arg Arg Gly Ala Tyr Ala Leu Pro Ala Gly Leu Ser Ala Pro Ala
        275                 280                 285
Arg Cys Leu Val Arg Cys Leu Leu Arg Arg Glu Pro Ala Glu Arg Leu
    290                 295                 300
Thr Ala Thr Gly Ile Leu Leu His Pro Trp Leu Arg Gln Asp Pro Met
305                 310                 315                 320
Pro Leu Ala Pro Thr Arg Ser His Leu Trp Glu Ala Ala Gln Val Val
                325                 330                 335
Pro Asp Gly Leu Gly Leu Asp Glu Ala Arg Glu Glu Glu Gly Asp Arg
            340                 345                 350
Glu Val Val Leu Tyr Gly
        355

<210> 15
<211> 1074
<212> DNA
<213> Homo sapience

<400> 15
atgcgagcca cccctctggc tgctcctgcg ggttccctgt ccaggaagaa gcggttggag      60
ttggatgaca acttagatac cgagcgtccc gtccagaaac gagctcgaag tgggccccag     120
cccagactgc ccccctgcct gttgcccctg agcccaccta ctgctccaga tcgtgcaact     180
gctgtggcca ctgcctcccg tcttgggccc tatgtcctcc tggagcccga ggagggcggg     240
cgggcctacc aggccctgca ctgccctaca ggcactgagt atacctgcag ggtgtacccc     300
gtccaggaag ccctggccgt gctggagccc tatgcgcggc tgcccccgca caagcatgtg     360
gctcggccca ctgaggtcct ggctggtacc cagctcctct acgccttttt cactcggacc     420
catggggaca tgcacagcct ggtgcgaagc cgccaccgta tccctgagcc tgaggctgcc     480
gtgctcttcc gccagatggc caccgccctg gcgcactgtc accagcacgg tctggtcctg     540
cgtgatctca agctgtgtcg ctttgtcttc gctgaccgtg agaggaagaa gctggtgctg     600
gagaacctgg aggactcctg cgtgctgact gggccagatg attccctgtg ggacaagcac     660
gcgtgcccag cctacgtggg acctgagata ctcagctcac gggcctcata ctcgggcaag     720
gcagccgatg tctggagcct gggcgtggcg ctcttcacca tgctggccgg ccactacccc     780
ttccaggact cggagcctgt cctgctcttc ggcaagatcc gccgcggggc ctacgccttg     840
cctgcaggcc tctcggcccc tgcccgctgt ctggttcgct gcctccttcg tcgggagcca     900
gctgaacggc tcacagccac aggcatcctc ctgcacccct ggctgcgaca ggacccgatg     960
cccttagccc caacccgatc ccatctctgg gaggctgccc aggtggtccc tgatggactg    1020
gggctggacg aagccaggga agaggaggga gacagagaag tggttctgta tggc          1074

<210> 16
<211> 358
<212> PRT

<213> Homo sapience

<400> 16
Met Arg Ala Thr Pro Leu Ala Ala Pro Ala Gly Ser Leu Ser Arg Lys
                5                   10                  15
Lys Arg Leu Glu Leu Asp Asp Asn Leu Asp Thr Glu Arg Pro Val Gln
            20                  25                  30
Lys Arg Ala Arg Ser Gly Pro Gln Pro Arg Leu Pro Pro Cys Leu Leu
        35                  40                  45
Pro Leu Ser Pro Pro Thr Ala Pro Asp Arg Ala Thr Ala Val Ala Thr
    50                  55                  60
Ala Ser Arg Leu Gly Pro Tyr Val Leu Leu Glu Pro Glu Glu Gly Gly
65                  70                  75                  80
Arg Ala Tyr Gln Ala Leu His Cys Pro Thr Gly Thr Glu Tyr Thr Cys
                85                  90                  95
Arg Val Tyr Pro Val Gln Glu Ala Leu Ala Val Leu Glu Pro Tyr Ala
            100                 105                 110
Arg Leu Pro Pro His Lys His Val Ala Arg Pro Thr Glu Val Leu Ala
        115                 120                 125
Gly Thr Gln Leu Leu Tyr Ala Phe Phe Thr Arg Thr His Gly Asp Met
    130                 135                 140
His Ser Leu Val Arg Ser Arg His Arg Ile Pro Glu Pro Glu Ala Ala
145                 150                 155                 160
Val Leu Phe Arg Gln Met Ala Thr Ala Leu Ala His Cys His Gln His
                165                 170                 175
Gly Leu Val Leu Arg Asp Leu Lys Leu Cys Arg Phe Val Phe Ala Asp
            180                 185                 190
Arg Glu Arg Lys Lys Leu Val Leu Glu Asn Leu Glu Asp Ser Cys Val
        195                 200                 205
Leu Thr Gly Pro Asp Asp Ser Leu Trp Asp Lys His Ala Cys Pro Ala
    210                 215                 220
Tyr Val Gly Pro Glu Ile Leu Ser Ser Arg Ala Ser Tyr Ser Gly Lys
225                 230                 235                 240
Ala Ala Asp Val Trp Ser Leu Gly Val Ala Leu Phe Thr Met Leu Ala
                245                 250                 255
Gly His Tyr Pro Phe Gln Asp Ser Glu Pro Val Leu Leu Phe Gly Lys
            260                 265                 270
Ile Arg Arg Gly Ala Tyr Ala Leu Pro Ala Gly Leu Ser Ala Pro Ala
        275                 280                 285
Arg Cys Leu Val Arg Cys Leu Leu Arg Arg Glu Pro Ala Glu Arg Leu
    290                 295                 300
Thr Ala Thr Gly Ile Leu Leu His Pro Trp Leu Arg Gln Asp Pro Met
305                 310                 315                 320
Pro Leu Ala Pro Thr Arg Ser His Leu Trp Glu Ala Ala Gln Val Val
                325                 330                 335
Pro Asp Gly Leu Gly Leu Asp Glu Ala Arg Glu Glu Glu Gly Asp Arg
            340                 345                 350
Glu Val Val Leu Tyr Gly
        355

<210> 17
<211> 1074
<212> DNA
<213> Homo sapience

<400> 17
atgcgagcca cccctctggc tgctcctgcg ggttccctgt ccaggaagaa gcggttggag      60
ttggatgaca acttagatac cgagcgtccc gtccagaaac gagctcgaag tgggccccag     120
cccagactgc ccccctgcct gttgcccctg agcccaccta ctgctccaga tcgtgcaact     180
gctgtggcca ctgcctcccg tcttgggccc tatgtcctcc tggagcccga ggagggcggg     240
cgggcctacc aggccctgca ctgccctaca ggcactgagt atacctgcaa ggtgtacccc     300
gtccaggaag ccctggccgt gctggagccc tatgcgcggc tgcccccgca caagcatgtg     360
gctcggccca ctgaggtcct ggctggtacc cagctcctct acgccttttt cactcggacc     420
catggggaca tgcacagcct ggtgcgaagc cgccaccgta tccctgagcc tgaggctgcc     480
gtgctcttcc gccagatggc caccgccctg gcgcactgtc accagcacgg tctggtcctg     540
cgtgatctca agctgtgtcg ctttgtcttc gctgaccgtg agaggaagaa gctggtgctg     600
gagaacctgg aggactcctg cgtgctgact gggccagatg attccctgtg ggacaagcac     660
gcgtgcccag cctacgtggg acctgagata ctcagctcac gggcctcata ctcgggcaag     720
gcagccgatg tctggagcct gggcgtggcg ctcttcacca tgctggccgg ccactacccc     780
ttccaggact cggagcctgt cctgctcttc ggcaagatcc gccgcggggc ctacgccttg     840
cctgcaggcc tctcggcccc tgcccgctgt ctggttcgct gcctccttcg tcgggagcca     900
gctgaacggc tcacagccac aggcatcctc ctgcacccct ggctgcgaca ggacccgatg     960
cccttagccc caacccgatc ccatctctgg gaggctgccc aggtggtccc tgatggactg    1020
gggctggacg aagccaggga agaggaggga gacagagaag tggttctgta tggc          1074

<210> 18
<211> 358
<212> PRT
<213> Homo sapience

<400> 18
Met Arg Ala Thr Pro Leu Ala Ala Pro Ala Gly Ser Leu Ser Arg Lys
                5                   10                  15
Lys Arg Leu Glu Leu Asp Asp Asn Leu Asp Thr Glu Arg Pro Val Gln
            20                  25                  30
Lys Arg Ala Arg Ser Gly Pro Gln Pro Arg Leu Pro Pro Cys Leu Leu
        35                  40                  45
Pro Leu Ser Pro Pro Thr Ala Pro Asp Arg Ala Thr Ala Val Ala Thr
    50                  55                  60
Ala Ser Arg Leu Gly Pro Tyr Val Leu Leu Glu Pro Glu Glu Gly Gly
65                  70                  75                  80
Arg Ala Tyr Gln Ala Leu His Cys Pro Thr Gly Thr Glu Tyr Thr Cys
                85                  90                  95
Lys Val Tyr Pro Val Gln Glu Ala Leu Ala Val Leu Glu Pro Tyr Ala
            100                 105                 110
Arg Leu Pro Pro His Lys His Val Ala Arg Pro Thr Glu Val Leu Ala
        115                 120                 125
Gly Thr Gln Leu Leu Tyr Ala Phe Phe Thr Arg Thr His Gly Asp Met
    130                 135                 140
His Ser Leu Val Arg Ser Arg His Arg Ile Pro Glu Pro Glu Ala Ala
145                 150                 155                 160
Val Leu Phe Arg Gln Met Ala Thr Ala Leu Ala His Cys His Gln His
                165                 170                 175
Gly Leu Val Leu Arg Asp Leu Lys Leu Cys Arg Phe Val Phe Ala Asp
            180                 185                 190
Arg Glu Arg Lys Lys Leu Val Leu Glu Asn Leu Glu Asp Ser Cys Val
        195                 200                 205
Leu Thr Gly Pro Asp Asp Ser Leu Trp Asp Lys His Ala Cys Pro Ala
    210                 215                 220
Tyr Val Gly Pro Glu Ile Leu Ser Ser Arg Ala Ser Tyr Ser Gly Lys
225                 230                 235                 240
Ala Ala Asp Val Trp Ser Leu Gly Val Ala Leu Phe Thr Met Leu Ala
                245                 250                 255
Gly His Tyr Pro Phe Gln Asp Ser Glu Pro Val Leu Leu Phe Gly Lys
            260                 265                 270
Ile Arg Arg Gly Ala Tyr Ala Leu Pro Ala Gly Leu Ser Ala Pro Ala
        275                 280                 285
Arg Cys Leu Val Arg Cys Leu Leu Arg Arg Glu Pro Ala Glu Arg Leu
    290                 295                 300
Thr Ala Thr Gly Ile Leu Leu His Pro Trp Leu Arg Gln Asp Pro Met
305                 310                 315                 320
Pro Leu Ala Pro Thr Arg Ser His Leu Trp Glu Ala Ala Gln Val Val
                325                 330                 335
Pro Asp Gly Leu Gly Leu Asp Glu Ala Arg Glu Glu Glu Gly Asp Arg
            340                 345                 350
Glu Val Val Leu Tyr Gly
        355

<210> 19
<211> 1080
<212> DNA
<213> Homo sapience

<400> 19
atgcgagcca cccctctggc tgctcctgcg ggttccctgt ccaggaagaa gcggttggag      60
ttggatgaca acttagatac cgagcgtccc gtccagaaac gagctcgaag tgggccccag     120
cccagactgc ccccctgcct gttgcccctg agcccaccta ctgctccaga tcgtgcaact     180
gctgtggcca ctgcctcccg tcttgggccc tatgtcctcc tggagcccga ggagggcggg     240
cgggcctacc aggccctgca ctgccctaca ggcactgagt atacctgcaa ggtgtacccc     300
gtccaggaag ccctggccgt gctggagccc tatgcgcggg tgcccccgca caagcatgtg     360
gctcggccca ctgaggtcct ggctggtacc cagctcctct acgccttttt cactcggacc     420
catggggaca tgcacagcct ggtgcgaagc cgccaccgta tccctgagcc tgaggctgcc     480
gtgctcttcc gccagatggc caccgccctg gcgcactgtc accagcacgg tctggtcctg     540
cgtgatctca agctgtgtcg ctttgtcttc gctgaccgtg accgtgagaa gaagaagctg     600
gtgctggaga acctggagga ctcctgcgtg ctgactgggc cagatgattc cctgtgggac     660
aagcacgcgt gcccagccta cgtgggacct gagatactca gctcacgggc ctcatactcg     720
ggcaaggcag ccgatgtctg gagcctgggc gtggcgctct tcaccatgct ggccggccac     780
taccccttcc aggactcgga gcctgtcctg ctcttcggca agatccgccg cggggcctac     840
gccttgcctg caggcctctc ggcccctgcc cgctgtctgg ttcgctgcct ccttcgtcgg     900
gagccagctg aacggctcac agccacaggc atcctcctgc acccctggct gcgacaggac     960

<400> 22
gctctagcca tacagcccca cctccccttc                                        30

<210> 23
<211> 27
<212> DNA
<213> Artificial Sequence

<220>
<223> Primer

<400> 23
gccatacaga accacttctc tgtctcc                                           27

<210> 24
<211> 30
<212> DNA
<213> Artificial Sequence

<220>
<223> Primer

<400> 24
gccgccatga gcgacgtggc tattgtgaag                                        30

<210> 25
<211> 30
<212> DNA
<213> Artificial Sequence

<220>
<223> Primer

<400> 25
aggatgagcc ggtcgtcgtg ccggactatc                                        30

## Claims

1. A prophylactic/therapeutic agent for neurodegenerative disease or diabetes, comprising a compound or its salt inhibiting the activity of a protein comprising the same or substantially the same amino acid sequence as the amino acid sequence represented by SEQ ID NO: 1, its partial peptide, or a salt thereof.

2. A prophylactic/therapeutic agent for neurodegenerative disease or diabetes, comprising a compound or its salt inhibiting the expression of a gene for a protein comprising the same or substantially the same amino acid sequence as the amino acid sequence represented by SEQ ID NO: 1, its partial peptide, or a salt thereof.

3. An antisense polynucleotide comprising the entire or part of a base sequence complementary or substantially complementary to a base sequence of a polynucleotide encoding a protein comprising the same or substantially the same amino acid sequence as the amino acid sequence represented by SEQ ID NO: 1, or a partial peptide thereof.

4. A pharmaceutical comprising the antisense polynucleotide according to claim 3.

5. The pharmaceutical according to claim 4, which is a prophylactic/therapeutic agent for neurodegenerative disease or diabetes.

6. An antibody to a protein comprising the same or substantially the same amino acid sequence as the amino acid sequence represented by SEQ ID NO: 1, its partial peptide, or a salt thereof.

7. A pharmaceutical comprising the antibody according to claim 6.

8. The pharmaceutical according to claim 7, which is a prophylactic/therapeutic agent for neurodegenerative disease or diabetes.

9. A diagnostic agent comprising the antibody according to claim 6.

10. The diagnostic agent according to claim 9, which is a diagnostic agent for neurodegenerative disease or diabetes.

11. A diagnostic agent for neurodegenerative disease or diabetes, comprising a polynucleotide encoding a protein comprising the same or substantially the same amino acid sequence as the amino acid sequence represented by SEQ ID NO: 1, or its partial peptide.

12. A method of screening a compound or its salt inhibiting the activity of a protein comprising the same or substantially the same amino acid sequence as the amino acid sequence represented by SEQ ID NO: 1, its partial peptide, or a salt thereof, which comprises using the protein, its partial peptide or a salt thereof.

13. A kit for screening a compound or its salt inhibiting the activity of a protein comprising the same or substantially the same amino acid sequence as the amino acid sequence represented by SEQ ID NO: 1, its partial peptide, or a salt thereof, comprising the protein, its partial peptide or a salt thereof.

14. A compound or its salt which is obtainable using the screening method according to claim 12 or the screening kit according to claim 13.

15. A method of screening a compound or its salt inhibiting the expression of a gene for a protein comprising the same or substantially the same amino acid sequence as the amino acid sequence represented by SEQ ID NO: 1, which comprises using a polynucleotide encoding the protein or its partial peptide.

16. A kit for screening a compound or its salt inhibiting the expression of a gene for a protein comprising the same or substantially the same amino acid sequence as the amino acid sequence represented by SEQ ID NO: 1, comprising a polynucleotide encoding the protein or its partial peptide.

17. A compound or its salt, which is obtainable using the screening method according to claim 15 or the screening kit according to claim 16.

18. A pharmaceutical comprising the compound or its salt according to claim 14 or 17.

19. The pharmaceutical according to claim 18, which is a prophylactic/therapeutic agent for neurodegenerative disease or diabetes.

20. A method of preventing/treating neurodegenerative disease or diabetes, which comprises administering an effective dose of the compound or its salt according to claim 14 or 17 to a mammal.

21. A method of preventing/treating neurodegenerative disease or diabetes, which comprises inhibiting the activity of a protein comprising the same or substantially the same amino acid sequence as represented by SEQ ID NO: 1, or its partial peptide, or inhibiting the expression of a gene for the protein.

22. Use of the compound or its salt according to claim 14 or 17 to manufacture a prophylactic/therapeutic agent for neurodegenerative disease or diabetes.

23. A prophylactic/therapeutic agent for a cancer or rheumatic disease, comprising a compound or its salt promoting the activity of a protein comprising the same or substantially the same amino acid sequence as the amino acid sequence represented by SEQ ID NO: 1, its partial peptide, or a salt thereof.

24. A prophylactic/therapeutic agent for a cancer or rheumatic disease, comprising a compound or its salt promoting the expression of a gene for a protein comprising the same or substantially the same amino acid sequence as the amino acid sequence represented by SEQ ID NO: 1, its partial peptide, or a salt thereof.

25. A pharmaceutical comprising a protein comprising the same or substantially the same amino acid sequence as the amino acid sequence represented by SEQ ID NO: 1, its partial peptide, or a salt thereof.

26. The pharmaceutical according to claim 25, which is a prophylactic/therapeutic agent for a cancer or rheumatic disease.

27. A pharmaceutical comprising a polynucleotide encoding a protein comprising the same or substantially the same amino acid sequence as the amino acid sequence represented by SEQ ID NO: 1, or its partial peptide.

28. The pharmaceutical according to claim 27, which is a prophylactic/therapeutic agent for a cancer or rheumatic disease.

29. A diagnostic agent for cancer or rheumatic disease, comprising a polynucleotide encoding a protein comprising the same or substantially the same amino acid sequence as the amino acid sequence represented by SEQ ID NO: 1, or its partial peptide.

30. A method of screening a compound or its salt promoting the activity of a protein comprising the same or substantially the same amino acid sequence as the amino acid sequence represented by SEQ ID NO: 1, its partial peptide, or a salt thereof, which comprises using the protein, its partial peptide, or a salt thereof.

31. A kit for screening a compound or its salt promoting the activity of a protein comprising the same or substantially the same amino acid sequence as the amino acid sequence represented by SEQ ID NO: 1, its partial peptide, or a salt thereof, comprising the protein, its partial peptide, or a salt thereof.

32. A compound or its salt, which is obtainable using the screening method according to claim 30 or the screening kit according to claim 31.

33. A method of screening a compound or its salt promoting the expression of a gene for a protein comprising the same or substantially the same amino acid sequence as the amino acid sequence represented by SEQ ID NO: 1, which comprises using a polynucleotide encoding the protein or its partial peptide.

34. A kit for screening a compound or its salt promoting the expression of a gene for a protein comprising the same or substantially the same amino acid sequence as the amino acid sequence represented by SEQ ID NO: 1, comprising a polynucleotide encoding the protein or its partial peptide.

35. A compound or its salt, which is obtainable using the screening method according to claim 33 or the screening kit according to claim 34.

36. A pharmaceutical comprising the compound or its salt according to claim 32 or 35.

37. The pharmaceutical according to claim 36, which is a prophylactic/therapeutic agent for a cancer or rheumatic disease.

38. A method of preventing/treating a cancer or rheumatic disease, which comprises administering an effective dose of the compound or its salt according to claim 32 or 35 to a mammal.

39. A method of preventing/treating a cancer or rheumatic disease, which comprises promoting the activity of a protein comprising the same or substantially the same amino acid sequence as the amino acid sequence represented by SEQ ID NO: 1 or its partial peptide, or promoting the expression of a gene for the protein.

40. Use of the compound or its salt according to claim 32 or 35 to manufacture a prophylactic/therapeutic agent for a cancer or rheumatic disease.
